Europäisches Patentamt

European Patent Office

Office européen des brevets

(19)

(11) **EP 1 146 895 B1**

(12) **EUROPEAN PATENT SPECIFICATION**

(45) Date of publication and mention
of the grant of the patent:
**12.11.2003 Bulletin 2003/46**

(51) Int Cl.[7]: **A61K 38/19**, A61K 35/12,
A61P 7/00

(21) Application number: **00913278.8**

(22) Date of filing: **28.01.2000**

(86) International application number:
**PCT/US00/02173**

(87) International publication number:
**WO 00/044398 (03.08.2000 Gazette 2000/31)**

(54) **THROMBOPOIETIN COMPOSITIONS FOR INCREASING CIRCULATING PLATELETS**

ERHÖHUNG VON ZIRKULIERENDEN PLÄTTCHEN MIT
THROMBOPOIETINZUSAMMENSETZUNGEN

AUGMENTATION DE PLAQUETTES CIRCULANTES AVEC DES COMPOSITIONS DE
THROMBOPOIETINE

(84) Designated Contracting States:
**AT BE CH CY DE DK ES FI FR GB GR IE IT LI LU
MC NL PT SE**

(30) Priority: **28.01.1999 US 239442**
**04.02.1999 US 244370**

(43) Date of publication of application:
**24.10.2001 Bulletin 2001/43**

(73) Proprietor: **BOARD OF REGENTS, THE
UNIVERSITY OF TEXAS SYSTEM
Austin, Texas 78701 (US)**

(72) Inventor: **VADHAN-RAJ, Saroj
Sugarland, TX 77479 (US)**

(74) Representative:
**Gowshall, Jonathan Vallance et al
FORRESTER & BOEHMERT
Pettenkoferstrasse 20-22
80336 München (DE)**

(56) References cited:
**WO-A-98/52598**

- **VAN DE VEN CARMELLA ET AL: "The
haemopoietic effects of thrombopoietin
administered post-myelosuppressive
carboplatin therapy compared to either pre- and
post- or pre-chemotherapy." BRITISH JOURNAL
OF HAEMATOLOGY, vol. 102, no. 3, August 1998
(1998-08), pages 775-782, XP000939371 ISSN:
0007-1048**

- **VADHAN-RAJ S ET AL: "Enhanced retention of
in vitro functional activity of rhTPO-induced
platelets following cryopreservation with
ThrombosolTM and 2 percent DMSO." BLOOD,
vol. 90, no. 10 SUPPL. 1 PART 1, 15 November
1997 (1997-11-15), page 38A XP000939382 39th
Annual Meeting of the American Society of
Hematology;San Diego, California, USA;
December 5-9, 1997 ISSN: 0006-4971**
- **VADHAN-RAJ SAROJ: "Recombinant human
thrombopoietin: Clinical experience and in vivo
biology." SEMINARS IN HEMATOLOGY, vol. 35,
no. 3, July 1998 (1998-07), pages 261-268,
XP000939399 ISSN: 0037-1963**
- **VADHAN-RAJ SAROJ ET AL: "Enhanced
retention of in vitro functional activity of
platelets from recombinant human
thrombopoietin-treated patients following
long-term cryopreservation with a
platelet-preserving solution (ThromboSol) and
2% DMSO." BRITISH JOURNAL OF
HAEMATOLOGY, vol. 104, no. 2, February 1999
(1999-02), pages 403-411, XP000939372 ISSN:
0007-1048**
- **Declaration of Saroj Vadhan-Raj (14.06.02) and
Exhibit 1-4**

Note: Within nine months from the publication of the mention of the grant of the European patent, any person may give
notice to the European Patent Office of opposition to the European patent granted. Notice of opposition shall be filed in
a written reasoned statement. It shall not be deemed to have been filed until the opposition fee has been paid. (Art.
99(1) European Patent Convention).

SEQUENCE LISTING

<110> Vadhan-Raj, Saroj

<120> METHODS FOR INCREASING CIRCULATING PLATELETS FOR COLLECTING AND CRYOPRESERVATION USING THROMBOPOIETIN COMPOSITIONS

<130> UTXC:601

<140> UNKNOWN
<141> 1999-02-04

<160> 2

<170> PatentIn Ver. 2.0

<210> 1
<211> 1774
<212> DNA
<213> HOMO SAPIENS

```
<220>
<221> CDS
<222> (216).. (1274)

<400> 1


        tcttcctacc catctgctcc ccagagggct gcctgctgtg cacttgggtc ctggagccct 60

        tctccacccg gatagattcc tcacccttgg cccgcctttg ccccaccCta ctctgcccag 120

        aagtgcaaga gcctaagccg cctccatggc cccaggaagg attcagggga gaggccccaa 180

        acagggagcc acgccagcca gacaccccgg ccaga atg gag ctg act gaa ttg      233
                                               Met Glu Leu Thr Glu Leu
                                                1               5

        ctc ctc gtg gtc atg ctt ctc cta act gca agg cta acg ctg tcc agc     281
        Leu Leu Val Val Met Leu Leu Leu Thr Ala Arg Leu Thr Leu Ser Ser
                     10                  15                  20

        ccg gct cct cct gct tgt gac ctc cga gtc ctc agt aaa ctg ctt cgt     329
        Pro Ala Pro Pro Ala Cys Asp Leu Arg Val Leu Ser Lys Leu Leu Arg
                 25                  30                  35

        gac tcc cat gtc ctt cac agc aga ctg agc cag tgc cca gag gtt cac     377
        Asp Ser His Val Leu His Ser Arg Leu Ser Gln Cys Pro Glu Val His
                 40                  45                  50

        cct ttg cct aca cct gtc ctg ctg cct gct gtg gac ttt agc ttg gga     425
        Pro Leu Pro Thr Pro Val Leu Leu Pro Ala Val Asp Phe Ser Leu Gly
         55                  60                  65                  70

        gaa tgg aaa acc cag atg gag gag acc aag gca cag gac att ctg gga     473
        Glu Trp Lys Thr Gln Met Glu Glu Thr Lys Ala Gln Asp Ile Leu Gly
                     75                  80                  85

        gca gtg acc ctt ctg ctg gag gga gtg atg gca gca cgg gga caa ctg     521
        Ala Val Thr Leu Leu Leu Glu Gly Val Met Ala Ala Arg Gly Gln Leu
                     90                  95                 100

        gga ccc act tgc ctc tca tcc ctc ctg ggg cag ctt tct gga cag gtc     569
        Gly Pro Thr Cys Leu Ser Ser Leu Leu Gly Gln Leu Ser Gly Gln Val
                    105                 110                 115

        cgt ctc ctc ctt ggg gcc ctg cag agc ctc ctt gga acc cag ctt cct     617
```

```
Arg Leu Leu Leu Gly Ala Leu Gln Ser Leu Leu Gly Thr Gln Leu Pro
    120             125             130

cca cag ggc agg acc aca gct cac aag gat ccc aat gcc atc ttc ctg   665
Pro Gln Gly Arg Thr Thr Ala His Lys Asp Pro Asn Ala Ile Phe Leu
135             140             145                 150

agc ttc caa cac ctg ctc cga gga aag gtg cgt ttc ctg atg ctt gta   713
Ser Phe Gln His Leu Leu Arg Gly Lys Val Arg Phe Leu Met Leu Val
            155             160             165

gga ggg tcc acc ctc tgc gtc agg cgg gcc cca ccc acc aca gct gtc   761
Gly Gly Ser Thr Leu Cys Val Arg Arg Ala Pro Pro Thr Thr Ala Val
            170             175             180

ccc agc aga acc tct cta gtc ctc aca ctg aac gag ctc cca aac agg   809
Pro Ser Arg Thr Ser Leu Val Leu Thr Leu Asn Glu Leu Pro Asn Arg
        185             190             195

act tct gga ttg ttg gag aca aac ttc act gcc tca gcc aga act act   857
Thr Ser Gly Leu Leu Glu Thr Asn Phe Thr Ala Ser Ala Arg Thr Thr
    200             205             210

ggc tct ggg ctt ctg aag tgg cag cag gga ttc aga gcc aag att cct   905
Gly Ser Gly Leu Leu Lys Trp Gln Gln Gly Phe Arg Ala Lys Ile Pro
215             220             225             230

ggt ctg ctg aac caa acc tcc agg tcc ctg gac caa atc ccc gga tac   953
Gly Leu Leu Asn Gln Thr Ser Arg Ser Leu Asp Gln Ile Pro Gly Tyr
            235             240             245

ctg aac agg ata cac gaa ctc ttg aat gga act cgt gga ctc ttt cct
1001
Leu Asn Arg Ile His Glu Leu Leu Asn Gly Thr Arg Gly Leu Phe Pro
            250             255             260

gga ccc tca cgc agg acc cta gga gcc ccg gac att tcc tca gga aca
1049
Gly Pro Ser Arg Arg Thr Leu Gly Ala Pro Asp Ile Ser Ser Gly Thr
        265             270             275

tca gac aca ggc tcc ctg cca ccc aac ctc cag cct gga tat tct cct
1097
Ser Asp Thr Gly Ser Leu Pro Pro Asn Leu Gln Pro Gly Tyr Ser Pro
    280             285             290

tcc cca acc cat cct cct act gga cag tat acg ctc ttc cct ctt cca
1145
Ser Pro Thr His Pro Pro Thr Gly Gln Tyr Thr Leu Phe Pro Leu Pro
295             300             305             310

ccc acc ttg ccc acc cct gtg gtc cag ctc cac ccc ctg ctt cct gac
1193
Pro Thr Leu Pro Thr Pro Val Val Gln Leu His Pro Leu Leu Pro Asp
            315             320             325

cct tct gct cca acg ccc acc cct acc agc cct ctt cta aac aca tcc
1241
Pro Ser Ala Pro Thr Pro Thr Pro Thr Ser Pro Leu Leu Asn Thr Ser
            330             335             340

tac acc cac tcc cag aat ctg tct cag gaa ggg taaggttctc agacactgcc
1294
Tyr Thr His Ser Gln Asn Leu Ser Gln Glu Gly
        345             350
```

```
gacatcagca ttgtctcatg tacagctccc ttccctgcag ggcgcccctg ggagacaact
1354

ggacaagatt tcctactttc tcctgaaacc caaagccctg gtaaaaggga tacacaggac
1414

tgaaaaggga atcatttttc actgtacatt ataaaccttc agaagctatt tttttaagct
1474

atcagcaata ctcatcagag cagctagctc tttggtctat tttctgcaga aatttgcaac
1534

tcactgattc tctacatgct ctttttctgt gataactctg caaaggcctg ggctggcctg
1594

gcagttgaac agagggagag actaaccttg agtcagaaaa cagagaaagg gtaatttcct
1654

ttgcttcaaa ttcaaggcct tccaacgccc ccatcccctt tactatcatt ctcagtggga
1714

ctctgatccc atattcttaa cagatcttta ctcttgagaa atgaataagc tttctctcag
1774
```

<210> 2
<211> 353
<212> PRT
<213> HOMO SAPIENS

<400> 2

```
Met Glu Leu Thr Glu Leu Leu Leu Val Val Met Leu Leu Leu Thr Ala
 1               5                  10                  15

Arg Leu Thr Leu Ser Ser Pro Ala Pro Pro Ala Cys Asp Leu Arg Val
            20                  25                  30

Leu Ser Lys Leu Leu Arg Asp Ser His Val Leu His Ser Arg Leu Ser
            35                  40                  45

Gln Cys Pro Glu Val His Pro Leu Pro Thr Pro Val Leu Leu Pro Ala
        50              55                  60

Val Asp Phe Ser Leu Gly Glu Trp Lys Thr Gln Met Glu Glu Thr Lys
 65              70                  75                  80

Ala Gln Asp Ile Leu Gly Ala Val Thr Leu Leu Leu Glu Gly Val Met
                85                  90                  95

Ala Ala Arg Gly Gln Leu Gly Pro Thr Cys Leu Ser Ser Leu Leu Gly
            100                 105                 110

Gln Leu Ser Gly Gln Val Arg Leu Leu Leu Gly Ala Leu Gln Ser Leu
            115                 120                 125

Leu Gly Thr Gln Leu Pro Pro Gln Gly Arg Thr Thr Ala His Lys Asp
        130             135                 140

Pro Asn Ala Ile Phe Leu Ser Phe Gln His Leu Leu Arg Gly Lys Val
145             150                 155                 160

Arg Phe Leu Met Leu Val Gly Gly Ser Thr Leu Cys Val Arg Arg Ala
            165                 170                 175

Pro Pro Thr Thr Ala Val Pro Ser Arg Thr Ser Leu Val Leu Thr Leu
            180                 185                 190
```

```
Asn Glu Leu Pro Asn Arg Thr Ser Gly Leu Leu Glu Thr Asn Phe Thr
        195             200             205

Ala Ser Ala Arg Thr Thr Gly Ser Gly Leu Leu Lys Trp Gln Gln Gly
        210             215             220

Phe Arg Ala Lys Ile Pro Gly Leu Leu Asn Gln Thr Ser Arg Ser Leu
225             230             235             240

Asp Gln Ile Pro Gly Tyr Leu Asn Arg Ile His Glu Leu Leu Asn Gly
            245             250             255

Thr Arg Gly Leu Phe Pro Gly Pro Ser Arg Arg Thr Leu Gly Ala Pro
            260             265             270

Asp Ile Ser Ser Gly Thr Ser Asp Thr Gly Ser Leu Pro Pro Asn Leu
            275             280             285

Gln Pro Gly Tyr Ser Pro Ser Pro Thr His Pro Pro Thr Gly Gln Tyr
        290             295             300

Thr Leu Phe Pro Leu Pro Pro Thr Leu Pro Thr Pro Val Val Gln Leu
305             310             315             320

His Pro Leu Leu Pro Asp Pro Ser Ala Pro Thr Pro Thr Pro Thr Ser
            325             330             335

Pro Leu Leu Asn Thr Ser Tyr Thr His Ser Gln Asn Leu Ser Gln Glu
            340             345             350

Gly
```

**Description**

## BACKGROUND OF THE INVENTION

The government owns rights in the present invention pursuant to grant number RO1 HL56416. RO1 HL54037 and PO1 HL53586 from the National Heart, Lung, and Blood Institute and U.S. Navy Contract N00014-96-C-0120.

### 1. Field of the Invention

The present invention also relates to the use of thrombopoietin for treating a patient having or at risk for thromocytopenia.

### 2. Description of Related Art

Thrombocytopenia is an important clinical problem in the management of patients in hematology and oncology practices. In particular, myelosuppression is one of the most serious complications in cancer patients receiving cytotoxic treatment. Thrombocytopenia has been predominantly managed by platelet transfusions and chemotherapy dose-modification. In the United States, the use of platelet transfusions to manage severe thrombocytopenia has steadily increased. Approximately 4 million units were transfused in 1982, and more than 8 million units were transfused in 1992 (Surgenor *et al.,* 1990; Wallace *et al.,* 1992). The use of single donor apheresis platelets has increased nearly 6-fold, mainly due to concerns related to the potential risks of transmission of infectious agents and alloimmunization (Hayman and Schiffer, 1990; Schiffer, 1997). This marked increase in the need for platelets has paralleled advances in organ transplantation, bone marrow transplantation, cardiac surgery, and the use of dose-intensive therapy in the treatment of chemosensitive malignant conditions. While platelet transfusions can reduce the risk for hemorrhagic complications, their repeated usage can increase the risk for transfusion reactions. alloimmunization, and transmission of infectious agents, and contributes to increased health care costs (Heyman and Schiffer, 1990). This enormous increase in the need for platelets has imposed a chronic burden on patients, health care systems and blood banking resources, and has motivated a search for improved methods of procurement, processing and storage of platelets, and for novel agents to stimulate production of platelets.

Over the past decade, the recognition that the myeloid growth factors control the production of neutrophils (G-CSF and GM-CSF) and erythrocytes (erythropoietin) has led to the clinical application of these factors for the management of patients with cancer who are undergoing myelosuppressive therapy (Nemunaitis *et al.,* 1991; Crawford *et al.,* 1991; Vose *et al.,* 1991; Am. Soc. of Clin. Oncol., 1994). The use of myeloid growth factors such as granulocyte colony-stimulating factor (G-CSF) and granulocyte-macrophage colony-stimulating factor (GM-CSF) has reduced the incidence of febrile neutropenia (Crawford *et al.,* 1991; Neumunaitis *et al.,* 1991). Several cytokines with thrombopoietic potential have undergone clinical evaluation, including Interleukin-1, Interleukin-3, Interleukin-6, Interleukin-11, and PIXY321 (Smith *et al.,* 1992; Vadhan-Raj *et al.,* 1994; D'Hondt *et al.,* 1993; Weber *et al.,* 1993; Tepler *et* al., 1996; Vadhan-Raj *et al.,* 1994) The clinical development of a number of these agents have been limited by their modest thrombopoietic activity and/or a toxicity profile unfavorable for a supportive agent. IL-11 is the only thrombopoietic cytokine that has been approved for clinical use, to date. In the randomized, placebo-controlled study, the use of IL-I (50mcg/kg) as a secondary prophylaxis was shown to decrease the likelihood of avoiding platelet transfusion in 30% (8 of 27) of patients (as compared to 4% on placebo) who had already been transfused platelets for thrombocytopenia ($\leq$20,000/mm$^3$) resulting from various chemotherapy regimens. Although, the duration of thrombocytopenia was reduced, it was not statistically significant. The significant side effects reported with IL-11 included atrial arrhythmias, syncope, dyspnea and edema.

Thrombopoietin, the ligand for the c-Mpl receptor (found on platelets and megakaryocyte progenitors), was recently cloned by several investigators and was shown to be a primary regulator of platelet production *in vivo* (de Sauvage *et al.*, 1994; Bartley *et al.,* 1994; Lok *et al.,* 1994; Kaushansky *et al.,* 1994; Wendling *et al.,* 1994). TPO is a growth factor that acts directly on early bone marrow stem cells and megakaryocyte progenitor cells (de Sauvage *et al.,* 1994; Lok *et al.,* 1994; Kaushansky *et al.,* 1994; Wendling *et al.,* 1994; Bartley *et al.,* 1994; Kuter and Rosenberg, 1994; Souyri *et al.,* 1990). TPO induces proliferation and differentiation to produce increased numbers of mature megakaryocytes, resulting in increased numbers of platelets in the circulation. The action of TPO on proliferation and differentiation appears to involve the JAK/STAT family of intracellular signaling molecules (Gruney *et al.,* 1995).

In preclinical studies done in normal mice and nonhuman primates, thrombopoietin increased platelet counts to a level higher than those previously achieved with other thrombopoietin cytokines (Kaushansky, 1995; Farese et *al.,* 1995). Moreover, in a murine model for myelosuppression, recombinant thrombopoietin given as a single dose decreased the nadir and accelerated platelet recovery in mice that had been rendered pancytopenic by sublethal radiation and chemotherapy (Thomas *et al.,* 1996). In these studies, more prolonged treatment (for as long as 8 days) provided no additional benefit and was associated with marked thrombocytosis during the recovery phase.

Early results of the trials using thrombopoietin (Vadhan-Raj *et al.,* 1996) as well as a polyethylene glycol-conjugated form of the molecule (Fanucchi *et al.,* 1997; Levin, 1997) demonstrate clinical activity. rhTPO has a prolonged half life (20 to 30 h) with a delayed peak response (median day 12) (Vadhan-Raj *et al.,* 1997; Bloedow *et al.,* 1996). Early clinical trials demonstrated that administration of both the full length molecule (termed recombinant human thrombopoietin [rhTPO]) or the truncated, pegylated version of the molecule (termed megakaryocyte growth and development factor [PEG-rHuMGDF]) resulted in a dose-related increase in circulating platelet counts prior to chemotherapy and enhanced platelet recovery to baseline following moderately myelosuppressive regimens (Fanucchi *et al.,* 1997; Vadhan-Raj et al., 1997; O'Malley *et al.,* 1996; Basser *et al.,* 1997; Suzuki *et al.,* 1995). However, previous studies with growth factors indicate the critical importance of the timing of growth factor administration in relation to chemotherapy to achieve maximal clinical benefit (Vadhan-Raj *et al.,* 1992; Neidhart *et al.,* 1992; Crawford et *al.,* 1992). The value of these agents in preventing severe thrombocytopenia and averting the need for platelet transfusions has not been established.

The transfusion of autologous platelets may overcome many problems including alloimmunization, risk of transmission of infectious agents, and a chronic supply crisis for blood banks. Prior experience with plateletpheresis of normal donors indicate that intensive multiunit plateletpheresis is a safe and practical means of obtaining large number of platelets for transfusion (Schiffer *et al.,* 1976; Schiffer *et al.,* 1974). Under current blood banking practices, platelets can be stored at 22°C for only 5 days due to the potential risk of bacterial contamination (Lazarus *et al.,* 1982; Murphy, 1985; Owens *et al.,* 1992). Alternatively, donated platelets can be cryopreserved to achieve a longer storage time. Unfortunately, the currently approved methods for platelet cryopreservation are labor intensive and require a high concentration of cryoprotectant. DMSO (5 to 6%). Furthermore, cryopreservation of platelets by this method leads to the loss of cell number and functional activity (Daly *et al.,* 1979; Balduini *et al.,* 1993; Bock *et al.,* 1995; Towell *et al.,* 1986). The recovery of platelets following freeze-thaw procedure is typically 50 to 70%. Following transfusion of these platelets, the recovery decreases to 20 to 30% of the original platelet number. In addition, the cryopreserved platelets exhibit elevated expression of activation marker CD-62, and a significant decrease in functional parameters. These detrimental effects of cryopreservation on platelets are due to the induction of a storage lesion that is thought to result from biochemical activation of platelets *via* second messenger pathways. ThromboSol is a newly developed platelet storage solution which consists of selected second messenger effectors that inhibit specific activation pathways endogenous to platelets, resulting in cells that are biochemically stabilized and protected against the cold storage lesions.

With the concept that increased dose-intensity might be important to improve the overall outcome, there has been an use of more aggressive therapy in chemosensitive malignancy. These considerations have led to the search for agents or methods that can attenuate thrombocytopenia and reduce the need for platelet transfusions. Additionally. agents or methods that can increase platelet production. and allow better storage of platelets for transfusions, would be an important advance.

## SUMMARY OF THE INVENTION

The present disclosure overcomes these and other drawbacks inherent in the prior art by providing novel uses of thrombopoietin (TPO) also known as MGDF. mpl ligand, and megapoietin for the manufacture of a medicament for enhancing platelet production in an animal. These uses of TPO can be used to ameliorate platelet reduction in an animal due to agent that causes or enhances thrombocytopenia.

The invention next provides a composition comprising TPO for the manufacture of a medicament for treating a mammal having or at risk of thrombocytopenia wherein the treatment is to comprise the administration to a mammal of an agent which can cause thromobocytopenia: administration to the mammal of one or more priming doses of a composition comprising TPO prior to administration of the agent; and admininstration to the mammal of two or more post-doses of a TPO composition after administration of the agent. In a preferred embodiment, the the priming or post-doses are to be administered other than daily. Herein certain embodiments, other than daily may mean about 2. about 3, about 4, about 5, about 6, about 7, about 8, about 9, to about 10 or more days between administrations of composition comprising TPO. In a particularly preferred embodiment, the doses are to be administered every other day. Multiple doses of TPO may be administered whenever the patient has or is at risk for thrombocytopenia. As used herein certain embodiments, "having thrombocytopenia" means an animal has less than about 100,000 platelets per μL blood. As such, within this definition, it would be understood that an animal having about 95,000, about 90,000, about 85,000, about 75,000, about 70,000, about 65.000, about 60,000. about 55.000. about 50,000, about 45,000, about 40,000, about 35,000, about 30,000, about 25,000, about 20,000, about 15,000, about 10,000, about 5,000, to about 1,000 platelets per μL blood or less would also have thrombocytopennia. Of course, in light of this definition and the disclosures herein, a skilled artisan would be able to diagnose an an animal as having thrombocytopenia so that the absolute number of platelets per μL would vary from the listed values. As used herein certain embodiments, "at risk for thrombocytopenia" means an animal is forseeably expected to develop thrombocytopenia due to a disease condition, treatment, or exposure to an agent known or suspected of reducing the number of platelets per µL blood of an animal. It is contemplated that a patient may receive doses comprising TPO during extended periods of or expected thrombocytopenia. Acute periods may last hours, days and/or weeks. It is also contemplated that a patient may receive doses of TPO on a semi-permanant basis, if the thrombocytopenia or the risk for thrombocytopenia is chronic. These chronic periods may last months or years, and TPO may be administered intermittent basis as needed to reduce thrombocytopenic conditions. One of skill in the art would be able to ascertain the need to administration of a composition comprising TPO over extended, semi-permanent periods of time based on the severity and persistence of a condition, disease, risk of exposure to an agent that causes, aggravates, or enhances thrombocytopenia, or response of mammal to the methods of the invention in treating thrombocytopenia. Thus, at least about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 40, about 45, about 50, about 55, about 55, about 60, about 65, about 70, about 75, about 80, about 85, about 90, about 95, about 100, about 110, about 120, about 130, about 140, about 150, about 160, about 170, about 180, about 190, about 200, about 210, about 220, about 230, about 240, about 250, to about 300 or more doses comprising TPO may be administered in the methods of the invention. Preferred doses for conditions including, but not limited to, cyclic thrombytopenia, chronic myodisplasia, aplasitc anemia, or bone marrow disorders, would be about 10 to about 150 doses, and, of course, all numbers between as described above, of a composition comprising TPO. However, it is contemplated that for many applications, such as a cycle of chemotherapy or radiation therapy, the total number of doses of TPO to be administered would preferably be less than about 20 doses. Doses may be administered to a patient until the recovery platelet count is about 50.000 platelets per µL blood or greater before discontinuing administration of TPO doses. A skilled clinician, however, would understand that TPO may be administered until the mammal's platelet count is about 55,000, about 60,000, about 65,000, about 70,000, about 75,000, about 80,000, about 85,000, about 90,000, about 95,000, to about 100.000 platelets per µL blood or so before discontinuing administration of TPO doses. Of course, in light of this description and the disclosures herein, a skilled artisan would be able to diagnose an animal as having alleviated the conditions of thrombocytopenia so that the absolute number of platelets per µL would vary from the listed values when the decision of discontinuation of administration of TPO is made. Additionally, TPO should be administered again should the patient's develop thrombocytopenia, particularly if the mammal's platelet count drops below 50,000 platelets per µL blood or so, or if the mammal is otherwise diagnosed as having thrombocytopenia. One of skill in the art will recognize that a lesser amount of TPO administered in each dose would be preferable as the total number of consecutive doses increases. The adjustments of dose levels to obtain optimum effect on platelet levels in a patient would be a routine matter in light of the present disclosures. It would be preferred that the doses are to be administered other than daily. In another preferred embodiment. the doses are to be administered every other day.

In one preferred embodiment, the agent is a chemotherapy agent or radiation therapy. In this embodiment, it is expected that the exposure to the agent would produce an acute period of thrombocytopenia. It is contemplated that at least about 2, about 3, about 4, about 5, to about 6 priming doses or more are to be administered. In one aspect of the invention, at least about 2, about 3, about 4, about 5, about 6, about 7, about 8, about 9, to about 10 post-doses of the TPO composition are to be admininstered. In certain aspects of the invention, at least about 2, about 3, about 4, about 5 about 6, about 7, about 8, about 9, about 10, about 11, to about 12 total doses of the TPO composition are to be administered. In a preferred embodiment, the priming-doses are to be administered other than daily. In another preferred embodiment, the doses are to be administered every other day.

In one preferred aspect the total number of priming or pre-doses may be about 3 to about 6 doses. In a preferred aspect, the total number of post-doses would be about 4 to about 8 doses. In another prefered aspect. when both pre- and post doses are to be administered, the total number of doses would be about 4 doses to about 10 doses. As used herein, unless specified as a pre- or priming dose, "doses" of TPO may refer to pre- or post doses.

The invention further provides a composition comprising TPO for the manufacture of a medicament for treating a mammal having or at risk of thrombocytopenia, wherein the treatment is to comprise the administration to a mammal of an agent which can cause early-radio thromocytopenia; and administration to the mammal of one or more priming doses of a composition comprising TPO prior to administration of the agent. In one aspect of the invention, priming doses may be about 1, about 2, about 3, about 4, about 5, and up to about 6 or more priming-doses of the TPO composition are to be administered. In a preferred embodiment, the priming-doses are administered other than daily. In another preferred embodiment, the doses are to be administered every other day.

There is disclosed the use of a composition comprising TPO for the manufacture of a medicament for treating a human having or at risk of thrombocytopenia, wherein the treatment is to comprise the steps of:

(a) administration to a human of an agent which can cause thromobocytopenia; and

(b) administration to the human of two or more post-doses of a composition comprising TPO after administering the agent, wherein the post-doses are administered other than daily,

wherein the post-doses may be about 2, about 3, about 4, about 5, about 6, about 7, about 9, about 10, about 11, about 12, about 13, about 14, to about 15 doses. Alternatively, in post chemotherapy TPO doses may be administered to a patient until the recovery (post nadir) platelet count is about 50,000 to about 100,000 per µL blood before discontinuing administration of TPO doses. TPO should be administered again should the patient's platelet count drop below about 50,000 per µL blood, or the animal develops thrombocytopenia as would be understood by a skilled artisan in light of the present disclosures. In a preferred aspect, the doses are to be administered other than daily. In another preferred aspect, the post doses are to be administered every other day.

In certain aspects of the method of the disclosure described in the paragraphs above, the agent may be administered of from about substantially simultaneously (*i.e.* within less than about a minute) as the administration as a compostion comprising TPO. In other aspects, the agent may be administerered within of from about 1 minute, about 5 minutes, about 10 minutes, about 20 minutes about 30 minutes, about 45 minutes, about 60 minutes, about 2 hours, about 3 hours, about 4 hours, about 5 hours, about 6 hours, about 7 hours about. 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 22 hours, about 23 hours, about 24 hours, about 25 hours, about 26 hours, about 27 hours, abour 28 hours, about 29 hours, about 30 hours, about 31 hours, about 32 hours, about 33 hours, about 34 hours, about 35 hours, about 36 hours, about 37 hours, about 38 hours, about 39 hours, about 40 hours, about 41 hours, about 42 hours, about 43 hours, about 44 hours, about 45 hours, about 46 hours, about 47 hours, about 48 hours, about 49 hours, about 50 hours, about 51 hours, about 52 hours, about 53 hours, about 54 hours, about 55 hours, about 56 hours, about 57 hours, about 58 hours. about 59 hours. about 60 hours, about 61 hours, about $6^2$ hours, about 63 hours, about 64 hours, about 65 hours, about 66 hours, about 67 hours, about 68 hours, about 69 hours, about 70 hours, about $7^1$ hours, about $7^2$ hours, about $7^3$ hours, about 74 hours, about 75 hours, about 76 hours, about 77 hours, about 78 hours, about 79 hours, about 80 hours, about 81 hours, about 82 hours, about 83 hours, about 84 hours, about 85 hours, about 86 hours, about 87 hours, about 88 hours, about 89 hours, about 90 hours, about 91 hours, about 92 hours, about 93 hours, about 94 hours, about 95 hours, about 96 hours, about 97 hours, about 98 hours, about 99 hours, to about 100 hours or more prior to or after the administration of a therapeutic composition comprising TPO. In certain other embodiments, the agent may be administerered within of from about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20, to about 21 days prior to the admininstration of a therapeutic composition comprising TPO. In certain other embodiments, the agent may be administerered within of from about 1 day, about 2 days, about 3 days, about 4 days, about 5 days, about 6 days, about 7 days, about 8 days, about 9 days, to about 10 days after the administration of a therapeutic composition comprising TPO. In some embodiments, the mammal is to be administered more than one therapeutic composition comprising TPO. In other embodiment, a therapeutic composition is to be administered as a single dose comprising TPO. In other embodiments more than one dose of a therapeutic composition comprising TPO is to be administered.

In certain aspects of the invention, each dose is to be administered of from about 24 to about 72 hours after a preceding dose comprising TPO. In some embodiments, each dose is to be administered of from about 36 to about 60 hours after a preceding dose comprising TPO. In some embodiments, each dose is to be administered of from about 42 to about 54 hours after a preceding dose comprising TPO. In some embodiments, each dose is to be administered about 48 hours after a preceding dose comprising TPO. In some preferred embodiments, each dose is to be administered every other day after a preceding dose comprising TPO. In other preferered embodiments, each dose is to be administered other than daily after a preceeding dose comprising TPO. One of ordinary skill in the art will recognize that the dose schedule may vary to combine the various aspects described above in the timing of administration. For example, a second dose may be administered about 36 to about 60 hours after a preceeding dose, and a subsequent dose may be administered about 48 hours after the second dose. Such modifications are encompassed by the present invention, as would be practiced by one of ordinary skill in the art in light of the disclosures herein.

In other aspects of the invention, the nadir or point of lowest platelet count after treatment with an agent is relatively early, of from about 7, about 8, about 9, about 10, about 12, about 13 to about 14 days. In certain embodiments, the nadir of platelet count is about 10 to about 14 days, with the median nadir being about 12 days after treatment with an agent. In these embodiments, it is preferred that the agent is first to be administered after the administration of the therapeutic composition comprising TPO. In this aspect, it is preferred that more than one dose of the therapeutic composition comprising TPO is to be administered. In this aspect, it is preferred that each dose is to be administered every other day after a preceding dose of the therapeutic composition comprising TPO. It is particularly preferred that about 3 to about 6 doses are to be administered, with about 3 to about 4 doses considered especially preferred. In this aspect, it is also preferred that one dose to about 8 comprising TPO is to be administered after administration of the agent. In this embodiment, about 1, about 2, about 3, about 4, about 5, to about 6 doses or so may be administered. In one aspect, the composition comprising TPO is to be administered when there is a platelet count of less than about 100,000/per µL blood.

In other aspects of the invention, the agent is to be administered over a period of from about 3 to about 6 days. In this embodiment, it is preferred that the agent is first to be administered after the therapeutic composition comprising TPO. In this embodiment, it is also preferred that more than one dose of the therapeutic composition comprising TPO is to be administered. It is preferred that each dose is to be administered every other day after a preceding dose of the therapeutic composition comprising TPO. In this aspect, it is preferred that each dose is to be administered every other day after a preceding dose of the therapeutic composition comprising TPO. It is particularly preferred that about 3 to about 6 doses are to be administered, with 4 doses considered especially preferred. In this embodiment, about 1, about 2, about 3, about 4, about 5, to about 6 doses or so may be administered. In this aspect, it is preferred that one dose comprising TPO is to be administered after administration of the agent. In one aspect, the composition comprising TPO is to be administered when there is a platelet count of less than about 100,000/per µL blood.

In other aspects of the invention, the nadir of the agent is relatively late, of from about 15, about 16, about 17, about 18, about 19, about 20, about 21, about 22, about 23, about 24, about 25, about 26, about 27, about 28, about 29, about 30, about 31, about 32, about 33, about 34, to about 35 days or more. In this aspect, it is preferred that the agent is to be administered prior to the administration of the therapeutic composition comprising TPO. However, it is contemplated that agents with early nadirs, such as from about day 12 to about 25 days may also be preferred in this embodiment. It is preferred that more than one dose comprising TPO is to be administered. In this aspect, it is preferred that each dose is to be administered every other day after a preceding dose of the therapeutic composition comprising TPO. It is particularly preferred that about 3 to about 6 doses are to be administered, with about 3 to about 4 doses considered especially preferred. In this embodiment, about 1, about 2, about 3. about 4, about 5, to about 6 doses or so may be administered. In one aspect, the composition comprising TPO is to be administered when there is a platelet count-of less than about 100,000/per µL blood.

In other aspects of the invention, the agent is to be administered over a period of from about 1 to about 5 days, or more preferably about 1 to 2 days. In this embodiment, it is preferred that the agent is first to be administered prior the administration of the therapeutic composition comprising TPO. It is preferred that more than one dose comprising TPO is to be administered. In this aspect, it is preferred that each dose is to be administered every other day after a preceding dose of the therapeutic composition comprising TPO. It is particularly preferred that about 3 to about 6 doses are to be administered, with about 4 doses to about 6 doses considered especially preferred. In this embodiment, about 1, about 2, about 3, about 4, about 5, about 6, about 7, about 8, about 9, to about 10 doses or so may be administered. In this aspect, it is preferred that at least one dose comprising TPO is to be administered after administration of the agent. In one aspect, the composition comprising TPO is to be administered when there is a platelet count of less than about 100.000/per µL blood.

In certain embodiments, pre-doses, post doses, and combinations thereof may be administered when the nadir is intermediate, of say from about 12, about 13, to about 14 days. In this embodiment, the preferred use may be that described for either early nadir agents or late nadir agents.

In certain aspects of the invention, a cycle of treatment comprises multiple administrations of the agent to a mammal. In other aspects, the cycle of treatment comprises treatment of the mammal with more than one agent known to induce thrombocytopenia. The mammal may be treated in one or more cycles. or multiple cycles wherein an agent is administered over of from about 1, about 2. about 3, about 4, about 5, about 6, about 7, about 8, about 9, about 10, about 11, about 12, about 13, about 14, about 15, about 16, about 17, about 18, about 19, to about 20 or more days per cycle. The agent may be administered continuously, daily, or other than daily in its schedule of administration. More than one agent may be administered in a treatment schedule or cycle. In one embodiment, it is preferred that the agent is to be administered of from about I to about 5 days total per cycle. Thus, in this aspect, the agent may be administered about 1, about 2, about 3, about 4, or about 5 days or so per cycle.

In certain aspects of the invention, a therapeutic compostion comprising TPO may be a TPO protein, polypeptide, and/or peptide isolated from a human or another animal. The TPO protein, polypeptide, and/or peptide may be a protein produced by recombinant DNA technology, or may be chemically synthesized, and may be modified by any of the techniques known to those of skill in the art to alter or modify a wild type sequence of a TPO protein, polypeptide, and/or peptide while leaving some or all of its thrombopoietic and/or cryopreservative activity. Such modified TPO molecules include, but are not limited to, a PEGylated TPO molecule. A composition of TPO may comprise a combination of both modified and unmodified TPO protein, polypeptide, and/or peptide, and may even incorporate inactive forms of TPO. As used herein, the terms "TPO" or "thrombopoietin" may refer to any of these compositions described in the preceding passages of this paragraph. It is contemplated that other peptides, proteins, or polypeptides may mimic the activity of TPO by action as a thrombopoietin receptor agonist. Such TPO activity peptide mimics of TPO activity or thrombinpoietin receptor agonists include, but are not limited to, the peptides disclosed by Cwirla *et al,* 1997. It is contemplated that such a TPO peptide peptide mimic or receptor agonist may be used in any of the compositions and uses disclosed herein. Additionally, it is contemplated that such a receptor agonist or TPO activity peptide mimic or other receptor agonist may be produced, isolated, synthesized, altered, or otherwise modified by any of the techniques disclosed herein or would be known by those of ordinary skill in the art and still retain its thrombopoietic and/ or cyropreservative activity.

As used herein in the context of various of the instant compositions and methods, the term "protein" will be understood to mean a proteinaceous segment that is longer than about 201 contiguous amino acids and in most aspects comprises more that about 70% of the amino acids encoded by the coding regions of a gene. Thus, a TPO protein may be about 201, about 202, about 203, about 204, about 205, about 206, about 207, about 208, about 209, about 210, about 211, about 212, about 213, about 214, about 215, about 216, about 217, about 218, about 219, about 220. about 221, about 222, about 223, about 224, about 225, about 226, about 227, about 228, about 229, about 230, about 231, about 232, about 233, about 234, about 235, about 236, about 237, about 238, about 239, about 240, about 241, about 242, about 243, about 244, about 245, about 246, about 247, about 248, about 249, about 250, about 251, about 252, about 253, about 254, about 255, about 256, about 257, about 258, about 259, about 260, about 261, about 262, about 262, about 263, about 264, about 265, about 266, about 267, about 268, about 269, about 270, about 271, about 272, about 273, about 274, about 275, about 276, about 277, about 278, about 279, about 280, about 281, about 282, about 283, about 284, about 285, about 286, about 287, about 288, about 289, about 290, about 291, about 292, about 293, about 294, about 295, about 296. about 297, about 298, about 299, about 300, about 301, about 302, about 303, about 304, about 305, about 306, about 307, about 308, about 309, about 310, about 311, about 312, about 313, about 314, about 315, about 316, about 317, about 318. about 319, about 320, about 321 to about 322 contiguous amino acids in length. A preferred TPO protein comprises the sequence of SEQ ID NO:1. As used herein in the context of various of the instant compositions and methods, the term "polypeptide" will be understood to mean a proteinaceous segment that is between about 100 and about 200 contiguous amino acids in length. Thus, a TPO polypeptide may be about 101, about 102, about 103, about 104, about 105, about 106, about 107, about 108, about 109, about 110, about 111, about 112, about 113, about 114, about 115, about 116. about 117, about 118. about 119, about 120, about 121, about 122, about 123, about 124, about 125, about 126, about 127, about 128, about 129, about 130, about 131, about 132, about 133, about 134, about 135, about 136, about 137, about 138, about 139, about 140, about 141, about 142, about 143, about 144, about 145, about 146, about 147, about 148, about 149, about 150, about 151, about 152, about 153, about 154, about 155, about 156, about 157, about 158, about 159, about 160, about 161, about 162, about 162, about 163, about 164, about 165, about 166, about 167, about 168, about 169, about 170, about 171, about 172, about 173, about 174, about 175, about 176, about 177, about 178, about 179, about 180, about 181, about 182, about 183, about 184, about 185, about 186, about 187, about 188, about 189, about 190, about 191, about 192, about 193, about 194, about 195, about 196, about 197, about 198, about 199, to about contiguous 200 amino acids in length. A preferred polypeptide is a polypeptide comprising the first N-terminal 153 amino acids of mature human TPO. A particularly preferred polypeptide comprises residues 1 to 153 of SEQ IN NO:1. The term "peptide" will be understood to mean a proteinaceous segment that is between about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, about 50, about 51, about 52, about 53, about 54, about 55, about 56, about 57, about 58, about 59, about 60, about 61, about 62, about 63, about 64, about 65, about 66, about 67, about 68, about 69, about 70, about 71, about 72, about 73, about 74, about 75, about 76, about 77, about 78, about 79, about 80, about 81, about 82, about 83, about 84, about 85, about 86, about 87, about 88, about 89, about 90, about 91, about 92, about 93. about 94, about 95, about 96. about 97, about 98. about 99, to about 100 contiguous amino acids in length. Thus, TPO proteinaceous segments of varying overall length that retain regulatory or thrombopoietic properties and functions are contemplated as being useful in the uses and compositions disclosed herein. One of ordinary skill in the art will recognize that amino acid sequences may be added, mutated, altered, modified, and/or removed in the production of a recombinant (rTPO) peptide, polypeptide, or protein, and an active TPO molecule that promotes thrombopoietic activity or cryopreservative activity will be produced for use in the present invention. Methods for producing such variants are described herein.

In certain aspects of the invention, the route the therapeutic composition is to be administered may be by parenteral administration. The parenteral administration may be intravenous injection, subcutaneous injection, intramuscular injection. intramedullary injection or a combination thereof. In certain aspects, the composition comprising TPO is to be administered from about 0.1 to about 10 microgram/kg/body weight per dose. In certain aspects, the composition comprising TPO is to be administered from about 1 to about 5 microgram/kg/body weight per dose. In certain aspects, the composition comprising TPO is to be administered from about 1.2 to about 3.6 microgram/kg/body weight per dose. In certain aspects, the composition comprising TPO is to be administered from about 1.2 to about 2.4 microgram/kg/body weight per dose. In preferred aspects, the amount of TPO administered per dose may be about 0.1, about 0.2, about 0.3, about 0.4, about 0.5, about 0.6, about 0.7, about 0.8, about 0.9, about 1.0, about 1.1, about 1.2, about 1.3, about 1.4, about 1.5, about 1.6, about 1.7, about 1.8, about 1,9, about 2.0, about 2.1. about 2.2, about 2.3, about 2.4, about 2.5, about 2.6, about 2.7, about 2.8. about 2.9, about 3.0, abot 3.1, about 3.2, about 3.3, about 3.4, about 3 5, about 3.6, about 3.7, about 3.8, about 3.9, about 4.0, about 4.1, about 4.2, about 4.3, about 4.4, about 45, about 4.6, about 4.7, about 4.8, abotu 4.9, about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, about 6.5, about 6.6, about 6.7, about 6.8, about 6.9, about 7.0, about 7.1, about 7.2, about 7.3, about 7.4, about 7.5, about 7.6, about 7.7, about 7.8, about 7.9, about 8.0, about 8.1, about 8.2, about 8.3, about 8.4, about 8.5, about 8.6, about 8.7, about 8.8, about 8.9, about 9.0, about 9.1, about 9.2, about 9.3, about 9.4, about 9.5, about 9.6. about 9.7, about 9.8. about 9.9. to about 10.0 or more microgram/kg/body.

In certain aspects of the invention, the agent known to induce thrombocytopenia is a myelosuppressive treatment, myelodysplasia, aplastic anemia, congenital thrombocytopenia, immune thrombocytopenia, cyclic thrombocytopenia, a chemical, exposure to radiation or a combination thereof. In certain aspects, the chemical may be a drug or poison. In certain aspects, the myelosuppressive treatment may be a cytotoxic agent used for progenitor cell mobilization therapy and leukapheresis procedure, chemotherapy or radiation therapy, or a combination thereof. In certain preferred aspects, the myelosuppressive treatment may be a cytotoxic agent used for progenitor cell mobilization therapy and leukapheresis procedure. The cytotoxic agent used for progenitor cell mobilization therapy and leukapheresis procedure may be for a stem cell transplant. In particularly preferred aspects, the myelosuppressive treatment is chemotherapy. In these aspects, the chemotherapy may be myeloablative chemotherapy for an autologous bone marrow transplant, a myeloablative chemotherapy for an allogeneic bone marrow transplant, a myelotoxic chemotherapy for treatment of leukemia or a myelotoxic chemotherapy for treatment of solid tumors, or a combination thereof. In certain aspects, the chemotherapy comprises administration of chemotherapeutic agent, wherein the agent includes, but is not limited to adriamycin, asparaginase, bleomycin, calcium leucovorin, carmustine, carboplatin, cisplatin, cyclophosphamide, cytarabine, dacarbazine, dactinomycin, daunorubicin, doxorubicin, epirubicin, etoposide, fluorouracil, fluoxymesterone, flutamide, hexamethylmelamine, hydroxyurea, ifosfamide, gencitabin, levamisole, busulfan, lomustine, mechlorethamine, melphalan, mercaptopurine, methotrexate, methyl-CCNU, topoasin, mitomycin C, mitoxantrone, prednisolone, procarbazine, streptozocin, taxol, taxane, taxotere, thioguanine, triethylene-thiophosphoramide, vinblastine, vincristine or a combination thereof. In a preferred embodiment, the chemotherapy is by administration of carboplatin, ifosfamide, adriamycin or a combination thereof.

In certain aspects, early nadir agents may include, but are not limited to, azacytadine, chlorambucil, 2-chlorodeoxyadenosine, cytarbine, dacarbazine, daunorubicin, epirubicin, fluorouracil, hydrocyurea, idarubicin, ifosfamide, mchlorethamine, methotrexane, mitoxantrone. taxol. taxotere. teniposide, thiotepa. trimetrexate, vinblastine, vincristine, regiments of chemotherapeutic agents, such as AI, MAID, CyaDIC, taxol plus platinum, CTX with or without platinum, topatecan. ifex, ESHAP, DHAP, or a combination thereof. In this aspect, the nadir would preferrably be between about 10 and about 14 days. For ESHAP or DHAP, the nadir would preferably be about 14 to about 15 days. These agents and any therapeutic combination of agents may be administered according to any method or regiment known to those of ordinary skill in the art. References exemplary for administration. regiments and combinations of a such agents include "Combination Cancer Chemotherapy Regimens" Roger W. anderson and William J. Dana, Eds., Laderley Laboratories (1991) and "The Cerenex Handbook", Robert S. Benjamin. Ed., Cerenex Pahrmaceuticals, Research Triangle Park, N.C. (1993).

In certain aspects, late nadir agents may include, but are not limited to, busulfan, carboplatin, carmustine, dactinomycin, etoposide, fludarabine, lomustine, melphalin, mitomycin, procarbazine, thioguanine, thiotepa, regiments of chemotherapuetic agents, such as CEP, ICE (ifosfamide, carboplatin, etoposide), carboplatin and taxol, carboplatin and etoposide. BUCAT (busulfan, thiotepa, carboplatin). and combinations thereof. In this aspect, it is preferred that the nadir is of from about 15 to about 35 days.

In certain aspects of the invention, the myelosuppressive treatment is radiation therapy. The radiation may comprise irradiating the mammal or administrating to the mammal a therapeutic composition comprising a radioactive material.

In certain aspects of the invention, the mammal having or at risk for thrombocytopenia has or is at risk for a liver disorder, leukemia, solid tumors, myelodysplasia, aplastic anemia, congenitel thrombocytopenic, bacterial or viral invfectiion, hepatitis and HIV immune throbocytopenia or tuberculosis, or a combination thereof.

In certain aspects of the use of the invention further comprises the step of administration of a therapeutically effective composition comprising a cytokine, hematapoietin, colony stimulating factor, interleukin, growth factor or combination thereof. In a preferred aspect, the combination thereof is a therapeutically effective composition comprising a cytokine. a colony stimulating factor and an interleukin. As used herein certain embodiments, the terms "cytokine", "lineage-specific cytokine", "treatment" and "mammal" are the same as described in U.S. Patent No. 5,851,984, incorporated herein by reference in its entirety, which reads in relevant part:

"The term 'cytokine' is a generic term for proteins released by one cell population which act on another cell as intercellular mediators. Examples of such cytokines are lymphokines, monokines, growth factors and traditional polypeptide hormones. Included among the cytokines are growth hormones such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen, OB protein; tumor necrosis factor-.alpha. and -.beta.; mullerian-inhibiting substance; mouse gonadotropin-associated peptide; inhibin; activin; vascular endothelial growth factor; integrin; thrombopoietin (TPO); nerve growth factors such as NGF-.beta.; platelet-growth factor; transforming growth factors (TGFs) such as TGF-.alpha. and TGF-.beta.; insulin-like growth factor-I and -II; erythropoietin (EPO); osteoinductive factors; interferons such as interferon-.alpha., - .beta., and -y; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1.alpha., IL-2, IL-3, IL-4, IL-5. IL-6, IL-7, IL-8, IL-9, IL-11, IL-12; and other polypeptide factors including leukemia inhibit factor (LIF) and kit ligand (KL). As used herein, the term cytokine includes proteins from natural sources or from recombinant cell culture and biologically active equivalents of the native sequence cytokines.

A 'lineage-specific cytokine' is one which acts on relatively committed cells in the hematopoietic cascade and gives rise to an expansion in blood cells of a single lineage. Examples of such cytokines include EPO, TPO, and G-CSF.

'Treatment' refers to both therapeutic treatment and prophylactic or preventative measures. Those in need of treatment include those already with the disease or disorder as well as those in which the disease or disorder is to be prevented."

In addition to the cytokines described above, a "cytokine' may be a hematopoietic growth factor, SCF, FLT-3 ligand or a combination thereof of any cytokines listed herein. In a particularly preferred aspect, the cytokine has thromopoietic activity. Preferred cytokines include, but are not limited to, kit-ligand. LIF, G-CSF, GM-CSF, M-CSF, EPO, FLT-3, IL-1, IL-2, IL-3, IL-5, IL-6, IL-7, IL-8, JL-9, IL-10, IL-11 or IL-12. Other particularly preferred cytokines are G-CSF or GM-CSF. In certain embodiments, the growth hormone may be a insulin-like growth factor, a human growth hormone or bovine growth hormone. In some aspects, the glycoprotein hormones is follicle stimulating hormone, thryoid stimulating hormone or leutinizing hormone. In some aspects of the present invention, the tumor necrosis factor is TNF-$\alpha$. or TNF-$\beta$. In certain aspects of the invention, the nerve growth factor is NGF-$\beta$. In certain aspects of the invention, the interferon is interferon-$\alpha$, interferon -$\beta$ or interferon -$\gamma$. In certain aspects of the invention, the colony stimulating factors may be, but is not limited to, M-CSF, GM-CSF or G-CSF. In certain aspects of the invention, the interleukin may be. but is not limited to, IL-1. IL-1$\alpha$. IL-2, IL-3, IL-4, IL-5, IL-6, IL-7, IL-8, IL-9, IL-11 or IL-12.

In certain aspects of the invention, the administration of a therapeutic composition comprising TPO reduces neutropenia.

As used herein, "administration" means delivery or contacting of an agent, cytokine, TPO molecule, or other composition described herein the methods and compositions of the invention using any techniques described or as would be known to one of ordinary skill in the art. Such methods include administration within a pharmaceutically acceptable carrier.

As defined herein, "animal' refers to any organism classified as an animal, either vertebrate or invertebrate. Preferred animals for the use with the methods and compositions described herein include "mammals", which includes any classified as a mammal, including humans, domestic and farm animals, and zoo, sports, or pet animals, such as dogs, horses, cats, cows, rodents, etc. Preferably, the mammal is human. Additionally, a "patient" is one who undergoes treatment as described herein. Preferred patients are mammals, and particularly preferred patients are humans.

In certain aspects of the invention, the mammal has cancer. The cancer may include, but is not limited to, breast, colon, gastric, genitourinary, head and neck, leukemia, lung, lymphoma, malignant melanoma, multiple myeloma, ovarian, endometrial, pancreatic, pediatric, sarcoma, ovarian carcinoma, primary peritoneal malignancy, postate cancer, brain tumors, Hodgkins disease, germ cell tumors, gynocolgical maglignancy, endocrine tumors, or a combination thereof.

As used herein, it will be understood that the terms "a", "an", and "the" may refer to one or more than one.

## BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

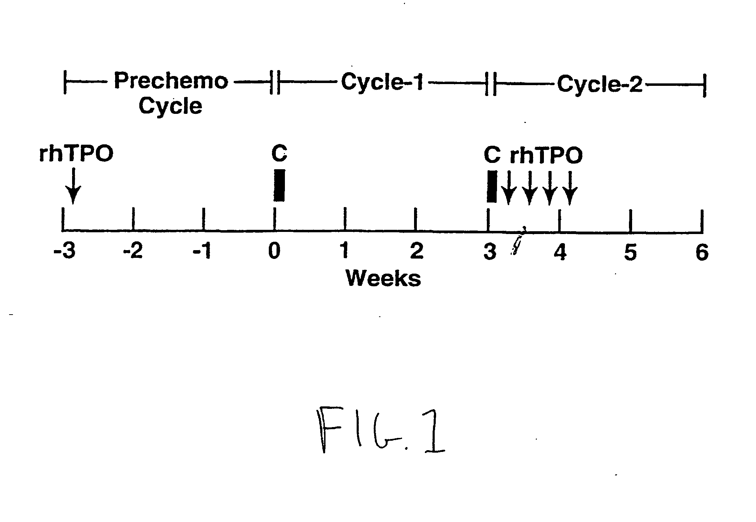

FIG. 1. Schema of the Therapeutic Schedule. rhTPO was administered (rhTPO labeled arrows) at dose levels 0.6, 1.2, 2.4, or 3.6 mcg/kg by subcutaneous injection as a single dose 3 weeks prior to and following a second cycle of carboplatin (C labeled •) (AUC=11). Each arrow represents a dose.

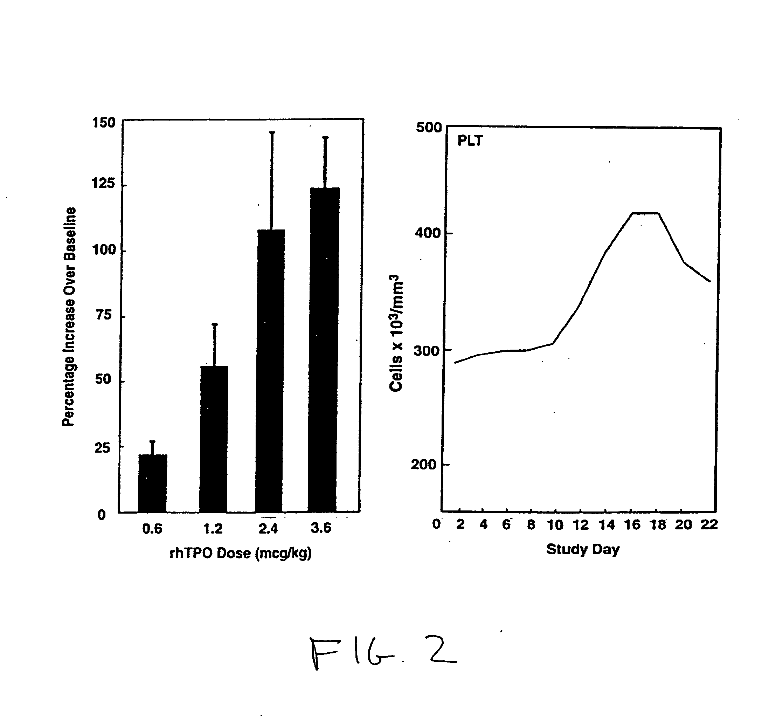

FIG. 2. The left panel shows the dose effect of rhTPO on circulating platelet counts. Maximal percentage increases from baseline (mean ± SEM) after various doses of rhTPO are shown. The right panel shows the kinetics of platelet response after a single dose of rhTPO. The data given represents the mean values for all patients (n=16).

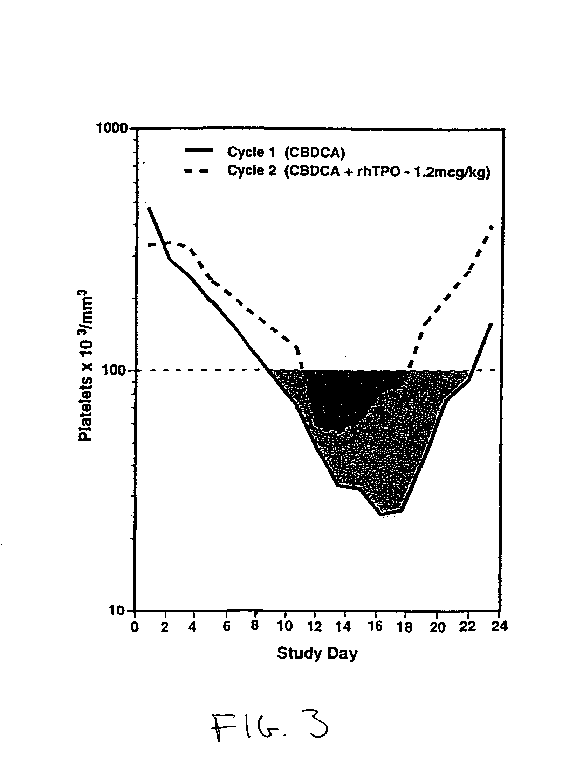

**FIG. 3.** Effects of rhTPO (1.2 mcg/kg on days 2, 4, 6, 8) used as a secondary prophylaxis on mean platelet counts (n=6) following carboplatin in Cycle-2 (hatched line) as compared with carboplatin alone in Cycle-1 (solid line). The darkly shaded area represents the degree and duration of thrombocytopenia in cycle 2, the lightly shaded area represents the degree and duration of thrombocytopenia in cycle 1.

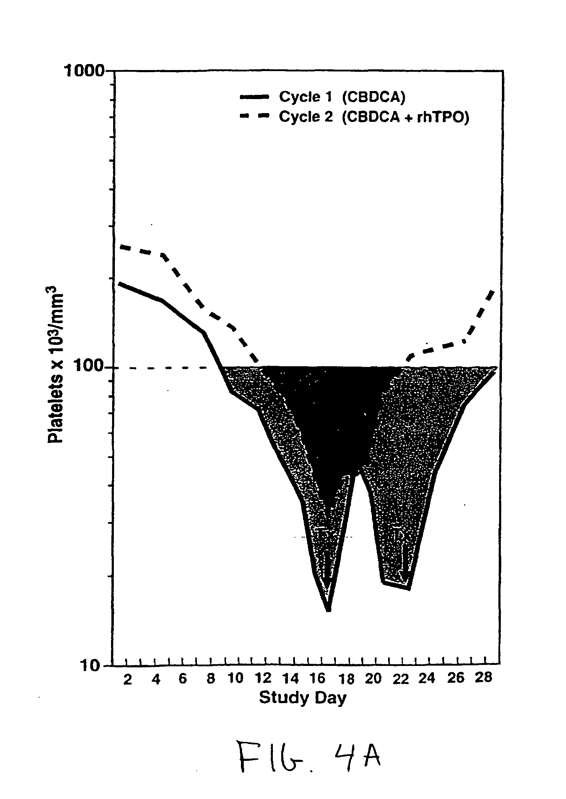

**FIG. 4A.** Example of reduced platelet nadir and enhanced platelet recovery, alleviating the need for platelet transfusion with rhTPO (1.2 mcg/kg on days -1, 1, 3, 5) used as a secondary prophylaxis following carboplatin in Cycle-2 (hatched line) compared with carboplatin alone in Cycle- 1 (solid line) during which the patient required two platelet transfusions (Tx labeled arrows). The darkly shaded area represents the degree and duration of thrombocytopenia in cycle 2, the lightly shaded area represents the degree and duration of thrombocytopenia in cycle 1.

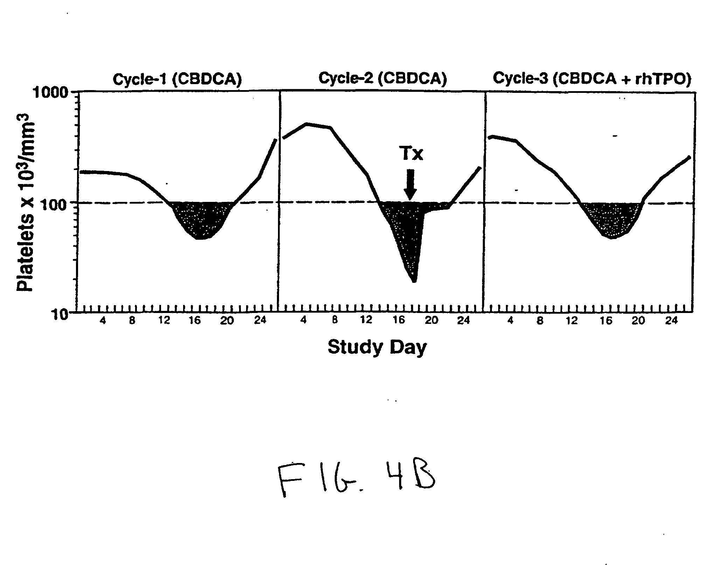

**FIG. 4B.** Example of abrogation of cumulative severe thrombocytopenia and the need for platelet transfusion in Cycle-3 with rhTPO (1.2 mcg/kg on days 1, 1, 3, 5) used as a secondary prophylaxis in a patient with documented severe thrombocytopenia in Cycle-2 with carboplatin alone, requiring a platelet transfusion (Tx labeled arrow). The shaded area represents the degree and duration of thrombocytopenia in each cycle.

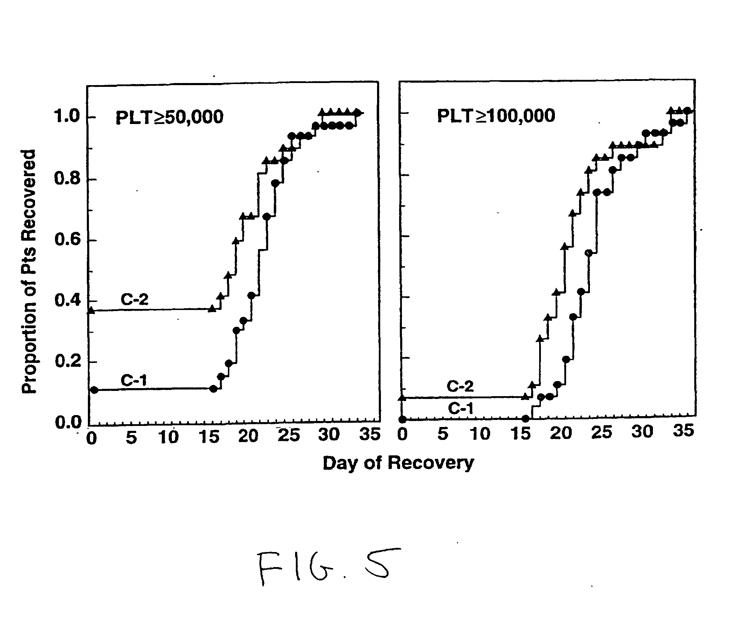

**FIG. 5.** The left panel shows time to platelet recovery to $\geq$50,000/mm$^3$ ($P$<0.019) and the right panel shows time to recovery to $\geq$100,000/mm$^3$ ($P$<0.017) in Cycle-1 (without rhTPO) and Cycle-2 (with rhTPO). TPO enhanced platelet recovery to $\geq$50,000/mm$^3$ (left panel), and $\geq$100.000/mm$^3$ in cylce-2 as opposed to cycle-1.

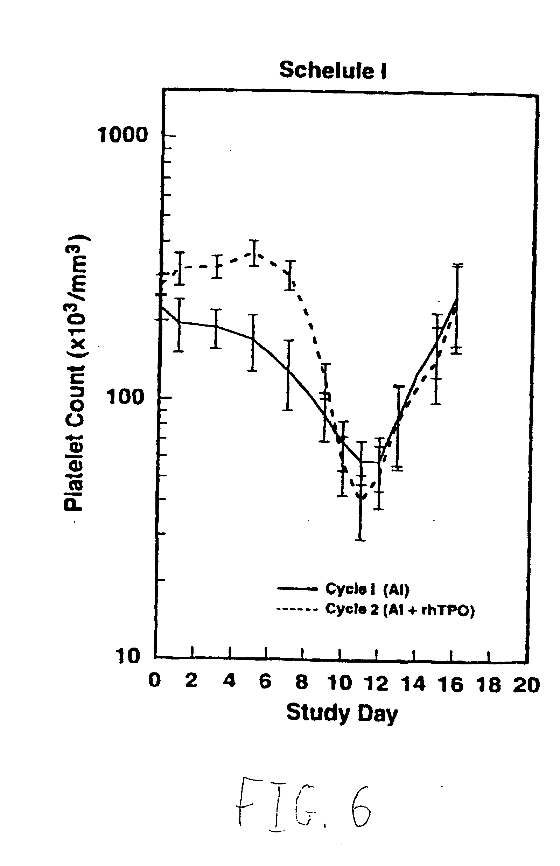

**FIG. 6.** Effect of rhTPO (Schedule I: one pre-dose and 3 post-doses) on AI chemotherapy-induced thrombocytopenia. See example 8 for details.

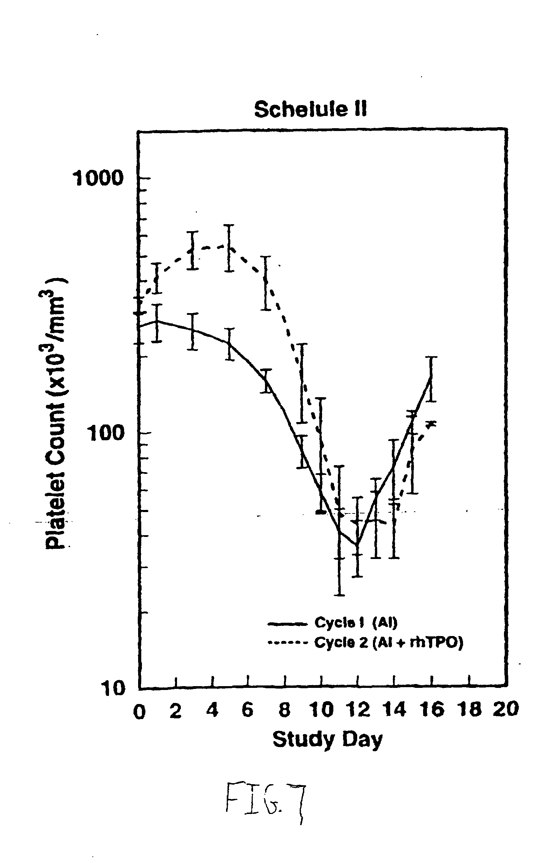

**FIG. 7.** Effect of rhTPO (Schedule II: two pre-doses and 2 post-doses) on AI chemotherapy-induced thrombocytopenia. See example 8 for details.

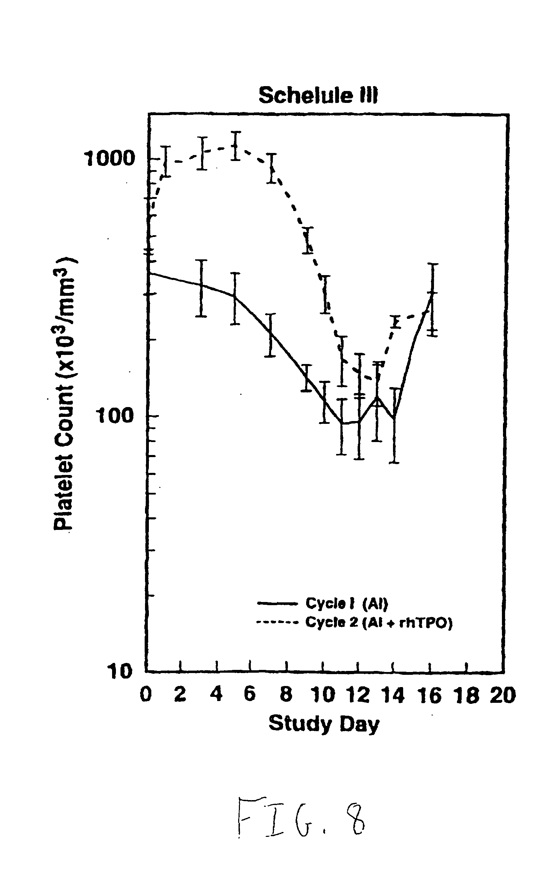

**FIG. 8.** Effect of rhTPO (Schedule III: 3 pre-doses and 1 post-dose) on AI chemotherapy-induced thrombocytopenia. See example 8 for details.

## DESCRIPTION OF ILLUSTRATIVE EMBODIMENTS

### I. Crypreservation of Platelets

The present invention relates to novel uses of compositions of TPO. TPO is a newly discovered thrombopoietic cytokine that can raise circulating platelets very high and thereby increase the yield of apheresis by several fold and markedly reduce the pheresis time. To pursue clinical development in this area, the inventor investigated whether platelets from TPO-treated patients can be stored for long-term by cryopreservation. The studies demonstrated that cryopreserved platelets from TPO-treated patients showed significantly greater retention of morphology and functions as compared to cryopresrved platelets from normal donors. The studies indicate that TPO can increase platelets by several-fold and potentially allow collection of multiple units of platelets that can be cryopreserved for long term (up to 6 months), with excellent recovery of cell number and functions.

This disclosure thus encompasses the induction of thrombocytosis with TPO treatment, collection of multiunits of platelets, cryopreservation of platelets for long-term storage and stock-piling of autologous platelets, single donor or directed donor platelets, random donor platelets for emergency use, and the creation of storage banks for HLA-matched platelets. Thus, stock-piling of cryopreserved platelets may alleviate several problems of transfusion medicine including the availability of matched platelets for emergency situations, supply crisis. and inventory problems for blood bank. This stock-piling autologous platelets prior to multicycle myelosuppressive or myeloablative therapy may be useful in supporting periods of severe thrombocytopenia with rapidly accessible autologous cells.

One of the common problems which limit the usefulness of multiple platelet transfusions from random donors is alloimmunization. To minimize this problem, exposure to minimum donors would be preferrable. TPO induced thrombocytosis would facilitate procurement of a large number of platelets to create storage banks of HLA-matched platelets that can be available as needed, especially for use in highly alloimmunized patients.

The strategy of cryopreservation of rhTPO-induced platelets can also be applied in other non-oncologic areas where thrombocytopenia is an expected complication of planned treatment (*e.g.,* cardiac surgery) (Alvarez *et al.,* 1992; Graylee *et al..* 1994). The use of autologous platelets in this setting would also alleviate undue anxiety and concerns of transmission of infectious agents. In normal donors, the potent biologic effect and lack of significant toxicity allows TPO to be used in normal donors or family members to facilitate procurement of a large number of platelets that can be cryopreserved and be available as needed. Thus, this novel strategy has a potential to overcome the limitations of the currently available methods.

It is contemplated that cancer patients may be treated with TPO and/or rhTPO to raise platelets prior to. during, or after chemotherapy, and the platelets may be collected by apheresis, that they may be stored by cryopreservation in the methods described herein, and that they may be transfused back into a patient. The patient will preferably be a patient suffering thrombocytopenia such as a cancer patient undergoing chemotherapy.

ThromboSol is a newly developed platelet-stabilizing solution that inhibits specific activation pathways and protect against the detrimental effect of cold storage. The method of the present invention is unique in that it uses TPO to markedly increase the platelet yield for apheresis (for autologous as well as allogeneic use), and it uses novel method of platelet cryopreservation (i.e., preferably ThromboSol and 2% DMSO). *In vitro* studies of the inventor shows that platelets from TPO-treated patients have enhanced retention of cell functions as compared to platelets from normal donor cryopreserved with conventional methods (5% or 6% DMSO) or with ThromboSol + 2% DMSO. It is contemplated that TPO amy be used as a cryopreservative agent for the storage of platelets. Additionally, TPO may be administered before, during, and/or after administration of platelets to help enhance or retain the platelets activity.

While the protocol is designed based on the sound rationale and prior observations, it is likely that it may undergo refinement and fine-tuning based on the crucial observations that are made during the treatment of a patient. Based on prior experience, it is also possible that the modifications to the original design might be necessary to optimize the timing and yield of collection, cryopreservation, and transfusion of the autologous platelets. For instance, in the setting where a patient can not mount adequate response in platelet counts to donate sufficient units of platelets prior to chemotherapy or in the setting where the patient has rapidly progressive disease and can not wait to receive TPO and have platelet pheresis prior to chemotherapy, the inventor may be able to collect platelets post-chemotherapy + TPO during recovery rebound thrombocytosis. The clincial and recentl laboratory study would support that. Similarly, additional doses of TPO administration may be necessary, prior to, during and post transfusion of cryopreserved platelets to improve the survival/function of the transfused platelets.

## II. Administration of TPO to Reduce Thrombocytopenia

Administration of a single dose of recombinant human TPO (rhTPO) in cancer patients prior to chemotherapy is a potent stimulus for prolonged platelet production and increased circulating platelet counts to one million or higher in several patients. This finding raised the possibility that rhTPO can be given to cancer patients for autologous donation prior to multicycle myelosuppressive therapy or myeloblative treatment. Thus, invention comprises uses TPO for the manufacture of a medicament to reduce or prevent thrombocytopenia. The present invention demonstrates that an optimum TPO dose schedule is other than daily administration.

Timing of the administration of TPO in relation to the myelosuppressive treatment produces optimized results for the agent being used, as described above in the Summary and below in the specific examples. With the regimens that are long (such as AI - ≥4 days) and/or have early nadir (such as AI - around day 12), the pre-dosing of rhTPO along with post-dosing may be beneficial. Whereas, with the regimens that are short (such as carboplatin as a single agent - 1 or 2 day regimen) and/or have a delayed nadir (<day 12, around day 16, such as with carboplatin), post-dosing (alone) with rhTPO may be effective. These findings indicate that earlier administration of rhTPO (i.e., pre-chemotherapy or pre-dosing) may be beneficial. In one example, where one dose of rhTPO was given prior to AI (pre-dosing ) and two doses following AI (post-dosing), rhTPO enhanced platelet recovery by this method, and the nadir platelet count was higher in few patients. These findings indicate that multiple pre-dosing with reTPO may provide improved effect on platelets nadir and recovery.

## III. Thrombopoietin Nucleic Acids

### A. Genes and DNA Segments

The present invention involves compositions comprising thrombopoietin (TPO). The nucleotide and protein, polypeptide and peptide sequences for TPO and other cytokines and peptide hormones have been previously disclosed, and may be found at the National Center for Biotechnology Information's Genbank and GenPept databases (http://www.ncbi.nlm.nih.gov/). In particular, the nucleotide sequences including, but not limited to, Genbank accession numbers AB014683, AB014682, AB014681, L36052, L36051, E12215, E12214, E12183, E12182 and E12181 are contemplated as being useful for the production of recombinant TPO (rTPO) for the uses and compositions of the present invention. Nucleotide sequences derived from human TPO sequences, including but not limited to, Genbank accession numbers AB014683, AB014682, AB014681, L36052, L36051, E12215, E12214, E12183 and E12182 are contemplated as being particularly useful for the production of recombinant human TPO (rhTPO) for the uses and compositions of the present invention.

The present disclosure also concerns isolated DNA segments and recombinant vectors encoding thrombopoietin, and the creation and use of recombinant host cells through the application of DNA technology, that express wild-type, polymorphic or mutant thrombopoietin, using the sequence of SEQ ID NO:1, and biologically functional equivalents thereof. In addition to the methods for obtaining, isolating, synthesizing, mutating, chemically modifying, expressing, purifying, detecting the activity of, and administering and/or using TPO, rTPO and related thrombopoietic nucleotide and amino acid sequences described herein, such methods are exemplified by U.S. patent Nos. 5,641,655, 5,830,647, 5,795,569, 5,766,897, 5,766,581, 5,756,083, 5,744,587, 5,733,746, 5,696,250, 5,593,666, 5,571,686, 5,250,732 and 4,894,440.

The present disclosure concerns DNA segments, isolatable from mammalian cells, particularly porcine, murine and human cells, that are free from total genomic DNA and that are capable of expressing a protein, polypeptide, or peptide that has thrombopoietin activity.

As used herein, the term "DNA segment" refers to a DNA molecule that has been isolated free of total genomic DNA of a particular species. Therefore, a DNA segment encoding TPO refers to a DNA segment that contains TPO coding sequences yet is isolated away from, or purified free from, total mammalian, particularly porcine, murine or human, genomic DNA. Included within the term "DNA segment", are DNA segments and smaller fragments of such segments, and also recombinant vectors, including, for example, plasmids, cosmids, phage, viruses, and the like.

Similarly, a DNA segment comprising an isolated or purified thrombopoietin. gene refers to a DNA segment including thrombopoietin protein coding sequences and, in certain aspects, regulatory sequences, isolated substantially away from other naturally occurring genes or protein encoding sequences. In this respect, the term "gene" is used for simplicity to refer to a functional protein, polypeptide or peptide encoding unit. As will be understood by those in the art, this functional term includes both genomic sequences. cDNA sequences and smaller engineered gene segments that express, or may be adapted to express, proteins, polypeptides. domains, peptides. fusion proteins and mutants.

"Isolated substantially away from other coding sequences" means that the gene of interest, in this case the thrombopoietin gene, forms the significant part of the coding region of the DNA segment, and that the DNA segment does not contain large portions of naturally-occurring coding DNA, such as large chromosomal fragments or other functional genes or cDNA coding regions. Of course, this refers to the DNA segment as originally isolated, and does not exclude genes or coding regions later added to the segment by the hand of man.

In particular embodiments, the invention concerns isolated DNA segments and recombinant vectors incorporating DNA sequences that encode a TPO protein, polypeptide or peptide that includes within its amino acid sequence a contiguous amino acid sequence in accordance with, or essentially as set forth in, SEQ ID NO:2.

The term "a sequence essentially as set forth in SEQ ID NO:2" means that the sequence substantially corresponds to a portion of SEQ ID NO:2 and has relatively few amino acids that are not identical to, or a biologically functional equivalent of, the amino acids of SEQ ID NO:2.

The term "biologically functional equivalent" is well understood in the art and is further defined in detail herein. Accordingly, sequences that have between about 70% and about 80%; or more preferably, between about 81% and about 90%; or even more preferably, between about 91% and about 99%; of amino acids that are identical or functionally equivalent to the amino acids of SEQ ID NO:2 will be sequences that are "a sequence essentially as set forth in SEQ ID NO:2" provided the biological activity, *i.e.* the thrombopoietic and/or crypreservative activity of the protein, polypeptide or peptide is maintained.

In certain other embodiments, the disclosure concerns isolated DNA segments and recombinant vectors that include within their sequence a nucleic acid sequence essentially as set forth in SEQ ID NO:1. The term "essentially as set forth in SEQ ID NO:1" is used in the same sense as described above and means that the nucleic acid sequence substantially corresponds to a portion of SEQ ID NO:1 and has relatively few codons that are not identical, or functionally equivalent, to the codons of SEQ ID NO:1. Again, DNA segments that encode proteins exhibiting thrombopoietic and/or crypreservative activity will be most preferred.

The term "functionally equivalent codon" is used herein to refer to codons that encode the same amino acid, such as the six codons for arginine or serine, and also refers to codons that encode biologically equivalent amino acids. For optimization of expression of TPO in human cells, the codons are shown in preference of use from left to right, in Table 1. The most preferred codon for alanine is thus "GCC", and the least is "GCG" (see Table 1, below).

TABLE 1

| Preferred Human DNA Codons | | | | | | | | |
|---|---|---|---|---|---|---|---|---|
| **Amino Acids** | | | | **Codons** | | | | |
| Alanine | Ala | A | | GCC | GCT | GCA | GCG | |
| Cysteine | Cys | C | | TGC | TGT | | | |
| Aspartic acid | Asp | D | | GAC | GAT | | | |
| Glutamic acid | Glu | E | | GAG | GAA | | | |
| Phenylalanine | Phe | F | | TTC | TTT | | | |
| Glycine | Gly | G | | GGC | GGG | GGA | GGT | |
| Histidine | His | H | | CAC | CAT | | | |

TABLE 1   (continued)

| Preferred Human DNA Codons | | | | | | | | | |
|---|---|---|---|---|---|---|---|---|---|
| **Amino Acids** | | | | **Codons** | | | | | |
| Isoleucine | Ile | I | | ATC | ATT | ATA | | | |
| Lysine | Lys | K | | AAG | AAA | | | | |
| Leucine | Leu | L | | CTG | CTC | TTG | CTT | CTA | TTA |
| Methionine | Met | M | | ATG | | | | | |
| Asparagine | Asn | N | | AAC | AAT | | | | |
| Proline | Pro | P | | CCC | CCT | CCA | CCG | | |
| Glutamine | Gln | Q | | CAG | CAA | | | | |
| Arginine | Arg | R | | CGC | AGG | CGG | AGA | CGA | CGT |
| Serine | Ser | S | | AGC | TCC | TCT | AGT | TCA | TCG |
| Threonine | Thr | T | | ACC | ACA | ACT | ACG | | |
| Valine | Val | V | | GTG | GTC | GTT | GTA | | |
| Tryptophan | Trp | W | | TGG | | | | | |
| Tyrosine | Tyr | Y | | TAC | TAT | | | | |

It will also be understood that amino acid and nucleic acid sequences may include additional residues, such as additional N- or C-terminal amino acids or 5' or 3' sequences, and yet still be essentially as set forth in one of the sequences disclosed herein. so long as the sequence meets the criteria set forth above. including the maintenance of biological protein activity where protein expression is concerned. The addition of terminal sequences particularly applies to nucleic acid sequences that may, for example, include various non-coding sequences flanking either of the 5' or 3' portions of the coding region or may include various internal sequences, i.e., introns, which are known to occur within genes.

Excepting intronic or flanking regions, and allowing for the degeneracy of the genetic code, sequences that have between about 70% and about 79%; or more preferably, between about 80% and about 89%; or even more preferably, between about 90% and about 99%; of nucleotides that are identical to the nucleotides of SEQ ID NO: 1 will be sequences that are "essentially as set forth in SEQ ID NO: 1".

Sequences that are essentially the same as those set forth in SEQ ID NO: 1 may also be functionally defined as sequences that are capable of hybridizing to a nucleic acid segment containing the complement of SEQ ID NO: 1 under relatively stringent conditions. Suitable relatively stringent hybridization conditions will be well known to those of skill in the art, as disclosed herein.

Hybridization is understood to mean the forming of a double stranded molecule or a molecule with partial double stranded nature. Stringent conditions are those that allow hybridization between two homologous nucleic acid sequences, but precludes hybridization of random sequences. For example, hybridization at low temperature and/or high ionic strength is termed low stringency. Hybridization at high temperature and/or low ionic strength is termed high stringency. Low stringency is generally performed at 0.15 M to 0.9 M salt (for example NaCl) at a temperature range of 20°C to 50°C. High stringency is generally performed at 0.02 M to 0.15 M salt (for example NaCl) at a temperature range of 50°C to 70°C. It is understood that the temperature and ionic strength of a desired stringency are determined in part by the length of the particular probe, the length and base content of the target sequences, and to the presence of formamide, tetramethylammonium chloride or other solvents in the hybridization mixture. It is also understood that these ranges are mentioned by way of example only, and that the desired stringency for a particular hybridization reaction is often determined empirically by comparison to positive and negative controls.

Accordingly, the nucleotide sequences of the disclosure may be used for their ability to selectively form duplex molecules with complementary stretches of genes or RNAs or to provide primers for amplification of DNA or RNA from tissues. Depending on the application envisioned. it is preferred to employ varying conditions of hybridization to achieve varying degrees of selectivity of probe towards target sequence.

For applications requiring high selectivity, it is preferred to employ relatively stringent conditions to form the hybrids. For example, relatively low salt and/or high temperature conditions, such as provided by about 0.02 M to about 0.10 M NaCl at temperatures of about 50°C to about 70°C. Such high stringency conditions tolerate little, if any, mismatch between the probe and the template or target strand, and would be particularly suitable for isolating specific genes or detecting specific mRNA transcripts. It is generally appreciated that conditions may be rendered more stringent by the addition of increasing amounts of formamide.

Naturally, the present disclosure also encompasses DNA segments that are complementary, or essentially complementary, to the sequence set forth in SEQ ID NO:1. Nucleic acid sequences that are "complementary" are those that are capable of base-pairing according to the standard Watson-Crick complementary rules. As used herein, the term "complementary sequences" means nucleic acid sequences that are substantially complementary, as may be assessed by the same nucleotide comparison set forth above, or as defined as being capable of hybridizing to the nucleic acid segment of SEQ ID NO: 1 under relatively stringent conditions such as those described herein.

The nucleic acid segments, regardless of the length of the coding sequence itself, may be combined with other DNA sequences, such as promoters, enhancers. polyadenylation signals, additional restriction enzyme sites, multiple cloning sites, other coding segments, and the like, such that their overall length may vary considerably. It is therefore contemplated that a nucleic acid fragment of almost any length may be employed, with the total length preferably being limited by the ease of preparation and use in the intended recombinant DNA protocol.

For example, nucleic acid fragments may be prepared that include a contiguous stretch of nucleotides identical to or complementary to SEQ ID NO:1, such as about 8, about 9, about 10, about 11, about 12, about 13, about 14, about 15, about 16. about 17. about 18, about 19. about 20. about 21, about 22. about 23. about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, about 50, about 51, about 52, about 53, about 54, about 55, about 56, about 57, about 58, about 59, about 60, about 61, about 62, about 63, about 64, about 65, about 66, about 67, about 68, about 69, about 70, about 71, about 72, about 73, about 74, about 75, about 76, about 77, about 78, about 79, about 80, about 81, about 82, about 83, about 84, about 85, about 86, about 87. about 88, about 89, about 90, about 91, about 92, about 93, about 94, about 95, about 96, about 97, about 98, about 99, about 100, about 125, about 150, about 250, about 300, about 400, about 600, about 750, about 800 or about 900 nucleotides, and that are up to about 1,000,000, about 750,000, about 500,000, about 250,000, about 100,000, about 50,000, about 20,000, or about 10,000, or about 5,000 base pairs in length, with segments of about 3,000 being preferred in certain cases. In certain cases, nucleotide segments of a million bases or more, including chromosome sized pieces of DNA, are contemplated as being useful. DNA segments with total lengths of about 1,000, about 500, about 200, about 100 and about 50 base pairs in length (including all intermediate lengths) are also contemplated to be useful.

It will be readily understood that "intermediate lengths", in these contexts, means any length between the quoted ranges, such as 10, 11, 12, 13, 14, 15, 16, 17, 18, 19, 20, *etc.;* 21, 22, 23, *etc.;* 30, 31, 32, *etc.;* 50, 51, 52, 53, *etc.;* 100, 101, 102, 103, *etc.;* 150, 151, 152, 153, *etc.;* including all integers through the 200-500; 500-1,000; 1,000-2,000; 2,000-3,000; 3,000-5,000; 5,000-10,000 ranges, up to and including sequences of about 12,001, 12,002, 13,001, 13,002, 15,000, 20,000 and the like.

The various probes and primers designed around the disclosed nucleotide sequences may be of any length. By assigning numeric values to a sequence, for example, the first residue is 1, the second residue is 2, *etc..* an algorithm defining all primers can be proposed:

$$n \text{ to } n + y$$

where n is an integer from 1 to the last number of the sequence and y is the length of the primer minus one, where n + y does not exceed the last number of the sequence. Thus, for a 10-mer, the probes correspond to bases 1 to 10, 2 to 11, 3 to 12 ... and so on. For a 15-mer, the probes correspond to bases 1 to 15, 2 to 16, 3 to 17 ... and so on. For a 20-mer, the probes correspond to bases 1 to 20, 2 to 21, 3 to 22 ... and so on.

It will also be understood that this disclosure is not limited to the particular nucleic acid and amino acid sequences of SEQ ID NO: 1. Recombinant vectors and isolated DNA segments may therefore variously include these coding regions themselves, coding regions bearing selected alterations or modifications in the basic coding region, or they may encode larger polypeptides that nevertheless include such coding regions or may encode biologically functional equivalent proteins, polypeptides or peptides that have variant amino acids sequences.

The DNA segments encompass biologically functional equivalent TPO proteins, polypeptides, and peptides. Such sequences may arise as a consequence of codon redundancy and functional equivalency that are known to occur naturally within nucleic acid sequences and the proteins thus encoded. Alternatively, functionally equivalent proteins, polypeptides or peptides may be created *via* the application of recombinant DNA technology, in which changes in the protein structure may be engineered, based on considerations of the properties of the amino acids being exchanged. Changes designed by man may be introduced, for example, through the application of site-directed mutagenesis techniques as discussed herein below, *e.g.,* to introduce improvements to the antigenicity of the protein, *i.e.* to reduce its antigenicity when administered to a patient, or to test mutants in order to examine protein thrombopoietin activity at the molecular level.

One may also prepare fusion proteins, polypeptides and peptides, *e.g.*, where the thrombopoietin protein coding regions are aligned within the same expression unit with other proteins or peptides having desired functions, such as for purification or immunodetection purposes (*e.g.*, proteins that may be purified by affinity chromatography and enzyme label coding regions. respectively).

In addition to the "standard" DNA and RNA nucleotide bases, modified bases are also contemplated for use in particular applications of the present invention. A table of exemplary, but not limiting, modified bases is provided herein below.

Table 2 -

| Modified Bases | | | |
|---|---|---|---|
| Abbr. | Modified base description | Abbr. | Modified base description |
| ac4c | 4-acetylcytidine | mam5s2u | 5-methoxyaminomethyl-2-thiouridine |
| chm5u | 5-(carboxyhydroxylmethyl)uridine | manq | beta,D-mannosylqueosine |
| cm | 2'-O-methylcytidine | mcm5s2u | 5-methoxycarbonylmethyl-2-thiouridine |
| cmnm5s2u | 5-carboxymethylaminomethyl-2-thioridine | mcm5u | 5-methoxycarbonylmethyluridine |
| cmnm5u | 5-carboxymethylaminomethyluridine | mo5u | 5-methoxyuridine |
| d | dihydrouridine | ms2i6a | 2-methylthio-N6-isopentenyladenosine |
| fm | 2'-O-methylpseudouridine | ms2t6a | N-((9-beta-D-ribofuranosyl-2-methylthiopurine-6-yl)carbamoyl)threonine |
| gal q | beta,D-galactosylqueosine | mt6a | N-((9-beta-D-ribofuranosylpurine-6-yl)N-methyl-carbamoyl)threonine |
| gm | 2'-O-methylguanosine | mv | uridine-5-oxyacetic acid methylester |
| i | inosine | o5u | uridine-5-oxyacetic acid (v) |
| i6a | N6-isopentenyladenosine | osyw | wybutoxosine |
| m1a | 1-methyladenosine | p | pseudouridine |
| m1f | 1-methylpseudouridine | q | queosine |
| m1g | 1-methylguanosine | s2c | 2-thiocytidine |
| m1i | 1-methylinosine | s2t | 5-methyl-2-thiouridine |
| m22g | 2,2-dimethylguanosine | s2u | 2-thiouridine |
| m2a | 2-methyladenosine | s4u | 4-thiouridine |
| m2g | 2-methylguanosine | t | 5-methyluridine |
| m3c | 3-methylcytidine | t6a | N-((9-beta-D-ribofuranosylpurine-6-yl)carbamoyl)threonine |
| m5c | 5-methylcytidine | tm | 2'-O-methyl-5-methyluridine |
| m6a | N6-methyladenosine | um | 2'-O-methyluridine |
| m7g | 7-methylguanosine | yw | wybutosine |
| mam5u | 5-methylaminomethyluridine | | 3-(3-amino-3-carboxypropyl)uridine |
| | 5-fluorouracil | | hypoxanthine |
| | 5-bromouracil | | xanthine |
| | 5-chlorouracil | | (acp3)w |
| | 5-iodouracil | | 2,6-diaminopurine |

Table 2 - (continued)

| Modified Bases | | | |
|---|---|---|---|
| Abbr. | Modified base description | Abbr. | Modified base description |
| | N6-adenine | | |

## B. Recombinant Vectors, Host Cells and Expression

The term "expression vector or construct" means any type of genetic construct containing a nucleic acid coding for a gene product in which part or all of the nucleic acid encoding sequence is capable of being transcribed. The transcript may be translated into a protein, but it need not be. Thus, in certain embodiments, expression includes both transcription of a gene and translation of a RNA into a gene product. In other embodiments, expression only includes transcription of the nucleic acid, for example, to generate antisense constructs.

Particularly useful vectors are contemplated to be those vectors in which the coding portion of the DNA segment. whether encoding a full length protein, polypeptide or smaller peptide, is positioned under the transcriptional control of a promoler. A "promoter" refers to a DNA sequence recognized by the synthetic machinery of the cell, or introduced synthetic machinery, required to initiate the specific transcription of a gene. The phrases "operatively positioned", "under control" or "under transcriptional control" means that the promoter is in the correct location and orientation in relation to the nucleic acid to control RNA polymerase initiation and expression of the gene.

The promoter may be in the form of the promoter that is naturally associated with an thrombopoietin gene, as may be obtained by isolating the 5' non-coding sequences located upstream of the coding segment or exon, for example, using recombinant cloning and/or PCR$^{TM}$ technology, in connection with the compositions disclosed herein (PCR$^{TM}$ technology is disclosed in U.S. Patent 4,683,202 and U.S. Patent 4,682,195, each incorporated herein by reference).

In other embodiments, it is contemplated that certain advantages will be gained by positioning the coding DNA segment under the control of a recombinant, or heterologous, promoter. As used herein, a recombinant or heterologous promoter is intended to refer to a promoter that is not normally associated with an thrombopoietin gene in its natural environment. Such promoters may include promoters normally associated with other genes, and/or promoters isolated from any other bacterial, viral, eukaryotic, or mammalian cell, and/or promoters made by the hand of man that are not "naturally occurring," *i.e.,* containing difference elements from different promoters, or mutations that increase, decrease, or alter expression.

Naturally, it will be important to employ a promoter that effectively directs the expression of the DNA segment in the cell type, organism, or even animal, chosen for expression. The use of promoter and cell type combinations for protein expression is generally known to those of skill in the art of molecular biology, for example, see Sambrook *et al.* (1989), incorporated herein by reference. The promoters employed may be constitutive, or inducible, and can be used under the appropriate conditions to direct high level expression of the introduced DNA segment, such as is advantageous in the large-scale production of recombinant proteins or peptides.

At least one module in a promoter generally functions to position the start site for RNA synthesis. The best known example of this is the TATA box, but in some promoters lacking a TATA box, such as the promoter for the mammalian terminal deoxynucleotidyl transferase gene and the promoter for the SV40 late genes, a discrete element overlying the start site itself helps to fix the place of initiation.

Additional promoter elements regulate the frequency of transcriptional initiation. Typically, these are located in the region 30-110 bp upstream of the start site, although a number of promoters have been shown to contain functional elements downstream of the start site as well. The spacing between promoter elements frequently is flexible, so that promoter function is preserved when elements are inverted or moved relative to one another. In the tk promoter, the spacing between promoter elements can be increased to 50 bp apart before activity begins to decline. Depending on the promoter, it appears that individual elements can function either co-operatively or independently to activate transcription.

The particular promoter that is employed to control the expression of a nucleic acid is not believed to be critical, so long as it is capable of expressing the nucleic acid in the targeted cell. Thus, where a human cell is targeted, it is preferable to position the nucleic acid coding region adjacent to and under the control of a promoter that is capable of being expressed in a human cell. Generally speaking, such a promoter might include either a human or viral promoter.

In various other embodiments, the human cytomegalovirus (CMV) immediate early gene promoter, the SV40 early promoter and the Rous sarcoma virus long terminal repeat can be used to obtain high-level expression of the instant nucleic acids. The use of other viral or mammalian cellular or bacterial phage promoters which are well-known in the art to achieve expression are contemplated as well, provided that the levels of expression are sufficient for a given purpose. Tables 3 and 4 below list several elements/promoters which may be employed, in the context of the present invention, to regulate the expression of an thrombopoietin gene. This list is not intended to be exhaustive of all the possible elements involved in the promotion of expression but, merely, to be exemplary thereof.

Enhancers were originally detected as genetic elements that increased transcription from a promoter located at a distant position on the same molecule of DNA. This ability to act over a large distance had little precedent in classic studies of prokaryotic transcriptional regulation. Subsequent work showed that regions of DNA with enhancer activity are organized much like promoters. That is, they are composed of many individual elements, each of which binds to one or more transcriptional proteins.

The basic distinction between enhancers and promoters is operational. An enhancer region as a whole must be able to stimulate transcription at a distance; this need not be true of a promoter region or its component elements. On the other hand, a promoter must have one or more elements that direct initiation of RNA synthesis at a particular site and in a particular orientation, whereas enhancers lack these specificities. Promoters and enhancers are often overlapping and contiguous, often seeming to have a very similar modular organization.

Additionally any, promoter/enhancer combination (as per the Eukaryotic Promoter Data Base EPDB) could also be used to drive expression. Use of a T3, T7 or SP6 cytoplasmic expression system is another possible embodiment. Eukaryotic cells can support cytoplasmic transcription from certain bacterial promoters if the appropriate bacterial polymerase is provided, either as part of the delivery complex or as an additional genetic expression construct.

TABLE 3

| Promoter and Enhancer Elements | |
| --- | --- |
| Promoter/Enhancer | References |
| Immunoglobulin Heavy Chain | Banerji *et al.,* 1983: Gilles *et al.*, 1983: Grosschedl and Baltimore, 1985; Atchinson and Perry, 1986, 1987; Imler *et al.,* 1987; Weinberger *et al.,* 1984; Kiledjian *et al.,* 1988; Porton *et al.;* 1990 |
| Immunoglobulin Light Chain | Queen and Baltimore, 1983; Picard and Schaffner, 1984 |
| T-Cell Receptor | Luria *et al.,* 1987; Winoto and Baltimore, 1989; Redondo *et al.;* 1990 |
| HLA DQ a and DQ β | Sullivan and Peterlin, 1987 |
| β-Interferon | Goodbourn *et al.,* 1986; Fujita *et al.,* 1987; Goodbourn and Maniatis, 1988 |
| Interleukin-2 | Greene *et al.,* 1989 |
| Interleukin-2 Receptor | Greene *et al.,* 1989; Lin *et al.,* 1990 |
| MHC Class II 5 | Koch *et al.,* 1989 |
| MHC Class II HLA-DRa | Sherman *et al.,* 1989 |
| β-Actin | Kawamoto *et al.,* 1988; Ng *et al.;* 1989 |
| Muscle Creatine Kinase | Jaynes *et al.,* 1988; Horlick and Benfield, 1989; Johnson *et al.,* 1989 |
| Prealbumin (Transthyretin) | Costa *et al.,* 1988 |
| Elastase I | Omitz *et al.,* 1987 |
| Metallothionein | Karin *et al.,* 1987; Culotta and Hamer, 1989 |
| Collagenase | Pinkert *et al.,* 1987; Angel *et al.,* 1987 |
| Albumin Gene | Pinkert *et al.,* 1987; Tronche *et al.,* 1989, 1990 |
| α-Fetoprotein | Godbout *et al.,* 1988; Campere and Tilghman, 1989 |
| t-Globin | Bodine and Ley, 1987; Perez-Stable and Constantini, 1990 |
| β-Globin | Trudel and Constantini, 1987 |
| e-fos | Cohen *et al.*, 1987 |
| c-HA-*ras* | Triesman, 1986; Deschamps *et al.,* 1985 |

TABLE 3   (continued)

| Promoter and Enhancer Elements | |
|---|---|
| Promoter/Enhancer | References |
| Insulin | Edlund *et al.*, 1985 |
| Neural Cell Adhesion Molecule (NCAM) | Hirsh *et al.*, 1990 |
| $\alpha_1$ -Antitrypain | Latimer *et al.,* 1990 |
| H2B (TH2B) Histone | Hwang *et al.*, 1990 |
| Mouse or Type I Collagen | Ripe *et al.,* 1989 |
| Glucose-Regulated Proteins (GRP94 and GRP78) | Chang *et al.,* 1989 |
| Rat Growth Hormone | Larsen *et al.,* 1986 |
| Human Serum Amyloid A (SAA) | Edbrooke *et al.,* 1989 |
| Troponin I (TN I) | Yutzey *et al.,* 1989 |
| Platelet-Derived Growth Factor | Pech *et al.,* 1989 |
| Duchenne Muscular Dystrophy | Klamut *et al.,* 1990 |
| SV40 | Banerji *et al.,* 1981; Moreau *et al.,* 1981; Sleigh and Lockett, 1985; Firak and Subramanian, 1986; Herr and Clarke, 1986; Imbra and Karin, 1986; Kadesch and Berg, 1986; Wang and Calame, 1986; Ondek *et al.,* 1987; Kuhl *et al.*, 1987; Schaffner *et al.,* 1988 |
| Polyoma | Swartzendruber and Lehman, 1975; Vasseur *et al.,* 1980; Katinka *et al.,* 1980, 1981; Tyndell *et al.,* 1981; Dandolo *et al.,* 1983: de Villiers *et al.,* 1984; Hen *et al.,* 1986; Satake *et al.,* 1988; Campbell and Villarreal. 1988 |
| Retroviruses | Kriegler and Botchan, 1982, 1983; Levinson *et al.,* 1982; Kriegler *et al.,* 1983, 1984a, b, 1988; Bosze *et al.,* 1986; Miksicek *et al.,* 1986; Celander and Haseltine, 1987; Thiesen *et al.,* 1988; Celander *et al.,* 1988; Chol *et al.,* 1988: Reisman and Rotter. 1989 |
| Papilloma Virus | Campo *et al.*, 1983; Lusky *et at.*, 1983: Spandidos and Wilkie, 1983; Spalholz *et al.,* 1985; Lusky and Botchan, 1986; Cripe *et al.,* 1987; Gloss *et al.,* 1987; Hirochika *et al.,* 1987; Stephens and Hentschel, 1987; Glue *et al.,* 1988 |
| Hepatitis B Virus | Bulla and Siddiqui, 1986; Jameel and Siddiqui, 1986; Shaul and Ben-Levy, 1987; Spandau and Lee, 1988; Vannice and Levinson, 1988 |
| Human Immunodeficiency Virus | Muesing *et al.,* 1987; Hauber and Cullan, 1988; Jakobovits *et al.,* 1988; Feng and Holland, 1988; Takebe *et al.,* 1988; Rosen *et al.,* 1988; Berkhout *et al.,* 1989; Laspia *et al.,* 1989; Sharp and Marciniak, 1989; Braddock *et al.,* 1989 |
| Cytomegalovirus | Weber *et al.,* 1984; Boshart *et al.,* 1985; Foecking and Hofstetter, 1986 |
| Gibbon Ape Leukemia Virus | Holbrook *et al.,* 1987; Quinn *et al.,* 1989 |

TABLE 4

| Inducible Elements | | |
|---|---|---|
| Element | Inducer | References |
| MT II | Phorbol Ester (TFA) Heavy metals | Palmiter *et al.,* 1982; Haslinger and Karin, 1985; Searle *et al.,* 1985; Stuart *et al.,* 1985; Imagawa *et al.,* 1987, Karin *et al.,* 1987; Angel *et al.,* 1987b; McNeall *et al.,* 1989 |
| MMTV (mouse mammary tumor virus) | Glucocorticoids | Huang *et al.,* 1981; Lee *et al.,* 1981: Majors and Varmus. 1983; Chandler *et al.,* 1983; Lee *et al.,* 1984; Ponta *et al.,* 1985; Sakai *et al.,* 1988 |
| β-Interferon | poly(rl)x poly(rc) | Tavernier *et al.,* 1983 |
| Adenovirus 5 E2 | Ela | Imperiale and Nevins, 1984 |
| Collagenase | Phorbol Ester (TPA) | Angel *et al.,* 1987a |
| Stromelysin | Phorbol Ester (TPA) | Angel *et al.,* 1987b |
| SV40 | Phorbol Ester (TPA) | Angel *et al.,* 1987b |
| Murine MX Gene | Interferon, Newcastle Disease Virus | |
| GRP78 Gene | A23187 | Resendez *et al.,* 1988 |
| α-2-Macroglobulin | IL-6 | Kunz *et al.,* 1989 |
| Vimentin | Serum | Rittling *et al.,* 1989 |
| MHC Class I Gene H-2Kb | Interferon | Blanar *et al.,* 1989 |
| HSP70 | Ela, SV40 Large T Antigen | Taylor *et al.,* 1989; Taylor and Kingston. 1990a, b |
| Proliferin | Phorbol Ester-TPA | Mordacq and Linzer, 1989 |
| Tumor Necrosis Factor | FMA | Hensel *et al.,* 1989 |
| Thyroid Stimulating Hormone a Gene | Thyroid Hormone | Chatterjee *et al.,* 1989 |

Turning to the expression of the thrombopoietin proteins, once a suitable clone or clones have been obtained, whether they be cDNA based or genomic, one may proceed to prepare an expression system. The engineering of DNA segment(s) for expression in a prokaryotic or eukaryotic system may be performed by techniques generally known to those of skill in recombinant expression. It is believed that virtually any expression system may be employed in the expression of the proteins.

Both cDNA and genomic sequences are suitable for eukaryotic expression, as the host cell will generally process the genomic transcripts to yield functional mRNA for translation into protein. Generally speaking, it may be more convenient to employ as the recombinant gene a cDNA version of the gene. It is believed that the use of a cDNA version will provide advantages in that the size of the gene will generally be much smaller and more readily employed to transfect the targeted cell than will a genomic gene, which will typically be up to an order of magnitude or more larger than the cDNA gene. However, it is contemplated that a genomic version of a particular gene may be employed where desired.

In expression, one will typically include a polyadenylation signal to effect proper polyadenylation of the transcript. The nature of the polyadenylation signal is not believed to be crucial to the successful practice of the invention, and any such sequence may be employed. Preferred embodiments include the SV40 polyadenylation signal and the bovine growth hormone polyadenylation signal, convenient and known to function well in various target cells. Also contemplated as an element of the expression cassette is a terminator. These elements can serve to enhance message levels and to minimize read through from the cassette into other sequences.

A specific initiation signal also may be required for efficient translation of coding sequences. These signals include the ATG initiation codon and adjacent sequences. Exogenous translational control signals, including the ATG initiation codon, may need to be provided. One of ordinary skill in the art would readily be capable of determining this and providing the necessary signals. It is well known that the initiation codon must be "in-frame" with the reading frame of the desired coding sequence to ensure translation of the entire insert. The exogenous translational control signals and initiation codons can be either natural or synthetic. The efficiency of expression may be enhanced by the inclusion of appropriate transcription enhancer elements.

It is proposed that TPO proteins, polypeptides or peptides may be co-expressed with other selected proteins, wherein the proteins may be co-expressed in the same cell or an TPO gene(s) may be provided to a cell that already has another selected protein. Co-expression may be achieved by co-transfecting the cell with two distinct recombinant vectors, each bearing a copy of either of the respective DNA. Alternatively, a single recombinant vector may be constructed to include the coding regions for both of the proteins, which could then be expressed in cells transfected with the single vector. In either event, the term "co-expression" herein refers to the expression of both the TPO gene(s) and the other selected protein in the same recombinant cell.

As used herein, the terms "engineered" and "recombinant" cells or host cells are intended to refer to a cell into which an exogenous DNA segment or gene, such as a cDNA or gene encoding an TPO protein has been introduced. Therefore, engineered cells are distinguishable from naturally occurring cells which do not contain a recombinantly introduced exogenous DNA segment or gene. Engineered cells are thus cells having a gene or genes introduced through the hand of man. Recombinant cells include those having an introduced cDNA or genomic gene, and also include genes positioned adjacent to a promoter not naturally associated with the particular introduced gene.

To express a recombinant TPO protein, polypeptide or peptide, whether mutant or wild-type, in accordance with the present invention one would prepare an expression vector that comprises a wild-type, or mutant thrombopoietin protein-encoding nucleic acid under the control of one or more promoters. To bring a coding sequence "under the control of" a promoter, one positions the 5' end of the transcription initiation site of the transcriptional reading frame generally between about 1 and about 50 nucleotides "downstream" of (*i.e.*, 3' of) the chosen promoter. The "upstream" promoter stimulates transcription of the DNA and promotes expression of the encoded recombinant protein. This is the meaning of "recombinant expression" in this context.

Many standard techniques are available to construct expression vectors containing the appropriate nucleic acids and transcriptional/translational control sequences in order to achieve protein, polypeptide or peptide expression in a variety of host-expression systems. Cell types available for expression include, but are not limited to, bacteria, such as *E. coli* and *B.* subtilis transformed with recombinant bacteriophage DNA, plasmid DNA or cosmid DNA expression vectors.

Certain examples of prokaryotic hosts are *E. coli* strain RR1, *E. coli* LE392, *E. coli* B, *E. coli X* 1776 (ATCC No. 31537) as well as *E. coli* W3110 (F-, lambda-, prototrophic. ATCC No. 273325); bacilli such as *Bacillus subtilis;* and other enterobacteriaceae such as *Salmonella typhimurium, Serratia marcescens,* and various *Pseudomonas* species.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, *E. coli* is often transformed using derivatives of pBR322, a plasmid derived from an *E. coli* species. pBR322 contains genes for ampicillin and tetracycline resistance and thus provides easy means for identifying transformed cells. The pBR plasmid, or other microbial plasmid or phage must also contain, or be modified to contain, promoters which can be used by the microbial organism for expression of its own proteins.

In addition, phage vectors containing replicon and control sequences that are compatible with the host microorganism can be used as transforming vectors in connection with these hosts. For example, the phage lambda GEM™-11 may be utilized in making a recombinant phage vector which can be used to transform host cells, such as *E. coli* LE392.

Further useful vectors include pIN vectors (Inouye *et al.,* 1985); and pGEX vectors, for use in generating glutathione S-transferase (GST) soluble fusion proteins for later purification and separation or cleavage. Other suitable fusion proteins are those with β-galactosidase, ubiquitin. and the like.

Promoters that are most commonly used in recombinant DNA construction include the β-lactamase (penicillinase), lactose and tryptophan (trp) promoter systems. While these are the most commonly used, other microbial promoters have been discovered and utilized, and details concerning their nucleotide sequences have been published, enabling those of skill in the art to ligate them functionally with plasmid vectors.

The following details concerning recombinant protein production in bacterial cells, such as *E. coli,* are provided by way of exemplary information on recombinant protein production in general, the adaptation of which to a particular recombinant expression system will be known to those of skill in the art.

Bacterial cells, for example, *E. coli,* containing the expression vector are grown in any of a number of suitable media, for example, LB. The expression of the recombinant protein may be induced, e.g., by adding IPTG to the media or by switching incubation to a higher temperature. After culturing the bacteria for a further period, generally of between 2 and 24 h, the cells are collected by centrifugation and washed to remove residual media.

The bacterial cells are then lysed, for example, by disruption in a cell homogenizer and centrifuged to separate the dense inclusion bodies and cell membranes from the soluble cell components. This centrifugation can be performed under conditions whereby the dense inclusion bodies are selectively enriched by incorporation of sugars, such as sucrose, into the buffer and centrifugation at a selective speed.

If the recombinant protein is expressed in the inclusion bodies, as is the case in many instances, these can be washed in any of several solutions to remove some of the contaminating host proteins, then solubilized in solutions containing high concentrations of urea *(e.g.* 8M) or chaotropic agents such as guanidine hydrochloride in the presence of reducing agents, such as β-mercaptoethanol or DTT (dithiothreitol).

Under some circumstances, it may be advantageous to incubate the protein for several h under conditions suitable for the protein to undergo a refolding process into a conformation which more closely resembles that of the native protein. Such conditions generally include low protein concentrations, less than 500 mg/ml. low levels of reducing agent, concentrations of urea less than 2 M and often the presence of reagents such as a mixture of reduced and oxidized glutathione which facilitate the interchange of disulfide bonds within the protein molecule.

The refolding process can be monitored, for example, by SDS-PAGE, or with antibodies specific for the native molecule (which can be obtained from animals vaccinated with the native molecule or smaller quantities of recombinant protein). Following refolding, the protein can then be purified further and separated from the refolding mixture by chromatography on any of several supports including ion exchange resins, gel permeation resins or on a variety of affinity columns.

For expression in *Saccharomyces,* the plasmid YRp7, for example, is commonly used. This plasmid already contains the *trpl* gene which provides a selection marker for a mutant strain of yeast lacking the ability to grow in tryptophan, for example ATCC No. 44076 or PEP4-1. The presence of the *trpl* lesion as a characteristic of the yeast host cell genome then provides an effective environment for detecting transformation by growth in the absence of tryptophan.

Suitable promoting sequences in yeast vectors include the promoters for 3-phosphoglycerate kinase or other glycolytic enzymes, such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. In constructing suitable expression plasmids, the termination sequences associated with these genes are also ligated into the expression vector 3' of the sequence desired to be expressed to provide polyadenylation of the mRNA and termination.

Other suitable promoters, which have the additional advantage of transcription controlled by growth conditions, include the promoter region for alcohol dehydrogenase 2, isocytochrome C, acid phosphatase, degradative enzymes associated with nitrogen metabolism, and the aforementioned glyceraldehyde-3-phosphate dehydrogenase, and enzymes responsible for maltose and galactose utilization.

In addition to micro-organisms, cultures of cells derived from multicellular organisms may also be used as hosts. In principle, any such cell culture is workable, whether from vertebrate or invertebrate culture. In addition to mammalian cells, these include insect cell systems infected with recombinant virus expression vectors (*e.g.*, baculovirus); and plant cell systems infected with recombinant virus expression vectors (*e.g.*, cauliflower mosaic virus, CaMV; tobacco mosaic virus, TMV) or transformed with recombinant plasmid expression vectors (*e.g.*, Ti plasmid) containing one or more TPO protein, polypeptide or peptide coding sequences.

In a useful insect system, *Autograph califomica* nuclear polyhedrosis virus (AcNPV) is used as a vector to express foreign genes. The virus grows in *Spodoptera frugiperda* cells. The TPO protein, polypeptide or peptide coding sequences are cloned into non-essential regions (for example the polyhedrin gene) of the virus and placed under control of an AcNPV promoter (for example the polyhedrin promoter). Successful insertion of the coding sequences results in the inactivation of the polyhedrin gene and production of non-occluded recombinant virus (*i.e.,* virus lacking the proteinaceous coat coded for by the polyhedrin gene). These recombinant viruses are then used to infect *Spodoptera frugiperda* cells in which the inserted gene is expressed (e.g., U.S. Patent No. 4,215,051, Smith, incorporated herein by reference).

Examples of useful mammalian host cell lines are VERO and HeLa cells, Chinese hamster ovary (CHO) cell lines, W138, BHK, COS-7, 293, HepG2, 3T3, RIN and MDCK cell lines. In addition, a host cell strain may be chosen that modulates the expression of the inserted sequences, or modifies and processes the gene product in the specific fashion desired. Such modifications (*e.g.*, glycosylation) and processing (*e.g.,* cleavage) of protein products may be important for the function of the protein.

Different host cells have characteristic and specific mechanisms for the post-translational processing and modification of proteins. Appropriate cells lines or host systems can be chosen to ensure the correct modification and processing of the foreign protein expressed.

Expression vectors for use in mammalian cells ordinarily include an origin of replication (as necessary), a promoter located in front of the gene to be expressed, along with any necessary ribosome binding sites, RNA splice sites, polyadenylation site, and transcriptional terminator sequences. The origin of replication may be provided either by construction of the vector to include an exogenous origin, such as may be derived from SV40 or other viral (e.g., Polyoma. Adeno, VSV, BPV) source, or may be provided by the host cell chromosomal replication mechanism. If the vector is integrated into the host cell chromosome, the latter is often sufficient.

The promoters may be derived from the genome of mammalian cells *(e.g.,* metallothionein promoter) or from mammalian viruses *(e.g.,* the adenovirus late promoter; the vaccinia virus 7.5K promoter). Further, it is also possible, and may be desirable, to utilize promoter or control sequences normally associated with the TPO gene sequence(s), provided such control sequences are compatible with the host cell systems.

A number of viral based expression systems may be utilized, for example, commonly used promoters are derived from polyoma. Adenovirus 2, and most frequently Simian Virus 40 (SV40). The early and late promoters of SV40 virus are particularly useful because both are obtained easily from the virus as a fragment which also contains the SV40 viral origin of replication. Smaller or larger SV40 fragments may also be used, provided there is included the approximately 250 bp sequence extending from the *Hin*dIII site toward the *Bgl*I site located in the viral origin of replication.

In cases where an adenovirus is used as an expression vector, the coding sequences may be ligated to an adenovirus transcription/ translation control complex. e.g., the late promoter and tripartite leader sequence. This chimeric gene may then be inserted in the adenovirus genome by *in vitro* or *in vivo* recombination. Insertion in a non-essential region of the viral genome *(e.g.,* region E1, E3, or E4) will result in a recombinant virus that is viable and capable of expressing TPO proteins, polypeptides or peptides in infected hosts.

Specific initiation signals may also be required for efficient translation of TPO protein, polypeptide or peptide coding sequences. These signals include the ATG initiation codon and adjacent sequences. Exogenous translational control signals, including the ATG initiation codon, may additionally need to be provided. One of ordinary skill in the art would readily be capable of determining this and providing the necessary signals. It is well known that the initiation codon must be in-frame (or in-phase) with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements and transcription terminators.

In eukaryotic expression, one will also typically desire to incorporate into the transcriptional unit an appropriate polyadenylation site (e.g., 5'-AATAAA-3') if one was not contained within the original cloned segment. Typically, the poly A addition site is placed about 30 to 2000 nucleotides "downstream" of the termination site of the protein at a position prior to transcription termination.

For long-term, high-yield production of a recombinant TPO protein, polypeptide or peptide, stable expression is preferred. For example, cell lines that stably express constructs encoding an TPO protein, polypeptide or peptide may be engineered. Rather than using expression vectors that contain viral origins of replication, host cells can be transformed with vectors controlled by appropriate expression control elements *(e.g.,* promoter, enhancer, sequences, transcription terminators, polyadenylation sites, *etc.),* and a selectable marker. Following the introduction of foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines.

A number of selection systems may be used, including, but not limited to, the herpes simplex virus thymidine kinase (tk), hypoxanthine-guanine phosphoribosyltransferase (hgprt) and adenine phosphoribosyltransferase (aprt) genes, in tk'. hgprt' or aprt' cells. respectively. Also. antimetabolite resistance can be used as the basis of selection for dihydrofolate reductase (dhfr), that confers resistance to methotrexate; gpt, that confers resistance to mycophenolic acid; neomycin (neo), that confers resistance to the aminoglycoside G-418; and hygromycin (hygro), that confers resistance to hygromycin.

Animal cells can be propagated *in vitro* in two modes: as non-anchorage dependent cells growing in suspension throughout the bulk of the culture or as anchorage-dependent cells requiring attachment to a solid substrate for their propagation (i.e., a monolayer type of cell growth).

Non-anchorage dependent or suspension cultures from continuous established cell lines are the most widely used means of large scale production of cells and cell products. However, suspension cultured cells have limitations, such as tumorigenic potential and lower protein production than adherent cells.

Large scale suspension culture of mammalian cells in stirred tanks is a common method for production of recombinant proteins. Two suspension culture reactor designs are in wide use - the stirred reactor and the airlift reactor. The stirred design has successfully been used on an 8000 liter capacity for the production of interferon. Cells are grown in a stainless steel tank with a height-to-diameter ratio of 1:1 to 3:1. The culture is usually mixed with one or more agitators, based on bladed disks or marine propeller patterns. Agitator systems offering less shear forces than blades have been described. Agitation may be driven either directly or indirectly by magnetically coupled drives. Indirect drives reduce the risk of microbial contamination through seals on stirrer shafts.

The airlift reactor, also initially described for microbial fermentation and later adapted for mammalian culture, relies on a gas stream to both mix and oxygenate the culture. The gas stream enters a riser section of the reactor and drives circulation. Gas disengages at the culture surface, causing denser liquid free of gas bubbles to travel downward in the downcomer section of the reactor. The main advantage of this design is the simplicity and lack of need for mechanical mixing. Typically, the height-to-diameter ratio is 10:1. The airlift reactor scales up relatively easily, has good mass transfer of gases and generates relatively low shear forces.

It is contemplated that the TPO proteins, polypeptides or peptides of the invention may be "overexpressed", *i.e.*, expressed in increased levels relative to its natural expression in cells. Such overexpression may be assessed by a variety of methods, including radio-labeling and/or protein purification. However, simple and direct methods are preferred, for example, those involving SDS/PAGE and protein staining or western blotting, followed by quantitative analyses, such as densitometric scanning of the resultant gel or blot. A specific increase in the level of the recombinant protein, polypeptide or peptide in comparison to the level in natural cells is indicative of overexpression, as is a relative abundance of the specific protein, polypeptides or peptides in relation to the other proteins produced by the host cell and, *e.g.,* visible on a gel.

### C. Nucleic Acid Detection

In addition to their use in directing the expression of TPO proteins, polypeptides or peptides, the nucleic acid sequences disclosed herein also have a variety of other uses. For example, they also have utility as probes or primers in nucleic acid hybridization embodiments.

### 1. Hybridization

The use of a hybridization probe of between 17 and 100 nucleotides in length, or in some aspect of the invention even up to 1-2 kb or more in length, allows the formation of a duplex molecule that is both stable and selective. Molecules having complementary sequences over stretches greater than 20 bases in length are generally preferred. in order to increase stability and selectivity of the hybrid, and thereby improve the quality and degree of particular hybrid molecules obtained. One will generally prefer to design nucleic acid molecules having stretches of 20 to 30 nucleotides, or even longer where desired. Such fragments may be readily prepared by, for example, directly synthesizing the fragment by chemical means or by introducing selected sequences into recombinant vectors for recombinant production.

Accordingly, the nucleotide sequences of the invention may be used for their ability to selectively form duplex molecules with complementary stretches of genes or RNAs or to provide primers for amplification of DNA or RNA from tissues. Depending on the application envisioned, one will desire to employ varying conditions of hybridization to achieve varying degrees of selectivity of probe towards target sequence.

For applications requiring high selectivity, one will typically desire to employ relatively stringent conditions to form the hybrids, *e.g.*, one will select relatively low salt and/or high temperature conditions, such as provided by about 0.02 M to about 0.10 M NaCl at temperatures of about 50°C to about 70°C. Such high stringency conditions tolerate little, if any, mismatch between the probe and the template or target strand, and would be particularly suitable for isolating specific genes or detecting specific mRNA transcripts. It is generally appreciated that conditions can be rendered more stringent by the addition of increasing amounts of formamide.

For certain applications, for example, substitution of nucleotides by site-directed mutagenesis, it is appreciated that lower stringency conditions are required. Under these conditions, hybridization may occur even though the sequences of probe and target strand are not perfectly complementary, but are mismatched at one or more positions. Conditions may be rendered less stringent by increasing salt concentration and decreasing temperature. For example, a medium stringency condition could be provided by about 0.1 to 0.25 M NaCl at temperatures of about 37°C to about 55°C, while a low stringency condition could be provided by about 0.15 M to about 0.9 M salt, at temperatures ranging from about 20°C to about 55°C. Thus, hybridization conditions can be readily manipulated depending on the desired results.

In other embodiments. hybridization may be achieved under conditions of, for example, 50 mM Tris-HCl (pH 8.3), 75 mM KCl, 3 mM $MgCl_2$, 1.0 mM dithiothreitol, at temperatures between approximately 20°C to about 37°C. Other hybridization conditions utilized could include approximately 10 mM Tris-HCl (pH 8.3), 50 mM KCl, 1.5 mM $MgCl_2$, at temperatures ranging from approximately 40°C to about 72°C.

In certain embodiments. it will be advantageous to employ nucleic acid sequences in combination with an appropriate means, such as a label, for determining hybridization. A wide variety of appropriate indicator means are known in the art, including fluorescent, radioactive, enzymatic or other ligands, such as avidin/biotin, which are capable of being detected. In preferred embodiments, one may desire to employ a fluorescent label or an enzyme tag such as urease. alkaline phosphatase or peroxidase, instead of radioactive or other environmentally undesirable reagents. In the case of enzyme tags, colorimetric indicator substrates are known that can be employed to provide a detection means visible to the human eye or spectrophotometrically, to identify specific hybridization with complementary nucleic acid-containing samples.

In general. it is envisioned that the hybridization probes described herein will be useful both as reagents in solution hybridization, as in PCR$^{TM}$, for detection of expression of corresponding genes, as well as in embodiments employing a solid phase. In embodiments involving a solid phase. the test DNA (or RNA) is adsorbed or otherwise affixed to a selected matrix or surface. This fixed, single-stranded nucleic acid is then subjected to hybridization with selected probes under desired conditions. The selected conditions will depend on the particular circumstances based on the particular criteria required (depending, for example, on the G+C content, type of target nucleic acid, source of nucleic acid, size of hybridization probe, *etc.).* Following washing of the hybridized surface to remove non-specifically bound probe molecules, hybridization is detected, or even quantified, by means of the label.

### 2. Amplification and PCR$^{TM}$

Nucleic acid used as a template for amplification is isolated from cells contained in the biological sample, according to standard methodologies (Sambrook *et al.,* 1989). The nucleic acid may be genomic DNA or fractionated or whole cell RNA. Where RNA is used, it may be desired to convert the RNA to a complementary DNA. In one embodiment, the RNA is whole cell RNA and is used directly as the template for amplification.

Pairs of primers that selectively hybridize to nucleic acids corresponding to thrombopoietin genes are contacted with the isolated nucleic acid under conditions that permit selective hybridization. The term "primer", as defined herein, is meant to encompass any nucleic acid that is capable of priming the synthesis of a nascent nucleic acid in a template-dependent process. Typically, primers are oligonucleotides from ten to twenty or thirty base pairs in length, but longer sequences can be employed. Primers may be provided in double-stranded or single-stranded form, although the single-stranded form is preferred.

Once hybridized, the nucleic acid:primer complex is contacted with one or more enzymes that facilitate template-dependent nucleic acid synthesis. Multiple rounds of amplification, also referred to as "cycles," are conducted until a sufficient amount of amplification product is produced.

Next, the amplification product is detected. In certain applications, the detection may be performed by visual means. Alternatively, the detection may involve indirect identification of the product via chemiluminescence, radioactive scintigraphy of incorporated radiolabel or fluorescent label or even *via* a system using electrical or thermal impulse signals (Affymax technology).

A number of template dependent processes are available to amplify the marker sequences present in a given template sample. One of the best known amplification methods is the polymerase chain reaction (referred to as PCR$^{TM}$) which is described in detail in U.S. Patent Nos. 4,683,195, 4,683,202 and 4,800,159.

Briefly, in PCR$^{TM}$, two primer sequences are prepared that are complementary to regions on opposite complementary strands of the marker sequence. An excess of deoxynucleoside triphosphates are added to a reaction mixture along with a DNA polymerase, *e.g., Taq* polymerase. If the marker sequence is present in a sample, the primers will bind to the marker and the polymerase will cause the primers to be extended along the marker sequence by adding on nucleotides. By raising and lowering the temperature of the reaction mixture, the extended primers will dissociate from the marker to form reaction products, excess primers will bind to the marker and to the reaction products and the process is repeated.

A reverse transcriptase PCR$^{TM}$ amplification procedure may be performed in order to quantify the amount of mRNA amplified. Methods of reverse transcribing RNA into cDNA are well known and described in Sambrook *et al.,* 1989. Alternative methods for reverse transcription utilize thermostable, RNA-dependent DNA polymerases. These methods are described in WO 90/07641, filed December 21, 1990. Polymerase chain reaction methodologies are well known in the art.

Another method for amplification is the ligase chain reaction ("LCR"), disclosed in EPA No. 320 308. In LCR, two complementary probe pairs are prepared, and in the presence of the target sequence. each pair will bind to opposite complementary strands of the target such that they abut. In the presence of a ligase, the two probe pairs will link to form a single unit. By temperature cycling, as in PCR$^{TM}$, bound ligated units dissociate from the target and then serve as "target sequences" for ligation of excess probe pairs. U.S. Patent 4,883,750 describes a method similar to LCR for binding probe pairs to a target sequence.

Qbeta Replicase, described in PCT Application No. PCT/US87/00880, may also be used as still another amplification method in the present invention. In this method, a replicative sequence of RNA that has a region complementary to that of a target is added to a sample in the presence of an RNA polymerase. The polymerase will copy the replicative sequence that can then be detected.

An isothermal amplification method, in which restriction endonucleases and ligases are used to achieve the amplification of target molecules that contain nucleotide 5'-[α-thio]-triphosphates in one strand of a restriction site may also be useful in the amplification of nucleic acids in the present invention.

Strand Displacement Amplification (SDA) is another method of carrying out isothermal amplification of nucleic acids which involves multiple rounds of strand displacement and synthesis. i.e., nick translation. A similar method. called Repair Chain Reaction (RCR), involves annealing several probes throughout a region targeted for amplification, followed by a repair reaction in which only two of the four bases are present. The other two bases can be added as biotinylated derivatives for easy detection. A similar approach is used in SDA. Target specific sequences can also be detected using a cyclic probe reaction (CPR). In CPR, a probe having 3' and 5' sequences of non-specific DNA and a middle sequence of specific RNA is hybridized to DNA that is present in a sample. Upon hybridization, the reaction is treated with RNase H, and the products of the probe identified as distinctive products that are released after digestion. The original template is annealed to another cycling probe and the reaction is repeated.

Still another amplification methods described in GB Application No. 2 202 328, and in PCT Application No. PCT/US89/01025, may be used in accordance with the present invention. In the former application, "modified" primers are used in a PCR™-like, template- and enzyme-dependent synthesis. The primers may be modified by labeling with a capture moiety *(e.g.,* biotin) and/or a detector moiety *(e.g.,* enzyme). In the latter application, an excess of labeled probes are added to a sample. In the presence of the target sequence, the probe binds and is cleaved catalytically. After cleavage, the target sequence is released intact to be bound by excess probe. Cleavage of the labeled probe signals the presence of the target sequence.

Other nucleic acid amplification procedures include transcription-based amplification systems (TAS), including nucleic acid sequence based amplification (NASBA) and 3SR (Gingeras *et al.,* PCT Application WO 88/10315). In NASBA, the nucleic acids can be prepared for amplification by standard phenol/chloroform extraction, heat denaturation of a clinical sample, treatment with lysis buffer and minispin columns for isolation of DNA and RNA or guanidinium chloride extraction of RNA. These amplification techniques involve annealing a primer which has target specific sequences. Following polymerization, DNA/RNA hybrids are digested with RNase H while double stranded DNA molecules are heat denatured again. In either case the single stranded DNA is made fully double stranded by addition of second target specific primer. followed by polymerization. The double-stranded DNA molecules are then multiply transcribed by an RNA polymerase such as T7 or SP6. In an isothermal cyclic reaction, the RNA's are reverse transcribed into single stranded DNA, which is then converted to double stranded DNA, and then transcribed once again with an RNA polymerase such as T7 or SP6. The resulting products, whether truncated or complete, indicate target specific sequences.

Davey *et al.,* EPA No. 329 822 disclose a nucleic acid amplification process involving cyclically synthesizing single-stranded RNA ("ssRNA"), ssDNA, and double-stranded DNA (dsDNA), which may be used in accordance with the present invention. The ssRNA is a template for a first primer oligonucleotide. which is elongated by reverse transcriptase (RNA-dependent DNA polymerase). The RNA is then removed from the resulting DNA:RNA duplex by the action of ribonuclease H (RNase H, an RNase specific for RNA in duplex with either DNA or RNA). The resultant ssDNA is a template for a second primer, which also includes the sequences of an RNA polymerase promoter (exemplified by T7 RNA polymerase) 5' to its homology to the template. This primer is then extended by DNA polymerase (exemplified by the large "Klenow" fragment of *E. coli* DNA polymerase I), resulting in a double-stranded DNA ("dsDNA") molecule, having a sequence identical to that of the original RNA between the primers and having additionally, at one end, a promoter sequence. This promoter sequence can be used by the appropriate RNA polymerase to make many RNA copies of the DNA. These copies can then re-enter the cycle leading to very swift amplification. With proper choice of enzymes, this amplification can be done isothermally without addition of enzymes at each cycle. Because of the cyclical nature of this process, the starting sequence can be chosen to be in the form of either DNA or RNA.

Miller *et al.,* PCT Application WO 89/06700 disclose a nucleic acid sequence amplification scheme based on the hybridization of a promoter/primer sequence to a target single-stranded DNA ("ssDNA") followed by transcription of many RNA copies of the sequence. This scheme is not cyclic, i.e., new templates are not produced from the resultant RNA transcripts. Other amplification methods include "RACE" and "one-sided PCR™" (Frohman, 1990, incorporated herein by reference).

Methods based on ligation of two (or more) oligonucleotides in the presence of nucleic acid having the sequence of the resulting "di-oligonucleotide", thereby amplifying the di-oligonucleotide, may also be used in the amplification step.

Following any amplification, it may be desirable to separate the amplification product from the template and the excess primer for the purpose of determining whether specific amplification has occurred. In one embodiment, amplification products are separated by agarose, agarose-acrylamide or polyacrylamide gel electrophoresis using standard methods (Sambrook *et al.,* 1989).

Alternatively, chromatographic techniques may be employed to effect separation. There are many kinds of chromatography which may be used in the present invention: adsorption. partition. ion-exchange and molecular sieve. and many specialized techniques for using them including column, paper, thin-layer and gas chromatography.

Amplification products must be visualized in order to confirm amplification of the marker sequences. One typical visualization method involves staining of a gel with ethidium bromide and visualization under UV light. Alternatively, if the amplification products are integrally labeled with radio- or fluorometrically-labeled nucleotides, the amplification products can then be exposed to x-ray film or visualized under the appropriate stimulating spectra, following separation.

Visualization may be achieved indirectly. Following separation of amplification products, a labeled, nucleic acid probe is brought into contact with the amplified marker sequence. The probe preferably is conjugated to a chromophore but may be radiolabeled. In another embodiment, the probe is conjugated to a binding partner, such as an antibody or biotin, and the other member of the binding pair carries a detectable moiety.

Detection may be by Southern blotting and hybridization with a labeled probe. The techniques involved in Southern blotting are well known to those of skill in the art and can be found in many standard books on molecular protocols. See Sambrook *et al.,* 1989. Briefly, amplification products are separated by gel electrophoresis. The gel is then contacted with a membrane, such as nitrocellulose, permitting transfer of the nucleic acid and non-covalent binding. Subsequently, the membrane is incubated with a chromophore-conjugated probe that is capable of hybridizing with a target amplification product. Detection is by exposure of the membrane to x-ray film or ion-emitting detection devices.

One example of the foregoing is described in U.S. Patent No. 5,279,721, which discloses an apparatus and method for the automated electrophoresis and transfer of nucleic acids. The apparatus permits electrophoresis and blotting without external manipulation of the gel and is ideally suited to carrying out methods according to the present invention.

### 3. Other Assays

Other methods for genetic screening to accurately detect mutations in genomic DNA, cDNA or RNA samples may be employed, depending on the specific situation.

Historically, a number of different methods have been used to detect point mutations, including denaturing gradient gel electrophoresis ("DGGE"), restriction enzyme polymorphism analysis, chemical and enzymatic cleavage methods, and others. The more common procedures currently in use include direct sequencing of target regions amplified by PCR$^{TM}$ (see above) and single-strand conformation polymorphism analysis ("SSCP").

Another method of screening for point mutations is based on RNase cleavage of base pair mismatches in RNA/DNA and RNA/RNA heteroduplexes. As used herein, the term "mismatch" is defined as a region of one or more unpaired or mispaired nucleotides in a double-stranded RNA/RNA. RNA/DNA or DNA/DNA molecule. This definition thus includes mismatches due to insertion/deletion mutations, as well as single and multiple base point mutations.

U.S. Patent No. 4,946,773 describes an RNase A mismatch cleavage assay that involves annealing single-stranded DNA or RNA test samples to an RNA probe, and subsequent treatment of the nucleic acid duplexes with RNase A. After the RNase cleavage reaction, the RNase is inactivated by proteolytic digestion and organic extraction, and the cleavage products are denatured by heating and analyzed by electrophoresis on denaturing polyacrylamide gels. For the detection of mismatches, the single-stranded products of the RNase A treatment, electrophoretically separated according to size, are compared to similarly treated control duplexes. Samples containing smaller fragments (cleavage products) not seen in the control duplex are scored as positive.

Currently available RNase mismatch cleavage assays, including those performed according to U.S. Patent No. 4,946,773, require the use of radiolabeled RNA probes. Myers and Maniatis in U.S. Patent No. 4,946,773 describe the detection of base pair mismatches using RNase A. Other investigators have described the use of an E. *coli* enzyme, RNase I, in mismatch assays. Because it has broader cleavage specificity than RNase A, RNase I would be a desirable enzyme to employ in the detection of base pair mismatches if components can be found to decrease the extent of non-specific cleavage and increase the frequency of cleavage of mismatches. The use of RNase I for mismatch detection is described in literature from Promega Biotech. Promega markets a kit containing RNase I that is shown in their literature to cleave three out of four known mismatches, provided the enzyme level is sufficiently high.

The RNase protection assay was first used to detect and map the ends of specific mRNA targets in solution. The assay relies on being able to easily generate high specific activity radiolabeled RNA probes complementary to the mRNA of interest by *in vitro* transcription. Originally, the templates for *in vitro* transcription were recombinant plasmids containing bacteriophage promoters. The probes are mixed with total cellular RNA samples to permit hybridization to their complementary targets, then the mixture is treated with RNase to degrade excess unhybridized probe. Also, as originally intended, the RNase used is specific for single-stranded RNA, so that hybridized double-stranded probe is protected from degradation. After inactivation and removal of the RNase, the protected probe (which is proportional in amount to the amount of target mRNA that was present) is recovered and analyzed on a polyacrylamide gel.

The RNase Protection assay was adapted for detection of single base mutations. In this type of RNase A mismatch cleavage assay, radiolabeled RNA probes transcribed *in vitro* from wild-type sequences, are hybridized to complementary target regions derived from test samples. The test target generally comprises DNA (either genomic DNA or DNA amplified by cloning in plasmids or by PCR<sup>TM</sup>), although RNA targets (endogenous mRNA) have occasionally been used. If single nucleotide (or greater) sequence differences occur between the hybridized probe and target, the resulting disruption in Watson-Crick hydrogen bonding at that position ("mismatch") can be recognized and cleaved in some cases by single-strand specific ribonuclease. To date, RNase A has been used almost exclusively for cleavage of single-base mismatches, although RNase I has recently been shown as useful also for mismatch cleavage. There are recent descriptions of using the MutS protein and other DNA-repair enzymes for detection of single-base mismatches.

**D. Mutagenesis**

Site-specific mutagenesis is a technique useful in the preparation of individual peptides, or biologically functional equivalent proteins or peptides, through specific mutagenesis of the underlying DNA. The technique further provides a ready ability to prepare and test sequence variants, incorporating one or more of the foregoing considerations, by introducing one or more nucleotide sequence changes into the DNA. Site-specific mutagenesis allows the production of mutants through the use of specific oligonucleotide sequences which encode the DNA sequence of the desired mutation, as well as a sufficient number of adjacent nucleotides, to provide a primer sequence of sufficient size and sequence complexity to form a stable duplex on both sides of the deletion junction being traversed. Typically, a primer of about 17 to 25 nucleotides in length is preferred. with about 5 to 10 residues on both sides of the junction of the sequence being altered.

In general, the technique of site-specific mutagenesis is well known in the art. As will be appreciated, the technique typically employs a bacteriophage vector that exists in both a single stranded and double stranded form. Typical vectors useful in site-directed mutagenesis include vectors such as the M13 phage. These phage vectors are commercially available and their use is generally well known to those skilled in the art. Double stranded plasmids are also routinely employed in site directed mutagenesis, which eliminates the step of transferring the gene of interest from a phage to a plasmid.

In general, site-directed mutagenesis is performed by first obtaining a single-stranded vector. or melting of two strands of a double stranded vector which includes within its sequence a DNA sequence encoding the desired protein. An oligonucleotide primer bearing the desired mutated sequence is synthetically prepared. This primer is then annealed with the single-stranded DNA preparation, and subjected to DNA polymerizing enzymes such as *E. coli* polymerase I Klenow fragment, in order to complete the synthesis of the mutation-bearing strand. Thus, a heteroduplex is formed wherein one strand encodes the original non-mutated sequence and the second strand bears the desired mutation. This heteroduplex vector is then used to transform appropriate cells, such as *E. coli* cells, and clones are selected that include recombinant vectors bearing the mutated sequence arrangement.

The preparation of sequence variants of the selected gene using site-directed mutagenesis is provided as a means of producing potentially useful species and is not meant to be limiting, as there are other ways in which sequence variants of genes may be obtained. For example, recombinant vectors encoding the desired gene may be treated with mutagenic agents, such as hydroxylamine, to obtain sequence variants. Methods for preparing modified nucleic acids and amino acid sequences for TPO are exemplified in U.S. Patent Nos. 5,753,083, 5,696,250, 5,641,655, 5,795,569, 5,766,581 and 5,766,897.

**IV. TPO Proteins, Polypeptides, and Peptides**

The present invention also provides purified, and in preferred embodiments, substantially purified. TPO proteins, polypeptides, or peptides. The term "purified TPO proteins, polypeptides, or peptides" as used herein, is intended to refer to an TPO proteinaceous composition, isolatable from mammalian cells or recombinant host cells, wherein the TPO protein. polypeptide, or peptide is purified to any degree relative to its naturally-obtainable state, *i.e.*, relative to its purity within a cellular extract. A purified TPO protein, polypeptide, or peptide therefore also refers to a wild-type or mutant TPO protein, polypeptide, or peptide free from the environment in which it naturally occurs.

The mature thrombopoietin proteins may be full length proteins, such as being 322 amino acids in length. The nucleotide and protein, polypeptide and peptide sequences for TPO and other cytokines and peptide hormones have been previously disclosed, and may be found at the National Center for Biotechnology Information's Genbank and GenPept databases (http://www.ncbi.nlm.nih.gov/). The amino acid sequences for TPO contemplated as being useful in the present invention include, but are not limited to, GenPept accession numbers AROO4221, AR004220, 3986139, 3986137, 3986135, 533217, 533215, P40225, P49745, P42706, P42705, P40226, 150264, 150263, 150262, 2351118, 971277, 577320, JC4125, 158350, JC4227, S45330, 180105, 1110579, 914226, 2013345A, 1401250, 1401248, 1401246, and 508541. The amino acid sequences for human TPO contemplated as being useful in the present invention include, but are not limited to, GenPept accession numbers AROO4221, 3986139, 3986137, 3986135, 533217, 533215, P40225, 150264, 150263, 2351118, 577320, 180105, 914226, 1401250, 1401248 and 1401246. A preferred amino acid sequence for TPO is shown in SEQ ID NO:2. The thrombopoietin proteins, polypeptides and peptides may also be less then full length proteins, such as individual domains, regions or even epitopic peptides. Where less than full length thrombopoietin proteins are concerned the most preferred will be those containing predicted immunogenic sites and those containing the functional domains identified herein.

Generally, "purified" will refer to an TPO protein, polypeptide, or peptide composition that has been subjected to fractionation to remove various non-thrombopoietin protein, polypeptide, or peptide, and which composition substantially retains its thrombopoietin activity, as may be assessed, for example, by the protein thrombopoietin assays, as described herein below.

Where the term "substantially purified" is used, this will refer to a composition in which the TPO protein, polypeptide. or peptide forms the major component of the composition, such as constituting about 50% of the proteins in the composition or more. In preferred embodiments, a substantially purified protein will constitute more than 60%, 70%, 80%, 90%, 95%, 99% or even more of the proteins in the composition.

A peptide, polypeptide or protein that is "purified to homogeneity," as applied to the present invention, means that the peptide, polypeptide or protein has a level of purity where the peptide, polypeptide or protein is substantially free from other proteins and biological components. For example, a purified peptide, polypeptide or protein will often be sufficiently free of other protein components so that degradative sequencing may be performed successfully.

Various methods for quantifying the degree of purification of thrombopoietin proteins, polypeptides, or peptides will be known to those of skill in the art in light of the present disclosure. These include, for example, determining the specific thrombopoietin protein activity of a fraction, or assessing the number of polypeptides within a fraction by gel electrophoresis.

To purify an thrombopoietin protein, polypeptide, or peptide a natural or recombinant composition comprising at least some thrombopoietin proteins, polypeplides. or peptides will be subjected to fractionation to remove various non-thrombopoietin components from the composition. In addition to those techniques described in detail herein below, various other techniques suitable for use in protein purification will be well known to those of skill in the art. These include, for example, precipitation with ammonium sulfate, PEG, antibodies and the like or by heat denaturation. followed by centrifugation: chromatography steps such as ion exchange, gel filtration, reverse phase, hydroxylapatite. lectin affinity and other affinity chromatography steps; isoelectric focusing; gel electrophoresis; and combinations of such and other techniques.

Another example is the purification of an thrombopoietin fusion protein using a specific binding partner. Such purification methods are routine in the art. As the present invention provides DNA sequences for thrombopoietin proteins, any fusion protein purification method can now be practiced. This is exemplified by the generation of an thrombopoietin-glutathione S-transferase fusion protein, expression in E. *coli,* and isolation to homogeneity using affinity chromatography on glutathione-agarose or the generation of a polyhistidine tag on the N- or C-terminus of the protein, and subsequent purification using Ni-affinity chromatography. However, given the TPO DNA and proteins are known, any purification method can now be employed.

Although preferred for use in certain embodiments. there is no general requirement that the TPO protein, polypeptide, or peptide always be provided in their most purified state. Indeed, it is contemplated that less substantially purified TPO protein, polypeptide or peptide, which are nonetheless enriched in thrombopoietin protein compositions, relative to the natural state, will have utility in certain embodiments.

Methods exhibiting a lower degree of relative purification may have advantages in total recovery of protein product. or in maintaining the activity of an expressed protein. Inactive products also have utility in certain embodiments, such as, *e.g.*, in determining antigenicity *via* antibody generation.

## V. Antibodies to Thrombopoietin Proteins

### A. Epitopic Core Sequences

Peptides corresponding to one or more antigenic determinants, or "epitopic core regions", of the thrombopoietin proteins. polypeptides and peptides can also be prepared. Such peptides should generally be at least five or six amino acid residues in length, will preferably be about 10, 15, 20, 25 or about 30 amino acid residues in length, and may contain up to about 35-50 residues or so.

Synthetic peptides will generally be about 35 residues long, which is the approximate upper length limit of automated peptide synthesis machines, such as those available from Applied Biosystems (Foster City, CA). Longer peptides may also be prepared, *e.g.,* by recombinant means.

U.S. Patent 4,554,101, (Hopp) incorporated herein by reference, teaches the identification and preparation of epitopes from primary amino acid sequences on the basis of hydrophilicity. Through the methods disclosed in Hopp, one of skill in the art would be able to identify epitopes from within an amino acid sequence such as the TPO sequence of SEQ ID NO:2.

Numerous scientific publications have also been devoted to the prediction of secondary structure, and to the identification of epitopes, from analyses of amino acid sequences (Chou and Fasman, 1974a,b; 1978a,b, 1979). Any of these may be used, if desired, to supplement the teachings of Hopp in U.S. Patent 4,554,101.

Moreover, computer programs are currently available to assist with predicting antigenic portions and epitopic core regions of proteins. Examples include those programs based upon the Jameson-Wolf analysis (Jameson and Wolf, 1988; Wolf *et al.,* 1988), the program PepPlot® (Brutlag *et al.,* 1990; Weinberger *et al.,* 1985), and other new programs for protein tertiary structure prediction (Fetrow and Bryant, 1993). Another commercially available software program capable of carrying out such analyses is Mac Vector (IBI, New Haven, CT).

In further embodiments, major antigenic determinants of a polypeptide may be identified by an empirical approach in which portions of the gene encoding the polypeptide are expressed in a recombinant host, and the resulting proteins tested for their ability to elicit an immune response. For example, PCR$^{TM}$ can be used to prepare a range of peptides lacking successively longer fragments of the C-terminus of the protein. The immunoactivity of each of these peptides is determined to identify those fragments or domains of the polypeptide that are immunodominant. Further studies in which only a small number of amino acids are removed at each iteration then allows the location of the antigenic determinants of the polypeptide to be more precisely determined.

Another method for determining the major antigenic determinants of a polypeptide is the SPOTs$^{TM}$ system (Genosys Biotechnologies, Inc., The Woodlands, TX). In this method, overlapping peptides are synthesized on a cellulose membrane, which following synthesis and deprotection, is screened using a polyclonal or monoclonal antibody. The antigenic determinants of the peptides which are initially identified can be further localized by performing subsequent syntheses of smaller peptides with larger overlaps, and by eventually replacing individual amino acids at each position along the immunoreactive peptide.

Once one or more such analyses are completed, polypeptides are prepared that remove at least the essential features of one or more antigenic determinants. The peptides are then employed in the methods of the invention to reduce the production of anti-TPO antibodies when TPO is administered to a mammal. Minigenes or gene fusions encoding these determinants can also be constructed and inserted into expression vectors by standard methods, for example, using PCR$^{TM}$ cloning methodology.

As used herein, the term "antibody" is intended to refer broadly to any immunologic binding agent such as IgG, IgM, IgA, IgD and IgE. Generally, IgG and/or IgM are preferred because they are the most common antibodies in the physiological situation and because they are most easily made in a laboratory setting.

The term "antibody" is used to refer to any antibody-like molecule that has an antigen binding region, and includes antibody fragments such as Fab', Fab, F(ab')$_2$, single domain antibodies (DABs), Fv, scFv (single chain Fv), and the like. The techniques for preparing and using various antibody-based constructs and fragments are well known in the art. Means for preparing and characterizing antibodies are also well known in the art (See, e.g., Antibodies: A Laboratory Manual, Cold Spring Harbor Laboratory, 1988).

## A. Immunodetection Methods

In general, the immunobinding methods include obtaining a sample suspected of containing an thrombopoietin protein, polypeptide or peptide. and contacting the sample with a first anti-TPO antibody, as the case may be, under conditions effective to allow the formation of immunocomplexes. The removal of an antigenic region may be determined by the lack of detectable binding of an altered or mutated TPO molecule to an antibody known to bind TPO.

These methods include methods for purifying wild-type or mutant thrombopoietin proteins, polypeptides or peptides as may be employed in purifying wild-type or mutant thrombopoietin proteins, polypeptides or peptides from patients' samples or for purifying recombinantly expressed wild-type or mutant thrombopoietin proteins, polypeptides or peptides. In these instances, the antibody removes the antigenic wild-type or mutant thrombopoietin protein, polypeptide or peptide component from a sample. The antibody will preferably be linked to a solid support, such as in the form of a column matrix, and the sample suspected of containing the wild-type or mutant thrombopoietin protein antigenic component will be applied to the immobilized antibody. The unwanted components will be washed from the column, leaving the antigen immunocomplexed to the immobilized antibody, which wild-type or mutant thrombopoietin protein antigen is then collected by removing the wild-type or mutant thrombopoietin protein, polypeptide or peptide from the column.

The immunobinding methods also include methods for detecting or quantifying the amount of a wild-type or mutant thrombopoietin protein reactive component in a sample, which methods require the detection or quantification of any immune complexes formed during the binding process. Here, one would obtain a sample suspected of containing a wild-type or mutant thrombopoietin protein, polypeptide or peptide, and contact the sample with an antibody against wild-type or mutant thrombopoietin. and then detect or quantify the amount of immune complexes formed under the specific conditions.

Contacting the chosen biological sample with the antibody under conditions effective and for a period of time sufficient to allow the formation of immune complexes (primary immune complexes) is generally a matter of simply adding the antibody composition to the sample and incubating the mixture for a period of time lone enough for the antibodies to form immune complexes with, *i.e.,* to bind to, any thrombopoietin protein antigens present. After this time, the sample-antibody composition, such as a tissue section, ELISA plate, dot blot or western blot, will generally be washed to remove any non-specifically bound antibody species, allowing only those antibodies specifically bound within the primary immune complexes to be detected.

In general, the detection of immunocomplex formation is well known in the art and may be achieved through the application of numerous approaches. These methods are generally based upon the detection of a label or marker, such as any of those radioactive, fluorescent, biological or enzymatic tags. U.S. Patents concerning the use of such labels include 3,817,837; 3,850.752; 3,939,350; 3,996,345; 4,277,437; 4,275,149 and 4,366,241. Of course, one may find additional advantages through the use of a secondary binding ligand such as a second antibody or a biotin/avidin ligand binding arrangement, as is known in the art.

The thrombopoietin antibody employed in the detection may itself be linked to a detectable label, wherein one would then simply detect this label, thereby allowing the amount of the primary immune complexes in the composition to be determined. Alternatively, the first antibody that becomes bound within the primary immune complexes may be detected by means of a second binding ligand that has binding affinity for the antibody. In these cases, the second binding ligand may be linked to a detectable label. The second binding ligand is itself often an antibody, which may thus be termed a "secondary" antibody. The primary immune complexes are contacted with the labeled, secondary binding ligand, or antibody, under conditions effective and for a period of time sufficient to allow the formation of secondary immune complexes. The secondary immune complexes are then generally washed to remove any non-specifically bound labeled secondary antibodies or ligands, and the remaining label in the secondary immune complexes is then detected.

Further methods include the detection of primary immune complexes by a two step approach. A second binding ligand, such as an antibody, that has binding affinity for the antibody is used to form secondary immune complexes, as described above. After washing, the secondary immune complexes are contacted with a third binding ligand or antibody that has binding affinity for the second antibody, again under conditions effective and for a period of time sufficient to allow the formation of immune complexes (tertiary immune complexes). The third ligand or antibody is linked to a detectable label, allowing detection of the tertiary immune complexes thus formed. This system may provide for signal amplification if this is desired.

## 1. ELISAs

As detailed above, immunoassays, in their most simple and direct sense, are binding assays. Certain preferred immunoassays are the various types of enzyme linked immunosorbent assays (ELISAs) and radioimmunoassays (RIA) known in the art. Immunohistochemical detection using tissue sections is also particularly useful. However, it will be readily appreciated that detection is not limited to such techniques, and western blotting, dot blotting, FACS analyses, and the like may also be used.

In one exemplary ELISA, the anti-TPO antibodies of the invention are immobilized onto a selected surface exhibiting protein affinity, such as a well in a polystyrene microtiter plate. Then, a test composition suspected of containing the wild-type or mutant thrombopoietin protein antigen. such as a clinical sample, is added to the wells. After binding and washing to remove non-specifically bound immune complexes, the bound wild-type or mutant thrombopoietin protein antigen may be detected. Detection is generally achieved by the addition of another anti-TPO antibody that is linked to a detectable label. This type of ELISA is a simple "sandwich ELISA". Detection may also be achieved by the addition of a second anti-TPO antibody, followed by the addition of a third antibody that has binding affinity for the second antibody, with the third antibody being linked to a detectable label.

In another exemplary ELISA, the samples suspected of containing the wild-type or mutant thrombopoietin protein antigen are immobilized onto the well surface and then contacted with the anti-TPO antibodies. After binding and washing to remove non-specifically bound immune complexes, the bound anti-TPO antibodies are detected. Where the initial anti-TPO antibodies are linked to a detectable label, the immune complexes may be detected directly. Again, the immune complexes may be detected using a second antibody that has binding affinity for the first anti-TPO antibody, with the second antibody being linked to a detectable label.

Another ELISA in which the wild-type or mutant thrombopoietin proteins, polypeptides or peptides are immobilized, involves the use of antibody competition in the detection. In this ELISA, labeled antibodies against wild-type or mutant thrombopoietin protein composition are added to the wells, allowed to bind, and detected by means of their label. The amount of wild-type or mutant thrombopoietin protein antigen in an unknown sample is then determined by mixing the sample with the labeled antibodies against wild-type or mutant thrombopoietin before or during incubation with coated wells. The presence of wild-type or mutant thrombopoietin protein in the sample acts to reduce the amount of antibody against wild-type or mutant thrombopoietin protein, polypeptide or peptide available for binding to the well and thus reduces the ultimate signal. This is also appropriate for detecting antibodies against wild-type or mutant thrombopoietin protein, polypeptide or peptide in an unknown sample, where the unlabeled antibodies bind to the antigen-coated wells and also reduces the amount of antigen available to bind the labeled antibodies.

Irrespective of the format employed. ELISAs have certain features in common. such as coating, incubating or binding, washing to remove non-specifically bound species, and detecting the bound immune complexes. These are described below.

In coating a plate with either antigen or antibody, one will generally incubate the wells of the plate with a solution of the antigen or antibody, either overnight or for a specified period of hours. The wells of the plate will then be washed to remove incompletely adsorbed material. Any remaining available surfaces of the wells are then "coated" with a nonspecific protein that is antigenically neutral with regard to the test antisera. These include bovine serum albumin (BSA), casein and solutions of milk powder. The coating allows for blocking of nonspecific adsorption sites on the immobilizing surface and thus reduces the background caused by nonspecific binding of antisera onto the surface.

In ELISAs, it is probably more customary to use a secondary or tertiary detection means rather than a direct procedure. Thus, after binding of a protein or antibody to the well, coating with a non-reactive material to reduce background, and washing to remove unbound material, the immobilizing surface is contacted with the biological sample to be tested under conditions effective to allow immune complex (antigen/antibody) formation. Detection of the immune complex then requires a labeled secondary binding ligand or antibody, or a secondary binding ligand or antibody in conjunction with a labeled tertiary antibody or third binding ligand.

"Under conditions effective to allow immune complex (antigen/antibody) formation" means that the conditions preferably include diluting the antigens and antibodies with solutions such as BSA, bovine gamma globulin (BGG) and phosphate buffered saline (PBS)/Tween. These added agents also tend to assist in the reduction of nonspecific background.

The "suitable" conditions also mean that the incubation is at a temperature and for a period of time sufficient to allow effective binding. Incubation steps are typically from about 1 to 2 to 4 h or so, at temperatures preferably on the order of 25°C to 27°C, or may be overnight at about 4°C or so.

Following all incubation steps in an ELISA, the contacted surface is washed so as to remove non-complexed material. A preferred washing procedure includes washing with a solution such as PBS/Tween. or borate buffer. Following the formation of specific immune complexes between the test sample and the originally bound material, and subsequent washing, the occurrence of even minute amounts of immune complexes may be determined.

To provide a detecting means, the second or third antibody will have an associated label to allow detection. Preferably, this will be an enzyme that will generate color development upon incubating with an appropriate chromogenic substrate. Thus, for example, one will desire to contact and incubate the first or second immune complex with a urease, glucose oxidase, alkaline phosphatase or hydrogen peroxidase-conjugated antibody for a period of time and under conditions that favor the development of further immune complex formation *(e.g.,* incubation for 2 h at room temperature in a PBS-containing solution such as PBS-Tween).

After incubation with the labeled antibody, and subsequent to washing to remove unbound material, the amount of label is quantified, *e.g.,* by incubation with a chromogenic substrate such as urea and bromocresol purple or 2,2'-azino-di-(3-ethyl-benzthiazoline-6-sulfonic acid (ABTS) and $H_2O_2$, in the case of peroxidase as the enzyme label. Quantification is then achieved by measuring the degree of color generation, e.g., using a visible spectra spectrophotometer.

### VI. Biological Functional Equivalents

As modifications and changes may be made in the structure of the thrombopoietin genes and proteins, and still obtain molecules having like or otherwise desirable characteristics, such biologically functional equivalents are also encompassed within the present invention.

For example, certain amino acids may be substituted for other amino acids in a protein structure without appreciable loss of interactive binding capacity with structures such as, for example, antigen-binding regions of antibodies, binding sites on substrate molecules or receptors, or such like. Since it is the interactive capacity and nature of a protein that defines that protein's biological functional activity, certain amino acid sequence substitutions can be made in a protein sequence (or, of course, its underlying DNA coding sequence) and nevertheless obtain a protein with like (agonistic) properties. It is thus contemplated by the inventor that various changes may be made in the sequence of thrombopoietin proteins, polypeptides or peptides, or the underlying nucleic acids, without appreciable loss of their biological utility or activity.

Equally, the same considerations may be employed to create a protein, polypeptide or peptide with countervailing, *e.g.*, antagonistic properties. This is relevant to the present invention in which thrombopoietin mutants or analogues may be generated. For example, an thrombopoietin mutant may be generated and tested for protein thrombopoietin activity to identify those residues important for thrombopoietin activity. Thrombopoietin mutants may also be synthesized to reflect an thrombopoietin mutant that is less antigenic but still retains its thrombopoietic and/or cryopreservative activities.

In terms of functional equivalents, it is well understood by the skilled artisan that, inherent in the definition of a "biologically functional equivalent" protein, polypeptide or peptide or gene, is the concept that there is a limit to the number of changes that may be made within a defined portion of the molecule and still result in a molecule with an acceptable level of equivalent biological activity. Biologically functional equivalent peptides are thus defined herein as those peptides in which certain, not most or all, of the amino acids may be substituted.

In particular, where shorter length peptides are concerned, it is contemplated that fewer amino acids changes should be made within the given peptide. Longer domains may have an intermediate number of changes. The full length protein will have the most tolerance for a larger number of changes. Of course, a plurality of distinct proteins/polypeptides/peptides with different substitutions may easily be made and used in accordance with the invention.

It is also well understood that where certain residues are shown to be particularly important to the biological or structural properties of a protein, polypeptide or peptide, *e.g.*, residues in binding regions or active sites, such residues may not generally be exchanged. In this manner, functional equivalents are defined herein as those peptides which maintain a substantial amount of their native biological activity.

Amino acid substitutions are generally based on the relative similarity of the amino acid side-chain substituents. for example, their hydrophobicity, hydrophilicity, charge, size, and the like. An analysis of the size, shape and type of the amino acid side-chain substituents reveals that arginine, lysine and histidine are all positively charged residues; that alanine, glycine and serine are all a similar size; and that phenylalanine, tryptophan and tyrosine all have a generally similar shape. Therefore, based upon these considerations, arginine. lysine and histidine; alanine, glycine and serine; and phenylalanine, tryptophan and tyrosine; are defined herein as biologically functional equivalents.

To effect more quantitative changes, the hydropathic index of amino acids may be considered. Each amino acid has been assigned a hydropathic index on the basis of their hydrophobicity and charge characteristics, these are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The importance of the hydropathic amino acid index in conferring interactive biological function on a protein is generally understood in the art (Kyte and Doolittle, 1982, incorporated herein by reference). It is known that certain amino acids may be substituted for other amino acids having a similar hydropathic index or score and still retain a similar biological activity. In making changes based upon the hydropathic index, the substitution of amino acids whose hydropathic indices are within ±2 is preferred, those which are within ±1 are particularly preferred, and those within ±0.5 are even more particularly preferred.

It is also understood in the art that the substitution of like amino acids can be made effectively on the basis of hydrophilicity, particularly where the biological functional equivalent protein, polypeptide or peptide thereby created is intended for use in immunological embodiments, as in certain embodiments of the present invention. U.S. Patent 4,554,101, states that the greatest local average hydrophilicity of a protein, as governed by the hydrophilicity of its adjacent amino acids, correlates with its immunogenicity and antigenicity. *i.e.* with a biological property of the protein.

\As detailed in U.S. Patent 4,554,101, the following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4).

In making changes based upon similar hydrophilicity values, the substitution of amino acids whose hydrophilicity values are within ±2 is preferred, those which are within ±1 are particularly preferred, and those within ±0.5 are even more particularly preferred.

While discussion has focused on functionally equivalent polypeptides arising from amino acid changes, it will be appreciated that these changes may be effected by alteration of the encoding DNA; taking into consideration also that the genetic code is degenerate and that two or more codons may code for the same amino acid. A table of amino acids and their codons is presented herein above for use in such embodiments, as well as for other uses, such as in the design of probes and primers and the like.

In addition to the thrombopoietin peptidyl compounds described herein, the inventor also contemplate that other sterically similar compounds may be formulated to mimic the key portions of the peptide structure. Such compounds, which may be termed peptidomimetics, may be used in the same manner as the peptides of the invention and hence are also functional equivalents.

Certain mimetics that mimic elements of protein secondary structure are described in Johnson *et al.* (1993). The underlying rationale behind the use of peptide mimetics is that the peptide backbone of proteins exists chiefly to orientate amino acid side chains in such a way as to facilitate molecular interactions, such as those of antibody and antigen. A peptide mimetic is thus designed to permit molecular interactions similar to the natural molecule.

Some successful applications of the peptide mimetic concept have focused on mimetics of β-turns within proteins, which are known to be highly antigenic. Likely β-turn structure within a polypeptide can be predicted by computer-based algorithms, as discussed herein. Once the component amino acids of the turn are determined, mimetics can be constructed to achieve a similar spatial orientation of the essential elements of the amino acid side chains.

The generation of further structural equivalents or mimetics may be achieved by the techniques of modeling and chemical design known to those of skill in the art. The art of receptor modeling is now well known, and by such methods a chemical that binds to thrombopoietin proteins, polypeptides or peptides can be designed and then synthesized. It will be understood that all such sterically designed constructs fall within the scope of the present invention.

In addition to the 20 "standard" amino acids provided through the genetic code, modified or unusual amino acids are also contemplated for use in the present invention. A table of exemplary, but not limiting, modified or unusual amino acids is provided herein below.

TABLE 5

| Modified and Unusual Amino Acids | | | |
|---|---|---|---|
| Abbr. | Amino Acid | Abbr. | Amino Acid |
| Aad | 2-Aminoadipic acid | EtAsn | N-Ethylasparagine |
| bAad | 3- Aminoadipic acid | Hyl | Hydroxylysine |
| bAla | β-alanine, β-Amino-propionic acid | aHyl | allo-Hydroxylysine |
| Abu | 2-Aminobutyric acid | 3Hyp | 3-Hydroxyproline |
| 4Abu | 4- Aminobutyric acid, piperidinic acid | 4Hyp | 4-Hydroxyproline |
| Acp | 6-Aminocaproic acid | Ide | Isodesmosine |
| Ahe | 2-Aminoheptanoic acid | alle | allo-Isoleucine |
| Aib | 2-Aminoisobutyric acid | MeGly | N-Methylglycine. sarcosine |
| bAib | 3-Aminoisobutyric acid | Melle | N-Methylisoleucine |
| Apm | 2-Aminopimelic acid | MeLys | 6-N-Methyllysine |
| Dbu | 2,4-Diaminobutyric acid | MeVal | N-Methylvaline |
| Des | Desmosine | Nva | Norvaline |
| Dpm | 2,2'-Diaminopimelic acid | Nle | Norleucine |
| Dpr | 2,3-Diaminopropionic acid | Orn | Ornithine |
| EtGly | N-Ethylglycine | | |

## VII. Pharmaceutical Compositions

### A. Pharmaceutically Acceptable Carriers

Aqueous compositions comprise an effective amount of the thrombopoietin protein, polypeptide, peptide, or such like, dissolved or dispersed in a pharmaceutically acceptable carrier or aqueous medium. The phrases "pharmaceutically or pharmacologically acceptable" refer to molecular entities and compositions that do not produce an adverse, allergic or other untoward reaction when administered to an animal, or a human. as appropriate.

As used herein, "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions. For human administration, preparations should meet sterility, pyrogenicity, general safety and purity standards as required by FDA Office of Biologics standards.

The biological material should be extensively dialyzed to remove undesired small molecular weight molecules and/or lyophilized for more ready formulation into a desired vehicle, where appropriate. The active compounds will then generally be formulated for parenteral administration, *e.g.,* formulated for injection *via* the intravenous, intramuscular, sub-cutaneous, intralesional, or even intraperitoneal routes. The preparation of an aqueous composition that contains an thrombopoietin agent as an active component or ingredient will be known to those of skill in the art in light of the present disclosure. Typically, such compositions can be prepared as injectables, either as liquid solutions or suspensions; solid forms suitable for using to prepare solutions or suspensions upon the addition of a liquid prior to injection can also be prepared; and the preparations can also be emulsified.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

An thrombopoietin protein, polypeptide and/or peptide can be formulated into a composition in a neutral or salt form. Pharmaceutically acceptable salts. include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. In terms of using peptide therapeutics as active ingredients, the technology of U.S. Patents 4,608,251; 4,601,903; 4,599,231; 4,599,230; 4,596,792; and 4,578,770.

The carrier can also be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. The preparation of more, or highly, concentrated solutions for direct injection is also contemplated, where the use of DMSO as solvent is envisioned to result in extremely rapid penetration, delivering high concentrations of the active agents to a small area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion, (see for example. "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

In addition to the compounds formulated for parenteral administration, such as intravenous or intramuscular injection, other pharmaceutically acceptable forms include, *e.g.,* tablets or other solids for oral administration; liposomal formulations; time release capsules; and any other form currently used, including cremes.

One may also use nasal solutions or sprays, aerosols or inhalants in the present invention. Nasal solutions are usually aqueous solutions designed to be administered to the nasal passages in drops or sprays. Nasal solutions are prepared so that they are similar in many respects to nasal secretions, so that normal ciliary action is maintained. Thus, the aqueous nasal solutions usually are isotonic and slightly buffered to maintain a pH of 5.5 to 6.5. In addition. antimicrobial preservatives, similar to those used in ophthalmic preparations, and appropriate drug stabilizers, if required, may be included in the formulation. Various commercial nasal preparations are known and include, for example, antibiotics and antihistamines and are used for asthma prophylaxis.

Additional formulations which are suitable for other modes of administration include vaginal suppositories and pessaries. A rectal pessary or suppository may also be used. Suppositories are solid dosage forms of various weights and shapes, usually medicated, for insertion into the rectum, vagina or the urethra. After insertion, suppositories soften, melt or dissolve in the cavity fluids. In general, for suppositories, traditional binders and carriers may include, for example, polyalkylene glycols or triglycerides; such suppositories may be formed from mixtures containing the active ingredient in the range of 0.5% to 10%, preferably 1%-2,%.

Oral formulations include such normally employed excipients as, for example, pharmaceutical grades of mannitol. lactose, starch. magnesium stearate, sodium saccharine, cellulose, magnesium carbonate and the like. These compositions take the form of solutions, suspensions, tablets, pills, capsules, sustained release formulations or powders. In certain defined embodiments, oral pharmaceutical compositions will comprise an inert diluent or assimilable edible carrier, or they may be enclosed in hard or soft shell gelatin capsule, or they may be compressed into tablets. or they may be incorporated directly with the food of the diet. For oral therapeutic administration, the active compounds may be incorporated with excipients and used in the form of ingestible tablets, buccal tables, troches, capsules, elixirs, suspensions, syrups, wafers, and the like. Such compositions and preparations should contain at least 0.1% of active compound. The percentage of the compositions and preparations may, of course, be varied and may conveniently be between about 2 to about 75% of the weight of the unit, or preferably between 25-60%. The amount of active compounds in such therapeutically useful compositions is such that a suitable dosage will be obtained.

The tablets, troches, pills, capsules and the like may also contain the following: a binder, as gum tragacanth, acacia, cornstarch, or gelatin; excipients, such as dicalcium phosphate: a disintegrating agent. such as corn starch. potato starch. alginic acid and the like; a lubricant, such as magnesium stearate; and a sweetening agent, such as sucrose. lactose or saccharin may be added or a flavoring agent, such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup of elixir may contain the active compounds sucrose as a sweetening agent methyl and propylparabens as preservatives, a dye and flavoring, such as cherry or orange flavor.

## B. Liposomes and Nanocapsules

In certain embodiments, the use of liposomes and/or nanoparticles is contemplated for the introduction of thrombopoietin protein, polypeptides, peptides or agents. or gene therapy vectors, including both wild-type and antisense vectors, into host cells. The formation and use of liposomes is generally known to those of skill in the art, and is also described below.

Nanocapsules can generally entrap compounds in a stable and reproducible way. To avoid side effects due to intracellular polymeric overloading, such ultrafine particles (sized around 0.1 μm) should be designed using polymers able to be degraded *in vivo.* Biodegradable polyalkyl-cyanoacrylate nanoparticles that meet these requirements are contemplated for use in the present invention, and such particles may be are easily made.

Liposomes are formed from phospholipids that are dispersed in an aqueous medium and spontaneously form multilamellar concentric bilayer vesicles (also termed multilamellar vesicles (MLVs). MLVs generally have diameters of from 25 nm to 4 μm. Sonication of MLVs results in the formation of small unilamellar vesicles (SUVs) with diameters in the range of 200 to 500 Å, containing an aqueous solution in the core.

The following information may also be utilized in generating liposomal formulations. Phospholipids can form a variety of structures other than liposomes when dispersed in water, depending on the molar ratio of lipid to water. At low ratios the liposome is the preferred structure. The physical characteristics of liposomes depend on pH, ionic strength and the presence of divalent cations. Liposomes can show low permeability to ionic and polar substances, but at elevated temperatures undergo a phase transition which markedly alters their permeability. The phase transition involves a change from a closely packed, ordered structure, known as the gel state. to a loosely packed. less-ordered structure. known as the fluid state. This occurs at a characteristic phase-transition temperature and results in an increase in permeability to ions, sugars and drugs.

Liposomes interact with cells *via* four different mechanisms: Endocytosis by phagocytic cells of the reticuloendothelial system such as macrophages and neutrophils; adsorption to the cell surface, either by nonspecific weak hydrophobic or electrostatic forces, or by specific interactions with cell-surface components; fusion with the plasma cell membrane by insertion of the lipid bilayer of the liposome into the plasma membrane, with simultaneous release of liposomal contents into the cytoplasm; and by transfer of liposomal lipids to cellular or subcellular membranes, or *vice versa,* without any association of the liposome contents. Varying the liposome formulation can alter which mechanism is operative. although more than one may operate at the same time.

**C. Kits**

Therapeutic kits of the present invention are kits comprising an thrombopoietin protein, polypeptide, peptide and/or other thrombopoietin effector. The kit may further comprise instructions to their use in the methods of the present invention, and/or as a platelet cryopreservative agent. Such kits will generally contain, in suitable container means, a pharmaceutically acceptable formulation of an thrombopoietin protein, polypeptide and/or peptide in a pharmaceutically acceptable formulation. The kit may have a single container means, or it may have distinct container means for each compound.

When the components of the kit are provided in one or more liquid solutions, the liquid solution is an aqueous solution, with a sterile aqueous solution being particularly preferred. The thrombopoietin compositions may also be formulated into a syringeable composition. In which case, the container means may itself be a syringe, pipette, or other such like apparatus, from which the formulation may be applied to an infected area of the body, injected into an animal, or even applied to and mixed with the other components of the kit.

However, the components of the kit may be provided as dried powder(s). When reagents or components are provided as a dry powder, the powder can be reconstituted by the addition of a suitable solvent. It is envisioned that the solvent may also be provided in another container means.

The container means will generally include at least one vial, test tube, flask, bottle, syringe or other container means, into which the thrombopoietin protein, polypeptide, peptide and/or other thrombopoietin effector formulation are placed, preferably, suitably allocated. The kits may also comprise a second container means for containing a sterile, pharmaceutically acceptable buffer or other diluent.

The kits of the present invention will also typically include a means for containing the vials in close confinement for commercial sale, such as, e.g., injection or blow-molded plastic containers into which the desired vials are retained.

Irrespective of the number or type of containers, the kits of the invention may also comprise, or be packaged with, an instrument for assisting with the injection/administration or placement of the ultimate thrombopoietin protein, polypeptide, peptide and/or other thrombopoietin effector composition within the body of an animal. Such an instrument may be a syringe, pipette, forceps, or any such medically approved delivery vehicle.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result.

**EXAMPLE 1**

**STIMULATION OF MEGAKARYOCYTE AND PLATELET PRODUCTION BY A SINGLE DOSE OF RECOMBINANT HUMAN THROMBOPOIETIN IN PATIENTS WITH CANCER**

To assess hematopoietic response to and clinical tolerance of recombinant human thrombopoietin, a recently cloned novel cytokine, the inventor initiated a phase I and II clinical and laboratory studies of recombinant human thrombopoietin in patients with cancer who were at high risk for severe chemotherapy-induced thrombocytopenia. This trial was divided into two parts: part I studied thrombopoietin given before chemotherapy, and part II studied thrombopoietin given after chemotherapy. The objective of part I, the results of which are reported here, was to assess the hematopoietic effects, pharmacodynamics, and clinical tolerance of this novel agent in patients who had normal hematopoietic function before chemotherapy.

**Methods**

*Patients*

Patients with sarcoma who had never had chemotherapy, were suitable candidates for subsequent chemotherapy, and did not have rapidly progressive disease were eligible for this trial. Patients were required to have a Karnofsky performance status score of 80 or more, adequate bone marrow (absolute neutrophil count $\geq 1.5 \times 10^9$/L; platelet count $\geq 150 \times 10^9$/L and $\geq 450 \times 10^9$/L), adequate renal function (serum creatinine level $\geq 120$ µmol/L), and adequate hepatic function (alanine aminotransferase level < 3 times normal; bilirubin level < 1.5 times normal). Patients with a history of thromboembolic or bleeding disorders, significant cardiac disease, or previous pelvic radiation were excluded. Written informed consent was obtained from all patients before study entry in accordance with institutional guidelines.

*Design*

During the phase I dose-ranging portion of this clinical cohort study, thrombopoietin was administered as a single intravenous dose 3 wk before chemotherapy. At study entry, three patients were assigned to each of four dose levels (0.3, 0.6, 1.2, and 2.4 μ*glkg* of body weight). Patients who had no dose-limiting toxicity and did not develop neutralizing antibodies to thrombopoietin were eligible to receive thrombopoietin at the same doses after chemotherapy.

*Recombinant Human Thrombopoietin*

The thrombopoietin used in this study was provided by Genentech. Inc. (South San Francisco, California). Thrombopoietin is a full-length glycosylated molecule produced in a genetically modified mammalian cell line and purified by standard techniques. It was mixed with preservative-free normal saline as a diluent for injections.

*Clinical and Laboratory Monitoring*

Before and during the clinical trial, patients were monitored by complete histories; physical examinations; and laboratory tests, including a complete blood cell count with differential counts, serum chemistry, coagulation profile, urinalysis, assessment of thrombopoietin antibody formation, chest radiography, and electrocardiography. Blood counts were obtained daily for the first 5 days and then at least three times per week. Peripheral smears were examined serially for platelet morphology. Platelet counts and the average size of platelets (mean platelet volume) were derived from 64 channel platelet histograms.

Bone marrow aspiration and biopsy were done before and 1 wk after thrombopoietin treatment. The bone marrow specimens were initially fixed in 10% neutral formalin, embedded in paraffin, cut into sections 5 μm thick, and stained with hematoxylin-eosin for morphologic analysis and with Masson trichrome for analysis of collagen fiber content. Fresh, air-dried smears of bone marrow were stained with Wright-Giemsa. Bone marrow samples were examined for overall cellularity and morphology in a blinded manner. Megakaryocyte counts were measured by choosing 10 high-power (40×) fields in areas without artifactual zones or trabecula. The relative size of the megakaryocyte was assessed by examining bone marrow aspirate smears using the Magiscan Image Analysis System (Compix, Cranberry, Pennsylvania). Bone marrow aspirates were also assayed for hematopoietic progenitor cell number and cycle status, for content of CD34$^+$ and CD41$^+$ cell subsets (by, flow cytometry), and for megakaryocyte ploidy (by flow cytometry). Blood samples were assayed for hematopoietic progenitor cell number and for platelet function.

*Pharmacokinetics Profiles*

Serum samples were collected before and at 2, 5, 10, 60, and 90 min and 2, 4, 6, 8,10, 12, 24, 48, 72, 96, and 120 hr after thrombopoietin administration. Concentration-time profile at each dose bevel was evaluated by using standard pharmacokinetics methods. Serum thrombopoietin levels were quantitated by enzyme-linked immunosorbent assay for thrombopoietin (Emmons *et al.,* 1996).

*Hematopoietic Progenitor Cell Assays*

Assays for colony-forming unit-granulocyte-macrophage (CFU-GM); burst-forming unit-erythroid (BFU-E); and colony-forming unit-granulocyte, erythroid, macrophage, megakaryocyte (CFU-GEMM) using low-density bone marrow (Vadhan-Raj *et al.,* 1995) and peripheral blood cells (Murray *et al.,* 1996) were done with methyl cellulose assays. The percentage of bone marrow CFU-GM and BFU-E in DNA synthesis (S-phase of cell cycle) was measured by a high-specific-activity tritiated thymidine suicide technique (Broxmeyer *et al.,* 1989). Assays for colony-forming unit-megakaryocyte (CFU-MK) and burst-forming unit-megakaryocyte (BFU-MK) were done using a fibrin clot assay (Bruno *et al.,* 1988).

*Ploidy Analysis*

Megakaryocyte-enriched cell fractions were prepared from bone marrow cell suspensions by using a Percoll gradient technique. Ploidy was determined by flow cytometric measurement of the relative DNA content after staining with propidium iodide in hypotonic citrate solution (Krishan, 1975). Cells were also stained with anti-CD41b (8D9)-FTTC (SyStemix. Palo Alto. California) to allow gating on CD41b$^+$ megakaryocytes. At least 3000 CD41$^+$ events were collected for each sample. The percentage of CD41$^+$ cells in ploidy class was determined from the fluorescence-activated cell-sorting dot plots.

*Platelet Function*

Platelet aggregation was measured in response to three agonists: adenosine diphosphate (final concentration. 20 µg/mL). collagen (6 µg/mL). and thrombin (5 µg/mL). Standard methods were used (Rodak, 1995). The concentrations of agonists were chosen on the basis of previous *in vitro* studies done on blood from normal controls. The instruments used for the assays were the Bio/Data Pap 4A (Horsham, Pennsylvania) and the Crono-log 560CA (Havertown, Pennsylvania).

*Immunophenotypic Analysis*

Immunophenotypic analysis was done using anti-CD34 (Becton Dickinson, San Jose, California) and anti-CD41 monoclonal antibodies (Immunotech. Westbrook, Maine) by a standard dual-color flow cytometry technique (Korbling *et al., 1995).*

*Statistical Analysis*

Continuous variables were compared by using the Wilcoxon matched-pairs signed-rank test. Trends for possible dose-response relation were evaluated using the Spearman rank correlation coefficients (r Sub S) between dose and outcome.

*Industry Role*

Thrombopoietin and partial funding for the study were provided by Genentech, Inc. The study was a collaborative effort between the inventor and the industrial sponsor. Data collection, data analysis, the writing of the manuscript, and the decision to publish the manuscript were under the control of the inventor. The manuscript was reviewed by the industrial sponsor before submission.

**Results**

Twelve chemotherapy-naive patients (7 men and 5 women) with sarcoma of diverse histologic sub-types were entered into the dose-ranging portion of this phase I trial. which studied thrombopoietin before chemotherapy. All patients were considered evaluable for clinical tolerance, and response to thrombopoietin. The median age of these patients was 42 yr (range, 16 to 63 yr), and the median Karnofsky performance status score was 90 (range, 80 to 100). Four patients had previously received radiation therapy, and eight had previously had surgery.

*Peripheral Blood Counts*

Treatment with a single dose of thrombopoietin was associated with increases (1.3-fold to 3.6-fold) in platelet counts (baseline mean, $264 \times 10^9$/L; maximal mean, $592 \times 10^9$/L) ($P$ = 0.002). The increase in platelet count was seen at all dose levels in all patients. Data was taken as the mean $\pm$ SE for all patients at each dose. The peak response in platelet count was dose-related; increases from baseline of 61%, 107%, 118%, and 212% were seen at the 0.3, 0.6, 1.2, and 2.4 µg/kg dose levels, respectively (r Sub S = 0.720, $P$ = 0.01). At the highest dose level tested (2.4 µg/kg), the platelet count increased to approximately 1 million or more in two of three patients treated ($970 \times 10^9$/L in one patient and $1392 \times 10^9$/L in the other).

An increase in platelet count was seen early; 10 of 12 patients showed increases from baseline by day 4 (mean increase. 1.2-fold: $P$ = 0.005). and all patients showed increases from base-line by day 8 (mean increase, 1.8-fold; $P$ = 0.002). Platelet counts peaked on median day 12 (range, 10 to 15 days) (mean increase, 2.2-fold; $P$ = 0.002). After maximum response, the platelet counts gradually declined toward baseline. By day 21 (before the initiation of chemotherapy), the platelet counts were still higher than baseline, especially at the highest dose level (at which they were increased 1.5-fold).

Overall platelet morphology appeared normal after treatment. At the time of peak platelet counts, the mean platelet volume was slightly lower than baseline (7.6 fL compared with 8.5 fL); by day 21, it was close to baseline (8.2 fL).

*Platelet Functions*

To determine whether platelets derived after thrombopoietin treatment were capable of mediating functions critical for homeostasis. studies of platelet aggregation in response to three agonists were done on platelets obtained from patients both before and after thrombopoietin treatment (day 7) and from normal controls (Table 6). No statistically significant changes in platelet aggregation were seen in relation to baseline or control samples after thrombopoietin treatment in response to adenosine diphosphate. collagen, or thrombin ($P$ > 0.2).

| Table 6 - Platelet Aggregation Studies | | | | | | | | |
|---|---|---|---|---|---|---|---|---|
| Variable | Adenosine Diphosphate | | | Collagen | | | Thrombin | | |
| | Controls | Patients | | Controls | Patients | | Controls | Patients | |
| | | Before Treatment | 7 Days after Treatment* | | Before Treatment | 7 Days after Treatment* | | Before Treatment | 7 Days after Treatment* |
| Patients, $n$ | 24 | 12 | 12 | 24 | 11 | 11 | 24 | 11 | 11 |
| Mean aggregation $\pm$ SE % | $78 \pm 1$ | $76 \pm 2$ | $80 \pm 2$ | $80 \pm 1$ | $79 \pm 2$ | $81 \pm 2$ | $90 \pm 1$ | $89 \pm 3$ | $93 \pm 2$ |

EP 1 146 895 B1

*Other Hemaiopoietic Cell Lineages*

Although a slight upward trend was seen in leukocyte counts after thrombopoietin treatment, the counts remained within normal limits; these changes were not clinically significant. Similarly, hemoglobin values did not change at any dose level. A minor overall decrease in hemoglobin level was seen with time (baseline mean, 13.4 ± 0.4; mean at day 21, 12.8 ± 0.4) that was probably related to the phlebotomies that were done frequently during the study.

*Pharmacokinetics*

Before treatment, endogenous serum thrombopoietin was detectable (0.096 to 0.24 ng/mL) in 4 of 12 patients. After thrombopoietin administration, the initial maximum thrombopoietin concentrations were proportional to the dose and ranged from 5 to 50 ng/mL. Data was taken as the mean ± SE for three patients per dose level. Thrombopoietin concentrations declined more slowly after the higher doses.
For example, at 6 hr, the mean serum thrombopoietin levels had declined by 50% after the 2.4-µg/kg dose and by 80% after the 0.3-µg/kg dose. The terminal serum half-life for the higher doses (1.2 to 2.4 µg/kg) ranged from 18 to 32 hr. Overall, thrombopoietin remained in the circulation for 5 to 6 days after intravenous bolus administration.

*Clinical Tolerance*

Treatment with thrombopoietin was well tolerated by all patients, and no significant adverse events were attributed to thrombopoietin. Six patients reported occasional mild headaches in their symptom-record diaries, but these headaches were not closely associated with the timing of thrombopoietin administration. No local skin reactions, bone pain, constitutional symptoms, weight gain, edema, or changes in chemistry related to the study drug were seen during this period.

*Antibody Analysis*

To determine whether thrombopoietin therapy was associated with the development of antibodies that might interfere with the hematopoietic elect of thrombopoietin, serum samples from all patients were screened weekly. Serum samples obtained on days 13 and 22 after dosing from 1 of the 12 patients tested positive for antibodies reactive to full-length thrombopoietin. These antibody titers were transient, were non-neutralizing, and were not associated with clinical sequelae.

*Effects on Bone Marrow*

To better understand the cellular basis for the hematopoietic response to thrombopoietin, the inventor examined the bone marrow both before and 7 days after thrombopoietin treatment for expansion of progenitor cells, lineage specificity, and maturation.

*Bone Marrow Morphology*

The increase in platelet count was associated with a significant increase in the number of bone marrow megakaryocytes ($P = 0.003$) in a dose-related manner; it ranged from a 1.2-fold increase at the lowest dose level to a 4-fold increase at the highest dose level (r Sub S = 0.867, $P < 0.001$). Although most megakaryocytes showed normal morphology, several large megakaryocytes with abundant cytoplasm and multilobulated nuclei were identified. Overall, the size of the megakaryocytes increased significantly from baseline (from a mean diameter of 36 µm [range, 17 to 64 µm] to a mean of 44 µm [range, 20 to 87 µm]; $P < 0.001$).
The granulocytic and erythroid series showed normal maturation without significant changes in the myeloid, erythroid cell ratio or bone marrow cellularity (Table 7). No significant increase in bone marrow collagen was identified after thrombopoietin treatment in any of the specimens examined.

TABLE 7 -

| Effects of Thrombopoietin Treatment on Bone Marrow | | | |
|---|---|---|---|
| Hematopoietic Variable | Baseline | 7 Days after Treatment | *P* Value |
| CD34+ cells, % | 3.2 ± 0.5 | 6.3 ± 1.0 | 0.012 |
| CD41+ cells, % | 6.2 ± 1.0 | 12.2 ± 1.8 | 0.002 |

TABLE 7 -   (continued)

| Effects of Thrombopoietin Treatment on Bone Marrow | | | |
|---|---|---|---|
| Hematopoietic Variable | Baseline | 7 Days after Treatment | *P* Value |
| Cellularity, % | 28 ± 3 | 30 ± 3 | >0.2 |
| Myeloid:erythroid ratio | 3.2 ± 0.5 | 2.5 ± 0.3 | >0.2 |

*Megakaryocyte Ploidy Analysis*

To determine whether thrombopoietin treatment affected megakaryocyte maturation, bone marrow megakaryocyte ploidy was assessed at all dose levels. Although the modal ploidy peak remained at 16N, increased percentages of megakaryocytes were seen at lower ploidy classes (*P* < 0.001 for 4N and 8N) at all dose levels. The data was taken as the mean ± SE for patients. Patients that had a higher ploidy distribution at the start of treatement were not included. In addition, a trend toward increased higher ploidy levels was seen at the higher doses (*P* = 0.06 for 64N and *P* = 0.06 for 128N). Thus, thrombopoietin treatment enhanced both maturation of megakaryocytes and expansion of more immature megakaryocytes.

*Bone Marrow Progenitors and CD34$^+$ Cells*

To determine whether thrombopoietin had a lineage-specific effect at the progenitor cell level, bone marrow progenitor cell frequencies were assayed for megakaryocyte (CFU-MK and BFU-MK), myeloid (CFU-GM), erythroid and multipotential (CFU-GEMM) cells. Frequency of bone marrow CFU-MK (2.7-fold to 33-fold) was increased in 5 of 11 patients examined. A significant increase was also seen in the number of mature subsets of myeloid (1.8-fold; *P* = 0.005) and erythroid (1.7-fold; *P* = 0.016) progenitors, and similar trends were seen for the immature subsets of myeloid and erythroid progenitors and multipotential progenitors. The percentage of bone marrow cells in S-phase was also significantly increased (*P* = 0.013) from baseline. Data was taken as the mean ± SE for all patients tested. In addition, the percentage of bone marrow CD34$^+$ (*P* = 0.012) and CD41$^+$ (*P* = 0.002) cells increased an average of 2-fold in number.

*Effects on Mobilization of Progenitors*

To determine whether the increase in bone marrow progenitors was associated with a mobilization effect, the peripheral blood was assayed for progenitor cells. A significant increase was seen in the number of progenitors of myeloid (CFU-GM), erythroid (BFU-E), and myelo-erythroid (CFU-MIX) cells. Data was taken as the peak response (mean ± SE) which was seen between days 3 and 7. Although individual patient responses varied, a dose-dependent trend was seen. At the highest dose level, a mean maximum increase of progenitor cell frequencies was seen as high as 5.7-fold for CFU-GM, 7.8-fold for BFU-E, and 10-fold for CFU-MIX.

**Discussion**

The inventor's objective was to evaluate the in vivo biological effects of thrombopoietin, including clinical tolerance and pharmacodynamics, and to define the nature of the hematopoietic response to this cytokine in patients with cancer who had normal hematopoietic function before receiving chemotherapy. The inventor shows that treatment with a single dose of thrombopoietin produced a marked increase in circulating platelet counts in a dose-related manner. Platelet response (increases of 1.3-fold to 3.6-fold) was seen in all patients, including the cohort that received the lowest dose of thrombopoietin. The platelets produced in response to thrombopoietin had normal morphologic appearance and displayed normal aggregation function in response to various stimuli. Thus, the studies indicate that thrombopoietin is a potent stimulus for the production of platelets with normal function in humans.

The inventor observed several interesting facets of the hematopoietic response to thrombopoietin in these patients. The increase in platelet count was seen by day 4 in most patients, suggesting that the initial component of the response was related to enhanced maturation of megakaryocytes, platelet release, or both. Platelet counts continued to increase, however, reaching a peak between days 10 and 15. This suggests that a second component of the response might be related to an expansion of the megakaryocyte compartment. After the maximum response, platelet counts declined toward baseline. However, platelet counts at day 21 before the initiation of chemotherapy were still higher than baseline counts, especially at the highest dose level; this study indicates a prolonged biological effect.

This sustained response after a single dose of thrombopoietin can be explained by several possible mechanisms. First, the inventor observed that thrombopoietin has a prolonged serum half-life of about 20 to 30 hr and remains in the circulation for about 5 to 6 days. Furthermore, although peak serum concentration was proportional to dose, the terminal half-life was prolonged at higher doses, perhaps because of the saturation of the thrombopoietin clearance process (thought to occur primarily in platelets by receptor-mediated endocytosis) (Fielder *et al.,* 1996).

A second explanation might be related to the proliferative effect of thrombopoietin at the bone marrow level. Some patients had an increase in the number of megakaryocytic progenitors (CFU-MK). The proliferative effect of thrombopoietin on bone marrow was also marked by a two-fold increase in the percentage of CD34[+] cells and a concomitant two-fold increase in CD41[+] subsets, reflecting a selective maturation along the megakaryocytic pathway. In fact, the number of megakaryocytes increased in proportion to the dose, and the megakaryocytes displayed normal morphology. Moreover, ploidy analysis indicated not only the maturation effect of thrombopoietin but also the expansion of immature subsets of megakaryocytes, which may be related to the proliferative effect. Third, because the life span of platelets is 9 to 10 days, the response in number of platelets would be expected to last for several days.

The proliferative effect of thrombopoietin on bone marrow was not limited to the megakaryocytic lineage. A significant increase was seen in the frequency of progenitors of myeloid, erythroid, and multipotential cell lineages. Furthermore, the proportion of progenitors in S-phase also increased after treatment with thrombopoietin. Despite this multilineage stimulators effect of thrombopoietin on bone marrow progenitors, no major effect was seen on peripheral leukocyte counts or hematocrit values. Thus, the proliferative effect at the progenitor level appeared to be coupled with differentiation primarily along the megakaryocytic pathway.

Despite the expansion of the progenitor pool in bone marrow, the overall cellularity remained unchanged, possibly because of the extramedullary release of progenitors into the peripheral blood. One of the biological properties of hematopoietic growth factors that has demonstrable clinical effect is the ability to mobilize hematopoietic progenitors that can be harvested and reinfused after dose-intensive or myeloablative therapy (Socinski *et al.,* 1988; Shea *et al.*, 1992: Bensinger *et al.,* 1993). In the study, a single dose of thrombopoietin strongly mobilized the progenitors of multiple lineages. This apparent increase in the circulating pool of progenitor cells was further supported by a several-fold increase in very primitive hematopoietic cells (CD34[+]/Thy-1[+]/Lin) in the peripheral blood (Murray *et al.,* 1996).

Treatment with thrombopoietin was well tolerated by all patients; no significant adverse events related to thrombopoietin or thrombocytosis were seen. Unlike other cytokines, thrombopoietin was not associated with fever, constitutional symptoms, bone pain, inflammatory symptoms, fluid retention, or any organ toxicity (Smith *et al.,* 1993; Vadhan-Raj *et al.,* 1994: Biesma *et al.,* 1992; Veldhuis *et al.,* 1995: Vadhan-Raj *et al.,* 1994: Tepler *et al.,* 1996). Thus, the lack of significant toxicity and the lineage-dominant biological effect of thrombopoietin may provide a better therapeutic ratio than do other cytokines with thrombopoietin potential.

One potential concern associated with inducing thrombocytosis is an increased risk for thrombosis, which is frequently seen in patients with myeloproliferative disorders. However, the lack of morphologic and functional platelet abnormalities and the transient nature of induced thrombocytosis may limit risk in this clinical setting.

In summary, the studies indicate that thrombopoietin is a potent stimulator of megakaryocytopoiesis and thrombopoiesis in humans. Thrombopoietin administered as a single dose produced prolonged increases in circulating platelet counts. Although it had a lineage-dominant effect on peripheral blood counts, it mediated a multilineage effect at the bone marrow progenitor cell level and mobilized progenitors of multiple lineages into the peripheral blood. These observations have several potential ramifications. For example, in a manner analogous to that of the clinical application of myeloid growth factors. thrombopoietin has the potential to attenuate chemotherapy-induced thrombocytopenia. The studies reported here show that the peak platelet response occurs between day 10 and day 15. Thus, treatment with thrombopoietin before chemotherapy may provide a holdover period until platelet production is resumed after chemotherapy and possibly further enhanced by thrombopoietin given after chemotherapy.

A second intriguing possibility is that the potent biological effect of a single dose will allow thrombopoietin to be given to patients or normal donors not only to mobilize progenitor cells for peripheral stem cell transplantation but also to collect platelets that can be transfused as an apheresis product, thus potentially reducing the risk for alloimmunization associated with exposure to multiple donors. It should be noted, however, that the trial involved a small, defined group of patients with sarcoma who had unperturbed normal hematopoiesis.

**EXAMPLE 2**

**SINGLE-DOES THERAPY WITH RECOMBINANT HUMAN THROMBOPOIETIN (rhTPO) IN PATIENTS RECEIVING CYTOTOXIC CHEMOTHERAPY**

**Background**

Preclinical models of intensive chemoradiotherapy demonstrated that a single dose of rhTPO raises the platelet nadir and shortens the period of severe thrombocytopenia. Interim results of two Phase I studies in which single doses of rhTPO were administered to cancer patients receiving chemotherapy are presented.

**Patients and Methods**

Both studies began with 21-day, pre-chemotherapy periods (cycle 0) for assessment of rhTPO safety and platelet response after single IV bolus injections of 0.3, 0.6, or 1.2 mcg/kg (3 patients per group in each study). Patients then received the same dose of rhTPO after chemotherapy in selected subsequent cycles. The first study population consisted of patients with advanced malignancies who received rhTPO the day following salvage thiotepa chemotherapy (65 mg/m2 q28d) in each of two consecutive chemotherapy cycles. The second study included chemotherapy naive patients with sarcoma undergoing induction treatment with AI chemotherapy (doxorubicin 90 mg/m2, ifosfamide 10 g/m2 q21d). Following cycle 0, patients in this study were monitored during the first chemotherapy cycle and received a single rhTPO injection the day following completion of chemotherapy (d5) during the second and subsequent cycles.

**Results**

14 patients have been treated to date. rhTPO was well tolerated with no reported serious adverse events attributed to study drug. Antibodies to rhTPO have not been observed. In cycle 0 the lowest (0.3 mcg/kg) dose was weakly active, with increased activity at higher doses as shown below.

Table 8

| rhTPO does (mcg/kg) | Patients N | Mean Baseline Platelet (/$\mu$l) (SD) | Median Maximum Platelet (/$\mu$l) (Range) | Median % Increase |
|---|---|---|---|---|
| 0.3 | 7 | 339 (133) | 510 (277-628) | 40 |
| 0.6 | 5 | 235 (69) | 486 (386-509) | 103 |
| 1.2 | 2 | 203 (46) | 523 (437, 608) | 158 |

The maximum platelet count during cycle 0 occurred on median day 11 (range 7-14). No significant changes were noted in WBC or HCT. FACS analysis of bone marrow showed increases in all cD34+ subsets in 2/2 patients following 0.6 mcg/kg. Increases in peripheral blood CD34+ cells were also seen in these patients, suggesting that TPO might have stem cell mobilizing activity. Dose escalation and post-chemotherapy treatment are ongoing.

**Conclusion**

Together these phase I studies suggest that single dose administration of rhTPO is safe and well tolerated. The 0.3, 0.6 and 1.2 mcg/kg dose levels show increasing thrombopoietic activity. Treatment of patients at higher dose levels will test the hypothesis that a single dose of rhTPO is efficacious in ameliorating thrombocytopenia following intensive chemotherapy.

**EXAMPLE 3**

**PHASE I-II INVESTIGATION OF RECOMBINANT HUMAN THROMBOPOIETIN (rhTPO) IN PATIENTS WITH SARCOMA RECEIVING HIGH DOSE CHEMOTHERAPY (CT) WITH ADRIAMYCIN (A) AND IFOSFAMIDE (I)**

AI is a highly active regimen in the treatment of sarcoma; however, the myelosuppression is cumulative and multilineage. In the preclinical model of severe myelosuppression, rhTPO (Genentech, Inc.) given as a single dose raised the pit nadir and reduced the period of severe thrombocytopenia. Based on these observations, the inventor initiated a phase I-II investigation of rhTPO alone and following AI to assess tolerance and investigate biologic effects. rhTPO was given by I.V. bolus as a single dose (0.3, 0.6, 1.2, 2.4 $\mu$g/kg) on Arm A or as two doses (0.3, 0.6, 1.2 $\mu$g/kg $\times$ 2) on Arm B, prior to AI (Cycle 0) and following a second cycle of AI (Cycle 2). Twenty-three patients have received rhTPO (16-Arm A, 7-Arm B). rhTPO prior to AI significantly increased pit counts (up to 3.6-and 4.4-fold on Arms A (n=12) and B (n=7), respectively). The rise in pits was sustained and peaked on a median day 12. This sustained response was associated with a prolonged serum half-life (20-30 h) after a single rhTPO dose. The plts had normal morphology and aggregation function. The response in pits was accompanied by up to 4-fold rise in BM megakaryocytes and a 2-fold rise in CD34+, CD41+ cell subsets, as well as significant increases in frequency and cycling rate of BM progenitors. In addition, mobilization of progenitor cells of multiple cell lineages was observed. Post-chemotherapy data (mean $\pm$ SEM) are shown below on 19 patients that received rhTPO:

Table 9

| rhTPO Schedule | Platelet Nadir ($\times 10^3$/µL) | | Days Platelets < 100 $\times$ 10$^3$/µL | | Days Platelets <50 $\times$ 10$^3$/µL | |
|---|---|---|---|---|---|---|
| | Cycle 1 | Cycle 2 | Cycle 1 | Cycle 2 | Cycle 1 | Cycle 2 |
| Single Dose: Arm A, N=14 (0.3-3.6 µg/kg) | 47 ± 9 | 36 ± 6 | 5.4 ± 0.8 | 5.2 ± 0.8 | 2.6 ± 0.7 | 2.7 ± 0.9 |
| Two Doses: Arm B, N=4 (0.6-1.2 µg/kg) | 81 ± 8 | 78 ± 27 | 3.8 ± 1.7 | 2.5 ± 1.0 | 0 | 0.8 ± 0.5 |

These data indicate that rhTPO is a potent stimulus for prolonged platelet production and exhibits multilineage stimulatory effect at the progenitor cell level.

**EXAMPLE 4**

**EFFECTS OF RECOMBINANT HUMAN THROMBOPOIETIN (rhTPO) ON BONE MARROW MEGAKARYOCYTES IN HUMANS**

To evaluate the effects of rhTPO (Genentech, Inc.) on bone marrow megakaryocyte (MK) morphology *in vivo*, the inventor examined bone marrow specimens from 12 patients who received a single dose of rhTPO (0.3-2.4 mcg/ kg) in chemotherapy-naive patients with sarcoma as part of a phase I-II investigation (Vadhan-Raj, 1996). BM specimen obtained both before and after (day 7) rhTPO were stained [H and E (biopsy); Wright-Giemsa (aspirate)] and examined for treatment-associated changes in size and morphology. The size of the megakaryocytes was assessed by examining the bone marrow aspirate smears by using the Magiscan Image Analysis System (Compic, PA). Twenty-five Mks with clearly defined margins and intact nucleus and cytoplasmic membranes were evaluated for cellular and nuclear areas. The number of megakaryocytes increased in a dose related manner ($p<0.01$). While most megakaryocytes appeared normal in abundant cytoplasm and multilobated nuclei were seen. The overall megakaryocytes increased significantly over the baseline values (from mean diameter 36 µm to 44 µm, $p<0.001$). The data (mean ± SEM) on cellular and nuclear areas and N/C ratios are shown below.

Table 10

| | Cellular area (µm) | Nuclear area (µm) | N/C ratio |
|---|---|---|---|
| Pre-rhTPO | 997 ± 87 | 402 ± 32 | 0.41 ± 0.01 |
| Post-rhTPO | 1517 ± 63* | 537 ± 22* | 0.36 ± 0.01* |

*p<0.01

These results demonstrate that rhTPO given as a single dose increase both the number and size of megakaryocytes. While both the nuclear and cellular areas increased in size, the increase appeared more pronounced in the cytoplasmic areas.

**EXAMPLE 5**

**PHARMACOKINETICS OF RECOMBINANT HUMAN THROMBOPOIETIN (rhTPO) AFTER INTRAVENOUS ADMINISTRATION IN CANCER PATIENTS**

rhTPO pharmacokinetics and platelet response were studied in 12 chemotherapy-naive patients (age 16-63 years) with sarcoma as part of a Phase I/II does-escalation clinical study (Vadhan-Raj, 1996)

**Methods**

rhTPO was administered as a single IV bolus dose 3 wk prior to chemotherapy. Three patients were treated in each dose level (0.3, 0.6, 1.2 or 2.4 µg/kg). Serum pharmacokinetic samples were collected over 5 days following dosing. TPO concentrations were measured by an ELISA that employs the recombinant c-mpl receptor for capture of TPO. Pharmacokinetic analysis was performed using non-compartmental methods. Platelet counts were routinely measured for 21 days.

### Results

Prior to rhTPO dosing, endogenous serum TPO was detected in 4/12 patients. After rhTPO administration, serum TPO was detectable for five days in all dose groups. The mean initial maximum serum TPO levels (about 5 and 50 ng/mL for the 0.3 and 2.4 $\mu$g/kg doses, respectively) reflected initial distribution into a volume of 60 mL/kg, which is slightly larger than serum volume. Within each dose group, initial maximum TPO levels tended to be lower in patients with higher baseline platelet counts, probably reflecting the initial binding of TPO to platelets. Serum TPO levels declined more slowly in patients who received the 1.2 and 2.4 $\mu$g/kg doses, compared to those receiving the lower doses. Terminal serum TPO half-life estimated following the 1.2 and 2.4 $\mu$g/kg doses ranged from 18 to 32 h. Consistent with the ability of platelets to clear TPO, the area under the TPO concentration vs. time curve (AUC) within each dose level was lower in patients with higher baseline platelet counts. Serum TPO clearance decreased with increasing rhTPO dose. Maximum changes in platelet counts (61 and 212% above baseline for the 0.3 and 2.4 $\mu$g/kg dose groups, respectively) were proportional to rhTPO dose and to TPO AUC. Increasing platelet counts were observed as early as Day 4, reaching a maximum by median Day 12 (range 10-15 days) after rhTPO dosing. The time to maximum platelet count was not influenced by the rhTPO dose level. Platelets remained elevated in some patients for up to 21 days.

### Summary

TPO remained in the circulation for at least five days following a single IV bolus dose and TPO levels declined with a terminal half-life of approximately 18 to 32 h. Within each dose group, TPO maximum levels and AUC were inversely related to baseline platelet counts. A single IV dose of rhTPO produced a dose proportional increase in platelet count and platelet count remained elevated for several days.

### EXAMPLE 6

### RECOMBINANT HUMAN THROMBOPOIETIN (RHTPO) ATTENUATES HIGH-DOSE CARBOPLATIN (C)-INDUCED THROMBOCYTOPENIA IN PATIENTS WITH GYNECOLOGIC MALIGNANCY

Carboplatin is effective in advanced-stage platin-sensitive recurrent disease; however, cumulative thrombocytopenia is frequently dose-limiting. In the preclinical studies, TPO decreased the nadir and accelerated platelet (PLT) recovery in mice rendered pancytopenic by RT + carboplatin. Based on this observation, the inventor carried out a phase I-II study of rhTPO alone and following carboplatin (AUC=11) to assess the tolerance and biologic effects. During dose-escalation phase, rhTPO was given by S.C. injection as a single dose (0.6, 1.2, 2.4, 3.6 mcg/kg) prior to carboplatin and for four doses following a second cycle of carboplatin. During dose-expansion, six additional patients received rhTPO at optimal biologic dose (OBD) only as a secondary prophylaxis for severe thrombocytopenia. rhTPO prior to carboplatin significantly increased platelet counts (up to 3.5- fold, $p<0.001$), with a peak effect on median day 15. Platelet response was accompanied by increases in bone marrow megakaryocytes and erythroid elements. Post-chemotherapy data (mean $\pm$ SEM) are shown below (Table 11) on 22 patients that received rhTPO at all doses.

Table 11

| Nadir PLT (x$10^3$/mm$^3$) | | Days PLT <50 $\times$ $10^3$/mm$^3$ | | Days PLT <100 $\times$ $10^3$/mm$^3$ | | No. (%) of PTS transfused | |
|---|---|---|---|---|---|---|---|
| C-1 | C-2* | C-1 | C-2* | C-1 | C-2* | C-1 | C-2 |
| (C) | (C+T) | (C) | (C+T) | (C) | (C+T) | (C) | (C+T) |
| 20 $\pm$5 | 52 $\pm$ 8 | 6.3 $\pm$1.0 | 2.9 $\pm$0.9 | 10.3 $\pm$ 1.3 | 6.3 $\pm$ 1.2 | 11 (50) | 5 (23) |

*$p<0.002$

In the 11 patients that received rhTPO at OBD (1.2mcg/kg) in Cycle-2, rhTPO avoided the need for plt transfusion in 71% of patients (7 pts. in cycle-1 vs. 2 pts. in cycle-2). No significant adverse event related to rhTPO was seen. These data indicate that rhTPO is effective in attenuating carboplatin-induced thrombocytopenia and can prevent platelet transfusions when used as a secondary prophylaxis.

**EXAMPLE 7**

**A PILOT STUDY OF ADRIAMYCIN + IFOSFAMIDE WITH RECOMBINANT HUMAN THROMBOPOIETIN (RHTPO) IN PATIENTS WITH SARCOMAS**

This example describes methods of how to determine the optimum dose schedule of administering the combination of adriamycin and ifosfamide (AI) with the rhTPO, and how to obtain preliminary information regarding the efficacy of the rhTPO at the schedules proposed in reducing AI-induced thrombocytopenia.

Adriamycin and ifosfamide are the two most active chemotherapeutic agents currently used in the treatment of sarcoma. Available data suggest a positive dose-response relationship for both of these agents. However. cumulative myelosuppression is frequently dose-limiting. rhTPO is a lineage-dominant regulator of thrombopoiesis and may attenuate cumulative thrombocytopenia. Results of sequential pilot studies and more recent Phase I-II trial suggest that this regimen is active with response rate over 60%. At the doses used in the inventor's trials (90 mg/m$^2$ adriamycin and 10 gm/m$^2$ ifosfamide), other than cumulative myelosuppression, the regimen is reasonably well tolerated. The inventor has, therefore, been investigating the use of hematopoietic growth factors (G-CSF and rhTPO) to attenuate hematopoietic toxicity.

Thrombopoietin (TPO), the ligand for C-Mpl receptors has recently been purified and cloned. TPO regulates all stages in the development of platelet production by promoting the proliferation of megakaryocyte progenitors and their maturation into platelet producing megakaryocytes. In preclinical studies, administration of TPO results in a rapid rise in platelet counts by several fold. In the murine model of myleosuppression, recombinant murine TPO has been shown to be effective in abrogating thrombocytopenia in mice rendered pancytopenic by carboplatin and irradiation. The major safety concerns that were raised as a result of extensive preclinical studies include immunogenicity and resultant thrombocytopenia.

Recombinant human TPO (rhTPO) is currently undergoing testing in several Phase I-II clinical studies in patients receiving both myelosuppressive and myeloablative chemotherapy. In ongoing studies, single and multiple IV doses of rhTPO have been demonstrated to be safe and without evidence of constitutional toxicities (such as fever, rigors, or hypotension). There have been two thrombotic events (deep vein thrombosis) reported that were considered to be possibly related to rhTPO in over 150 subjects treated. Anti-TPO antibodies have been observed in 10% of IV rhTPO-treated subjects; these antibodies were transient and non-neutralizing. To date. over 25 subjects have received rhTPO by subcutaneous (SC) injection, and anti-TPO antibodies have been observed in 10% of these. Potentially neutralizing antibodies were observed in a single subject who had received multiple SC injections of rhTPO over 5 months and had experienced prolonged thrombocytopenia prior to recovery. It is unclear whether the thrombocytopenia was due to the presence of antibodies or to the cumulative effects of myelosuppressive chemotherapy.

**Patient Inclusion Criteria**

To be included in the study, the patients must meet the following criteria. Patients must have sarcoma which is inoperable, locally advanced, at high risk for relapse or metastatic for whom treatment with AI is indicated. The patients' age should be 15-65 (for ages 13 to less than 15, at the discretion of the inventor). Females of childbearing potential must be using effective means of contraception. The patients must have adequate hematologic, renal, ant hepatic functions, a Karnofsky performance status ≥ 80, and a signed informed consent.

**Patient Exclusion Criteria**

Patients should be exclude for the following reasons. The patients would be pregnant or lactating women, patients with a co-morbid condition, which renders patients at high risk of treatment complication, patients with uncontrolled metastatic disease, patients with significant cardiac disease (NYHA Class III or IV), dysarrhythmia, or recent history of MI or ischemia, patients who are HBsAG positive, patients that have had prior chemotherapy or use of hematopoietic growth factor, prior surgery or RT within 2 weeks of study entry, prior history of pelvic radiation, or a history of any platelet disorder (ITP, TTP, *etc.*), bleeding disorder, or any increased risk of bleeding.

TREATMENT PLAN

A group of patients will be treated at each of the 5 schedules.

Table 12

| Schedule I | Cycle 1: AI(days 0-3) | Cycle 2: AI(days 0-3) |
|---|---|---|
| | No rhTPO | rhTPO (days -3, -1, 4, and 6) |
| Schedule II | Cycle 1: AI(days 0-3) | Cycle 2: AI(days 0-3) |
| | No rhTPO | rhTPO (days -5. -3, -1, and 4) |
| | | |
| Schedule III | Cycle 1 AI(days 0-3) | Cycle 2: AI(days 0-3) |
| | No rhTPO | rhTPO (days -1, 4, 6, and 8) |
| | | |
| Schedule IV | Cycle 1: AI(days 0-3) | Cycle 2: AI(days 0-3) |
| | No rhTPO | rhTPO (days 4, 6, 8, 10) |
| | | |
| Schedule V | Cycle 1: AI(days 0-3) | Cycle 2: AI(days 0-3) |
| | No rhTPO | rhTPO (days-7, -5, -3, -1) |

rhTPO will be administered by IV bolus injection. G-CSF at 5 mcg/kg/day to start on day 4, approximately 12 h after the first dose of rhTPO, and to be continued until ANC $\geq$ 1500/mm$^3$ for 2 consecutive days post nadir.

In patients with at least a stable or responding disease and a lack of excessive hematopoietic toxicity (i.e., platelet count <20,000/mm$^3$ for >7 days or thrombocytopenia associated with clinically significant bleeding), chemotherapy with rhTPO treatment can continue up to a total of six cycles.

The chemotherapy cycles will be repeated every 3 wk, if the ANC has recovered to $\geq$1500/mm$^3$ and platelets are $\geq$100,000/mm$^3$.

## STATISTICAL CONSIDERATIONS

The preliminary information regarding the efficacy of rhTPO will be obtained by comparing the nadir counts and the recovery of platelets between cycle-1 (no rhTPO) and cycle-2 (with rhTPO) and between different groups. The data generated will be used to help design future phase II-III clinical trials.

PATENT EVALUATION: (Pre-treatment and interim testing)

The patients' history, PE, performance status, CBC with differential, platelets, serum chemistry, electrolytes, magnesium, serum bank, bone marrow aspiration and biopsy (select patients), chest x-ray, radiologic studies for tumor treatments and EKG will be evaluated.

ESTIMATED ACCRUAL

It is estimated that accrual will be approximately 30 evaluable participants.

STUDY DESIGN

The safety and efficacy of rhTPO has been evaluated in the sarcoma patients receiving AI (adriamycin 90mg/m$^2$ and ifosfamide 10 gm/m$^2$). Of the seventy nine patients entered into the trial, 74 patients received rhTPO. No serious adverse event attributed to rhTPO has been observed in this trial. rhTPO administered prior to chemotherapy either as a single dose or two doses was associated with a dose-dependent increase in circulating platelet counts. Following chemotherapy, five different schedules of rhTPO administration have been examined: one dose after chemotherapy (A), two doses after chemotherapy (B), seven doses after chemotherapy (C), one dose before and two doses after chemotherapy (D) and a dose before, during and after chemotherapy (F). rhTPO at doses 1.2 - 2.4 mcg/kg administered as D schedule (days -1, 4 and 5) appear to reduce thrombocytopenia and accelerate platelet recovery. However, it is not uniformly effective in reducing thrombocytopenia, especially when used with this regimen in compromised patients *(i.e.,* as a secondary prophylaxis, in female patients or patients with age >50 years).

Rationale For the Study Design:

Since the nadir occurs early with the AI regimen *(i.e.,* day 12), and the peak effect of rhTPO is also seen around day 12 (days 10-15), earlier administration of rhTPO might be helpful. The inventor's finding from the clinical trial in patients with gynecologic malignancy indicate that 4 doses of rhTPO (starting from day after chemo every other day or one dose prior to chemo, one dose on the day of chemo and 2 doses after chemo) are effective in reducing carboplatin-induced thrombocytopenia. Therefore, the inventor would like to assess the feasibility of rhTPO given at 1.2 mcg/kg for 4 doses in 3 groups of patients by 3 slightly different schedules: rhTPO on days -3, -1, 4, and 6 (I). -5, -3. -1, and 4 (II), and -1. 4, 6, and 8 (III).

One potential concern of using a growth factor prior to or during chemotherapy may be that bone marrow progenitor cells might be actively proliferating at the time of chemotherapy administration and be sensitized to cytotoxic drugs. The inventor's laboratory findings on progenitor cell proliferative kinetics suggest that there is a significant increase in proliferative rate of progenitor cells on day 8 after rhTPO, but not on day 4. Further, the inventor have evaluated the schedules of rhTPO administration on a day prior to and on a day of chemotherapy administration in both sarcoma and gynecologic malignancy. These data do not suggest overtly detrimental effect of prechemotherapy rhTPO administration. For safety reasons, the inventor would start with one additional dose before chemo *(i.e.,* days -3 and -1). If this schedule appears to be safe and not associated with increased hematologic toxicity than anticipated, the inventor would add one more dose prior to chemotherapy *(i.e.,* days -5, -3, and - I ) in the next group.

**Clinical Update**

To date 21 patients (18 evaluable) were treated on this trial. The treatment with rhTPO is well tolerated. The preliminary analysis of the hematologic parameters indicate that the mean platelet nadir is higher in Cycle-2 than in Cycle-1 by the schedule where 3 doses of rhTPO are given before and one dose after chemotherapy. The inventor has previously shown that with a shorter regimen like carboplatin (AUC=11), rhTPO given as 4 doses following chemotherapy significantly reduces thrombocytopenia. Therefore, the inventor would like to treat two more cohorts, where all 4 doses of rhTPO are given after AI chemotherapy (Schedule IV) and all 4 doses of rhTPO are given before chemotherapy (Schedule V). This would be useful information prior to implementation of Phase III trial where all doses of rhTPO are planned to be given after chemotherapy.

**BACKGROUND DRUG INFORMATION**

All drugs are commercially available except for rhTPO. rhTPO will be provided by Genentech, Inc., 460 Point San Bruno Boulevard, South San Francisco, CA 94080, as a sterile liquid ready for parenteral administration. Each 3-mL glass vial of active compound contains 2 mL. rhTPO will be provided as 0.1 mg/mL in buffered solution. rhTPO will be shipped and must be stored under refrigeration at 2°C-8°C (35°F-46°F). The formulation does not contain a preservative and is suitable for single-dose administration only. Exposure to bright light should be avoided. Do not freeze. Do not use past the expiration date stamped on the vial.

**rhTPO Preparation And Administration**

Throughout the study, clinical pharmacists or other designated study staff will prepare the rhTPO injections based on the subject's weight as measured at baseline. The clinical pharmacist will provide the study staff with syringes of rhTPO labeled with the subject ID number (and any other identifier), the protocol number and the amount of rhTPO (in micrograms and milliliters). Injections of diluted rhTPO must be administered within 4 h of preparation.

**Patient Inclusion Criteria**

To be included, patients should have sarcoma which is inoperable, locally advanced, at high risk for relapse or metastatic for whom treatment with AI is indicated. Patients should be age 15-65 (for ages 13 to less than 15, at the discretion of the inventor). Females of childbearing potential must be using effective means of contraception. Patients should have adequate bone marrow function, and an absolute neutrophil count $\geq$ 1500/$\mu$L, a platelet count $\geq$ 150,000/$\mu$L and a Hgb $\geq$ 10 gm/dL. The patients should have adequate renal function, i.e. serum creatinine $\leq$ 1.5 mg%. The patients should have adequate hepatic function, i.e. SGPT <3 $\times$ normal and Bilirubin < 1.5 $\times$ normal. The patients Karnofsky performance status should be $\geq$ 80. And the patients should have a signed informed consent form.

**Patient Exclusion Criteria**

Patient would be excluded if they are pregnant or lactating women. Patients would be excluded if they have a comorbid condition which renders patients at high risk of treatment complication. Patients would be excluded if they have uncontrolled metastatic disease to CNS. Patients with significant cardiac disease (NYHA Class III or IV), dysarrhythmia, or recent history of MI or ischemia would be excluded. Patients who are HBsAG positive would be excluded. Patients would be excluded if they have had prior chemotherapy or use of hematopoietic growth factor, prior surgery or RT within 2 weeks of study entry, prior history of pelvic irradiation, or a history of any platelet disorder (ITP, TTP, *etc.),* bleeding disorder, or any increased risk of bleeding.

**Treatment Plan**

All patients will be registered in the patient data management system (PDMS).

**Study Design**

A group of 6 patients will be treated at each of the 3 schedules.

Table 13

| Schedule I | Cycle 1: AI(days 0-3) | Cycle 2: AI(days 0-3) |
|---|---|---|
|  | No rhTPO | rhTPO (days -3, -1, 4, and 6) |
|  |  |  |
| Schedule II | Cycle 1: AI(days 0-3) | Cycle 2: AI(days 0-3) |
|  | No rhTPO | rhTPO (days -5, -3, -1, and 4) |
|  |  |  |
| Schedule III | Cycle 1 AI(days 0-3) | Cycle 2: AI(days 0-3) |
|  | No rhTPO | rhTPO (days -1,4, 6, and 8) |
|  |  |  |
| Schedule IV | Cycle 1: AI(days 0-3) | Cycle 2: AI(days 0-3) |
|  | No rhTPO | rhTPO (days 4, 6, 8, 10) |
|  |  |  |
| Schedule V | Cycle 1: AI(days 0-3) | Cycle 2: AI(days 0-3) |
|  | No rhTPO | rhTPO (days-7, -5, -3, -1) |

rhTPO will be administered by IV bolus injection.

**Table 14 - STUDY SCHEMA**

**Cycle - 2:**

| | -7 -6 | -5 -4 | -3 -2 | -1 0 | 1 2 | 3 | 4 5 | 6 7 | 8 9 | 10 |
|---|---|---|---|---|---|---|---|---|---|---|
| I | | | T↓ | T↓ | Chemo | | T↓ | T↓ | | |
| II | | T↓ | T↓ | T↓ | Chemo | | T↓ | | | |
| III | | | | T↓ | Chemo | | T↓ | T↓ | T↓ | |
| IV | | | | | Chemo | | T↓↓ | T↓↓ | T↓↓ | T↓↓ |
| V | T↓ | T↓ | T↓ | T↓ | Chemo | | | | | |
| Day | -7 -6 | -5 -4 | -3 -2 | -1 0 | 1 2 | 3 | 4 5 | 6 7 | 8 9 | 10 |

*Chemotherapy*

Adriamycin will be administered at a dose of 90 mg/m$^2$ as a 72 h continuous IV injection, days 0-2. Ifosfamide will be administered over 3 h each day at 2.5 g/m$^2$/d $\times$ 4 days (total dose 10 g/m$^2$) on days 0-3. Mesna (500 mg/m$^2$ or 20% of the dose of ifosfamide) will be administered IV over 3 h with the initial dose of ifosfamide on day 0 and maintained as continuous infusion (1500 mg/m$^2$/day for a total of 6 gm/m$^2$) until 24 h after the final dose of ifosfamide.

G-CSF at 5 mcg/kg/day to start on day 4, approximately 12 h after the first dose of rhTPO, and to be continued until ANC $\geq$ 1500/mm$^3$ for 2 consecutive days post nadir. In addition, all patients will receive ciprofloxacin 500mg P.O. BII) starting from day 5 until recovery of ANC as an anti-microbial prophylaxis.

In patients with at least a stable or responding disease and a kick of excessive hematopoietic toxicity *(i.e.,* platelet count <20,000/mm$^3$ for >7 days or thrombocytopenia associated with clinically significant bleeding), chemotherapy with rhTPO treatment can continue up to a total of six cycles. The chemotherapy cycles will be repeated every 3 wk, if the ANC has recovered to $\geq$ 1,500/mm$^3$ and platelets are $\geq$ 100,000/mm$^3$. Patients will be transfused with platelets when platelet counts drop to <15,000/mm$^3$ or at a higher level if there is any clinically significant bleeding. Red cell transfusions will be used for the Hb <8 gms/dL or at a higher level when clinically indicated.

*Dose Modifications*

Every attempt will be made to deliver six cycles of full-dose chemotherapy to every subject in the study. In the case of the specific adverse events listed in Table 15, dose reductions or alterations of infusion duration of doxorubicin and/or ifosfamide may be carried out as stated, using the following parameters;

Table 15

| Adverse Event | Dose Modification |
|---|---|
| Febrile neutropenia with significant morbidity or sepsis syndrome | Reduce ifosfamide to 8 g/m$^2$ |
| Mucositis Grade $\geq$3 | Decrease time of doxorubicin infusion to 48 h (same total dose) |

Table 15   (continued)

| Adverse Event | Dose Modification |
|---|---|
| Recurrent mucositis Grade ≥3 | Decrease doxorubicin to 75 mg/m$^2$ over 48 h |
| Grade 3 CNS toxicity | Reduce ifosfamide to 8 g/m$^2$. Add albumin and methylene blue as per standard practice. |
| Recurrent Grade 3 or 4 CNS toxicity | Remove from study |
| Renal toxicity: serum creatinine before next course >2.0 mg/dl | Reduce ifosfamide to 8 g/m$^2$ |
| Recurrent renal toxicity as defined above | Remove from study |
| Grade 3 hematuria | Administer an additional 500-mg IV bolus of mesna; increase the dose of continuous IV infusion mesna to equal the dose of ifosfamide |

If further dose reductions/modifications are necessary, the subject will be taken off study. All subjects will be followed for at least 28 days after the last day of the last cycle with rhTPO.

**Pretreatment Evaluation**

Patients will be evaluated for standard history and physical examination with tumor measurements. Laboratory studies will include a CBC with differential, platelet count, electrolytes, magnesium and chemistry. Chest X-ray, electrocardiogram, radiologic studies for tumor measurement, and antibodies to TPO will be measured.

**Evaluation During Study**

Subjects will be closely monitored for safety, physical findings, and tumor response while on study. Subjects will be assessed for adverse events at each visit during the study and follow-up period. Vital signs will be measured at each visit, as well as before and after each rhTPO administration. In addition, laboratory evaluation of CBC, differential and platelet counts will be monitored prior to chemotherapy and at least three times a wk following chemotherapy. In addition, daily counts will be done when ANC <500/μL and platelet count <50,000/μL (approximately days 9 to 13). Chemistry, electrolytes and magnesium will be repeated prior to each course and as frequently as deemed necessary. Antibodies to TPO prior to each cycle will be measured. BM aspirate prior to (baseline) and following rhTPO administration in cycle-2 in select patients will be done.

**Criterial for Response and toxicity**

Standard sarcoma criteria will be employed.

**Criteria for Disconting Therapy**

Therapy will be discontinued if a progressive disease alters an initial response or failure of the patient to respond after a minimum of two courses of therapy. Therapy will be discontinued if unacceptable toxicity, defined as unpredictable, irreversible, or grade 4 non-hematologic toxicity, is found in a patient. Therapy will be discontinued due to non-compliance by patient with protocol requirements or patient refusal.

**Other Parameters**

Approximately 30 evaluable patients will be treated. If rhTPO at any of the proposed schedules attenuates severe thrombocytopenia or eliminates transfusion requirement uniformly, a formal Phase II-III protocol will be generated. Data will be collected and entered on PDMS by the research nurse assigned to the study. Deaths clearly related to treatment or unexpected life-threatening toxicities should be reported immediately to the Study Chairman, who, in turn, must notify the Surveillance Committee.

**EXAMPLE 8**

**SCHEDULE-DEPENDENT REDUCTION IN THROMBOCYTOPENIA BY RECOMBINANT HUMAN THROMBOPOIETIN (rhTPO) IN PATIENTS WITH SARCOMA RECEIVING HIGH DOSE CHEMOTHERAPY (CT) WITH ADRIAMYCIN (A) AND IFOSFAMIDE (I)**

Adriamycin and ifosfamide (AI) is an active regimen in the treatment of sarcoma; however, the myelosuppression is cumulative. The inventor has previously shown that rhTPO is effective in reducing severe thrombocytopenia when administered for 4 doses following high dose carboplatin (Blood 90(10):580a, 1997), but is not uniformly effective when given for 1. 2. or 7 doses following AI (Vadhan-Raj *et al..* 1996). The findings that the nadir of AI is early (~day 12), and the peak effects of rhTPO on platelets and progenitors in S-phase are late, indicated that earlier administration of rhTPO might be beneficial. The inventor, therefore, initiated a clinical trial to optimize the schedule of rhTPO. The first cycle of AI was administered without rhTPO. In Cyde-2 (C-2), rhTPO was given by I.V. bolus both prior to (pre-dosing) and following (post-dosing) AI (days 0-3) in 3 schedules: days--1. 4, 6, 8 (S-I), days -3, -1, 4, 6 (S-II), and days -5, -3, -1, 4 (S-III). Post chemotherapy data (mean $\pm$ SEM) are shown:

| Table 16 | | | | |
|---|---|---|---|---|
| rhTPO Schedule | # of Pre/Post Dose | Platelet Nadir (x10³/µL) | | Mean Difference in Platelet Nadir* (Cycle-2 - Cycle-1) |
| | | Cycle-1 | Cycle-2 | |
| S-I (n=6) | 1/3 | 49 (±7) | 34(±11) | -15 (±8) |
| S-II (n=6) | 2/2 | 31 (±9) | 23 (±12) | -7 (±5) |
| S-III (n=6) | 3/1 | 82 (±23) | 113 (±27) | 31 (±24) |

\* P = 0.042 by linear regression

In only 1 of 6 patients from S-III, the platelet nadir was lower in Cycle-2 than in Cycle-1 as compared to 5 of 6 patients on both SI and S-II. BM progenitor cell assays following CT and the last dose of rhTPO showed the presence of colonies in 3 of 4 (S-III), 1 of 2 (S-II) and 0 of 2 (S-I) patients. These studies indicate that timing of rhTPO administration in relation to chemotherapy is important, and pre-dosing with rhTPO may be beneficial with regimens that are longer ($\geq$4 days) and cause earlier nadir.

**EXAMPLE 9**

**A PHASE 1 TRIAL OF RECOMBINANT HUMAN THROMBOPOIETIN (rhTPO) GIVEN VIA SUBCUTANEOUS INJECTION TO SUBJECTS WITH RECURRENT OR ADVANCED GYNECOLOGIC MALIGNANCY RECEIVING CARBOPLATIN**

This example describes the results of preclinical animal studies with thrombopoietin, and the design rational for studies in humans that was done.

Results from preclinical safety studies indicate that after single and multiple doses. recombinant human thrombopoietin (rhTPO) produced expected increases in the number of megakaryocytes and/or circulating platelets in mice, rhesus monkeys, and chimpanzees. In the 4-wk studies in mice, mild to moderate changes in clinical and anatomical pathology were evident; in many instances, these correlated with the observed increase in platelet counts. After repeated subcutaneous (SC) administration in rhesus monkeys and chimpanzees, mild hematologic effects, aside from the effect on platelets, were observed but were not considered to represent biologically significant effects.

In rhesus monkeys, the platelet response to rhTPO peaked after 2 wk of dosing, but then declined toward baseline despite continued dosing. Also in rhesus monkeys, beginning 5-8 wk after the initiation of treatment, a reduction in platelet counts was observed after single IV/SC doses of 5000 µg/kg or multiple IV/SC doses of 1000 µg/kg. Consistent with this observation. megakaryocytic hypoplasia was found in the bone marrow of some animals 8 wk after single-dose IV or SC treatment at the 5000 µg/kg dose level. Similar histopathologic findings, but of greater severity, were observed in rhesus monkeys receiving multiple treatments with IV or SC rhTPO (1000 µg/kg).

Postdose reductions in platelet counts were also noted in chimpanzees dosed with rhTPO (3 or 50 µg/kg SC) daily for 2 wk, although the effect was somewhat later in onset and transient at the 3 µg/kg dose level. A significant increase in platelet count was also observed after redosing with 3 µg/kg in 1 chimpanzee after an 8-wk treatment-free period, indicating that this animal's response to the pharmacologic action of rhTPO was not dramatically reduced as a result of previous exposure.

The cause of the reduction in platelet counts appeared to be related to positive antibody titers to TPO, which were noted in all animals with low platelet values. The rhesus monkeys with the lowest postdose platelet values and megakaryocytic hypoplasia in the single-dose studies had the highest individual titer values. It has previously been reported that recombinant human granulocyte colony-stimulating factor (G-CSF) can cause anti-CSF antibody formation in dogs which results in the neutralization of endogenous canine G-CSF, leading to neutropenia (Hammond *et al.,* 1991). A similar mechanism might account for the effects observed in the rhesus monkey and chimpanzee studies. TPO-specific IgE was present in chimpanzees after multiple dosing.

These findings in chimpanzees suggest a possible risk of antibody formation and/or postdose platelet count decline in humans based on the predictive value of this species in assessing immunologic responses to recombinant human proteins and the homology between human and chimpanzee TPO (Hobson and Fuller, 1987).

Immunogenicity and efficacy were further evaluated in studies using recombinant murine thrombopoietin (rmTPO) in CD-1 mice. In normal CD-1 mice, significantly fewer mice developed antibodies to TPO in the single SC dose group, as compared with the 14-day dosing group. Similar levels of anti-TPO antibodies were observed in the groups receiving daily SC administration for 14 days or every 3 days. However, in the group that received rmTPO every 3 days by IV administration, only 2 mice developed anti-TPO antibodies at Day 31. These results indicate that reducing the frequency of rmTPO administration, or using IV administration, may reduce the immunogenicity of recombinant TPO. In a separate efficacy study, a single dose of rmTPO attenuated the thrombocytopenia induced by the combination of irradiation and carboplatin in the mouse model. These results suggest that there will be a therapeutic window for efficacy in the absence of immunogenicity.

In summary, rhTPO appeared to be slightly more immunogenic when administered SC than when given IV in animals. It is not known whether the SC route will be more immunogenic in humans. Preclinical data are limited regarding the cause, duration, and/or extent of reversibility of the platelet effect in animals. It is clear that a reduction in platelet counts below baseline is a potential important risk in patients for whom chemotherapy treatment is planned. Therefore, close monitoring with a specific safety plan regarding thrombocytopenia will be performed in this trial.

**Preclinical Pharmacokinetics**

The IV and SC pharmacokinetics of rhTPO have been studied in several animal species. Pharmacokinetic analysis of plasma TPO profiles from IV studies in rhesus monkeys indicated limited distribution of TPO from the plasma into other tissues (steady-state distribution volume of 62-66 mL/kg). The low plasma TPO clearance (3.3-5.4 mL/hr/kg) is reflected in a relatively long terminal plasma rhTPO half-life (11-18 h). Data from tissue distribution studies in mice indicated that platelets may be involved in the distribution and clearance of TPO. After SC injection in rhesus monkeys, ~43% of the dose was absorbed and plasma TPO levels peaked at ~6 h. The terminal half-life of rhTPO after SC injection was 13 h. No pharmacokinetic drug interaction was observed when SC rhTPO and SC G-CSF were co-administered daily for 14 days to rhesus monkeys.

**Conical Experience: Clinical Trials**

rhTPO has undergone testing in several open-label. Phase I studies. The first trial was initiated in October 1995. Intravenously administered rhTPO is being evaluated in subjects receiving moderate-to high-dose chemotherapy with thiotepa; in subjects receiving dose-intensive conventional chemotherapy with doxorubicin and ifosfamide for soft tissue sarcomas; in subjects undergoing peripheral progenitor cell mobilization prior to high-dose chemotherapy for high-risk breast cancer: and in subjects experiencing delayed platelet recovery following autologous or allogeneic transplantation.

rhTPO has been well tolerated through dosages of 0.3-2.4 µg/kg. Although antibody formation occurred, no neutralizing anti-TPO antibodies have been observed in the clinical trials. Anti-TPO antibodies have been observed in only 2/33 subjects; these were non-neutralizing in nature, resolved spontaneously, and were not associated with any clinical sequelae. A date, rhTPO administration by the IV route has demonstrated biological activity at the upper dose levels, but has not been associated with any effects on hematopoietic lineages other than early mobilization of peripheral CFU-GMs and BFU-Es. There have been no signs of thrombotic events or constitutional adverse effects and no evidence of dose-limiting toxicity (DLT).

**Clinical Pharmacokinetics**

Serum TPO concentrations have been measured after IV bolos injection of rhTPO to subjects with sarcoma or malignant neoplasm during a pre-chemotherapy cycle and again during a chemotherapy cycle (rhTPO was administered on the day after thiotepa or doxorubicin/ifosfamide). During the pre-chemotherapy cycle, the initial maximum TPO concentrations ($C_{max}$) are proportional to dose, ranging from ~5 to 50 ng/mL after the 0.3 and 2.4 µg/kg doses, respectively. This indicates that rhTPO appears to distribute into an initial distribution volume (60 mL/kg) similar to plasma volume. The apparent terminal elimination half-life for TPO is ~20-30 h. The area under the TPO concentration-time profile (AUC) increases disproportionally with increasing dose, probably reflecting a dose-related saturation of the TPO clearance process, which is thought to occur in platelets primarily by receptor-mediated endocytosis. After chemotherapy, the initial distribution of rhTPO is similar to that observed during the pre-chemotherapy cycle, but the apparent terminal half-life is prolonged during the chemotherapy cycle. This slower terminal decline in serum TPO may reflect an increase in endogenous serum TPO, an increase that has been observed during thrombocytopenia.

**Carboplatin**

Carboplatin has a broad spectrum of anti-tumor activity and is used in the treatment of ovarian cancer, cervical cancer, germ cell tumors, mesothelioma, small cell lung cancer, bladder cancer, and head and neck cancer.

Carboplatin is a suitable chemotherapy agent for this study because of the lack of non-hematologic toxicities at doses up to 1600 mg/m$^2$. It's major toxicity is myelosuppression, especially thrombocytopenia. Ozols *et al.* (Ozols *et al.,* 1987) treated 30 patients with recurrent ovarian cancer with high doses of carboplatin (800 mg/m$^2$) without cytokine support. Major responses were seen in 27% of patients; myelosuppression was the most common toxicity observed. Gore *et al.* (Gore *et al.,* 1987) delivered doses of up to 1600 mg/m$^2$ to lung cancer and mesothelioma patients. Relatively minor gastrointestinal, neurologic, and ototoxicities were observed, however, fairly significant myelosuppression was seen. Given at high doses, carboplatin is a suitable chemotherapy agent to utilize in evaluating the effect of rhTPO on dose-limiting thrombocytopenia.

**Primary Objectives**

The primary objectives of this study were to determine the safety and tolerability, to characterize the pharmacokinetic profile, and to determine the immunogenicity of rhTPO given via SC injection to subjects with recurrent gynecologic malignancy receiving carboplatin.

**Secondary Objectives**

The secondary objectives of this study were to evaluate the pharmacodynamics, as measured by the effect on the depth and duration of the post-chemotherapy platelet nadir, to determine the maximum tolerated dose (MTD) or, in the case of no MTD, to determine the optimum biologic dose (OBD) of rhTPO given *via* SC injection to subjects with recurrent gynecologic malignancy receiving carboplatin.

**Study Design: Overview**

This is an open-label, dose-escalation study in which patients with recurrent gynecologic malignancy will receive carboplatin for treatment of their disease. Up to 30 subjects will be enrolled in this Phase I study of SC administration of rhTPO. Three subjects will be assigned to each of six dose levels, beginning with the lowest dose level. Once the OBD is established, 12 additional subjects will be treated. Six subjects will receive carboplatin chemotherapy together with rhTPO either at one dose below the MTD or at the OBD. The other 6 subjects will receive carboplatin without rhTPO, but may receive rhTPO in subsequent cycles after any cycle in which the subject experiences thrombocytopenia (platelets <30,000/µL).

The study is divided into a 14-day screening period, three 21-day cycles, and a 28-day follow-up period. The first cycle (Cycle 0) will have rhTPO administered at day 1, and day 4 for subjects enrolled in dose levels 4, 5, and 6 only. The second cycle (Cycle 1) will have carboplatin chemotherapy administered only at day 1. The third cycle (Cycle 2) will have carboplatin administered at day 1, rhTPO administered at day 2, and rhTPO administered at day 5 only for subjects enrolled in dose levels 4, 5, and 6. A 28 day follow up period will follow cycle 2.

Dose-level escalation will proceed through all six dose levels unless the MTD or the OBD is determined. The MTD is defined as the dose level preceding the dose level at which any of the following is observed: > 1/3 or ≥ 2/6 subjects within one dose level develop a Grade ≥ 3 non-hematologic toxicity that is related to rhTPO and is not relieved by supportive measures; ≥ 1 subject develops a Grade 4 non-hematologic toxicity that is related to rhTPO and is not relieved by supportive measures: ≥ 2/3 or ≥ 4/6 subjects within one dose level develop a Grade ≥ 3 non-hematologic toxicity (regardless of relief), or any subject develops neutralizing anti-TPO antibodies.

The OBD is defined as the dose level at which comparison between dose levels demonstrates that the trend in platelet response appears to plateau during Cycle 2, as compared with the response in the preceding dose level.

**Description and Rationale**

This is a 63-day study followed by a 28-day follow-up period. Eligible subjects who appear to benefit from carboplatin chemotherapy will be offered an optional extension period (OEP) between the study period and the follow-up period, which will include additional cycles of chemotherapy with rhTPO. After the initial rhTPO administration in Cycle 0, serum samples for anti-TPO antibody levels will be obtained weekly.

All subjects will continue to be evaluated for adverse events and safety for at least 28 days following the last cycle during which they received rhTPO. Subjects with neutralizing anti-TPO antibody formation with associated thrombocytopenia will be followed until resolution of the thrombocytopenia.

All of the clinical studies of rhTPO to date have involved IV dosing. This route is not always practical, and in patients receiving chemotherapy, venous access may not always be easily established. The current study will evaluate safety, tolerance, pharmacokinetics pharmacodynamics. and potential immunogenicity of rhTPO given by the SC route.

**Study Outline**

**a. Screening Period**

The study will be preceded by a 1 4-day screening period. During this screening period, potential subjects will be evaluated for eligibility and informed consent will be obtained.

**b. Study Period (Cycles 0 to 2)**

Cycle 0 will consist of the first 21 days following the initial rhTPO administration. Subjects in Dose Levels 1, 2, and 3 will receive rhTPO as a single SC injection on Day 1. Subjects in Dose Levels 4, 5, and 6 will receive rhTPO as SC injections on Days 1 and 4. During Cycle 0, subjects will not receive chemotherapy; this will allow for the observation of pharmacokinetics and possible anti-TPO antibody formation in the absence of chemotherapy. If 2/3 subjects in Cycle 0 have a platelet count response of ≥ 1,000,000/µL Cycle 0 may be eliminated in subsequent subjects, after discussion between the Medical Monitor and the Inventor.

The remainder of the study will consist of two 21-day chemotherapy cycles. In Cycle 1, subjects will receive carboplatin chemotherapy without rhTPO to establish each subject's baseline platelet response to the chemotherapy. In Cycle 2, all subjects will receive carboplatin chemotherapy followed by rhTPO. Subjects in Dose Levels 1, 2, and 3 will receive rhTPO as a single SC injection on Day 2. Subjects in Dose Levels 4, 5, and 6 will receive rhTPO as SC injections on Days 2 and 5. Cycles I and 2 must begin within 21-35 days following the start of Cycles 0 and 1, respectively.

**c. Optional Extension Period (Cycles 3 6)**

After completion of the study period (Cycles 0-2), subjects with stable or responding disease who have not experienced severe thrombocytopenia *(i.e.,* platelet count of < 20,000/µL for > 7 days) and who have not developed neutralizing anti-TPO antibodies will be eligible to continue receiving 21-day cycles of carboplatin chemotherapy and rhTPO, according to the schedule in Cycle 2. for a maximum of six cycles in total. All OEP cycles must begin within 21-35 days following the start of the previous cycle.

**d. Follow-Up Period**

Subjects will be followed for an additional 28 days following their last cycle with rhTPO, during which they will continue to be evaluated for safety, continuing and new adverse events (including thrombocytopenia), and possible anti-TPO antibody formation. This is the follow-up period.

**Study Endpoints**

The following variables are the primary study endpoints: the safety and tolerability of rhTPO given SC, as measured by the incidence and severity of adverse events; serial serum levels of TPO measured per study flowsheet; the incidence of the formation of antibodies to TPO. The following variable is the secondary study endpoint: serial platelet counts and depth and duration of the platelet nadir following chemotherapy administration

**Safety Evaluations and Follow-Up**

All subjects will be closely followed and observed for adverse events during the course of the study. Subjects will be assessed for adverse events at each visit. All untoward medical occurrences and clinically significant changes from baseline (including laboratory results and vital signs) will be considered adverse events.
The inventor or his designee will evaluate all adverse events for the following criteria: severity; NCI Common Toxicity Grade; relationship to disease; and relationship to rhTPO.
The above information as well as onset, resolution, effect on rhTPO administration, and related treatment for the adverse event will be recorded on the Case Report Form (CRF). All adverse events will have a statement of resolution at the end of the follow-up period. Any anti-TPO antibody-related thrombocytopenia will be followed until resolution.
All adverse events regardless of causality will be recorded in the CRF. Adverse events will be reported at the maximum intensity experienced. If a reported adverse event increases in severity or frequency, it will be considered a new event. If a reported adverse event decreases in severity or frequency, it will be considered continuing until it has completely resolved.

**Safety Plan**

The major safety issues pertaining to rhTPO administration in this trial are as follows: formation of neutralizing anti-TPO antibodies, allergic reactions, including anaphylaxis, and potential thrombocytosis with increased risk of thrombotic events.

**a. Antibody Formation**

All subjects in this trial will undergo weekly anti-TPO antibody testing. Three serum samples for anti-TPO antibody levels will be obtained to help establish a firm baseline for anti-TPO antibody formation. These samples (two as baseline evaluations and one predose on Day 1 of Cycle 0) must be obtained at least I day apart from each other. If anti-TPO antibody formation is detected, additional testing will be done to determine whether the anti-TPO antibodies are neutralizing in nature. If the anti-TPO antibodies are neutralizing, that subject will not receive additional injections of rhTPO. All subjects will have frequent platelet counts measured during the study. Subjects with thrombocytopenia will be supported with platelet transfusions whether or not neutralizing anti-TPO antibodies are present. If the anti-TPO antibodies are non-neutralizing, that subject may continue on study and receive additional rhTPO according to the protocol.

**b. Allergic Reactions**

Formation of anti-TPO antibodies, including IgE, in the animal studies suggests the risk of possible allergic reactions. There have not been any anaphylactic reactions reported in any of the animal studies or the ongoing human trials, but experience with redosing is limited. All subjects enrolled in this trial will receive rhTPO in a closely monitored hospital setting with anaphylaxis precautions in place.

**c. Thrombocytosis**

Administration of rhTPO is expected to result in an increased platelet count. A dramatically elevated platelet count (thrombocytosis) may result in an increased risk of thrombosis. Subjects with a known history of thrombotic events will be excluded from this trial. Additional rhTPO administration may be discontinued in any subject with a platelet count of $\geq$ 1,000,000/uL, after discussion between the Medical Monitor and the inventor. If a subject experiences thrombocytosis. the Medical Monitor and the inventor will discuss treatment options, which may include platelet apheresis and/or aspirin therapy.

**d. Dose-Limiting Toxicity**

DLT is defined as any of the following: neutralizing anti-TPO antibody formation; anaphylaxis; clinically significant thromboembolic event; any Grade $\geq$ 3 non-hematologic toxicity (using the NCI Common Toxicity Criteria) that is determined to be related to rhTPO and is not relieved by supportive measures; or a Grade $\geq$ 3 NCI non-hematologic toxicity that is determined to be related to rhTPO and is relieved by supportive measures is not necessarily dose limiting unless it presents a potential health hazard or is an unacceptable toxicity.

### e. Dose-Level Escalation

In the absence of DLT, escalation will proceed through all dose levels. After Dose Level 1 has been enrolled, at least 2/3 subjects in each dose level must safely tolerate rhTPO administration before the next dose level may be assigned. Safe toleration is defined as the absence of DLT within the 21 days following the initial rhTPO administration (Day 1, Cycle 0).

The first three dose levels will be single-dose regimens. Multiple-dose regimens (Dose Levels 4 6) will not be enrolled until all single-administration regimens have been safely tolerated. If any of the single dose levels are omitted as the result of DLT, one or more of the multiple dose levels may be assigned. The cumulative dose of the multiple-dose regimens may not exceed the MTD in the single-dose regimen prior to discussion and agreement.

### Dosage and Duration

All dose levels of rhTPO in this trial are expected to be active. Subjects will receive a single SC injection of rhTPO in each cycle during which rhTPO administration is scheduled. The platelet count response is expected to occur within 7-10 days. Subjects will be allowed a maximum of six cycles of rhTPO.

### Compliance with Laws and Regulations

This study will be conducted in accordance with current U.S. Food and Drug Administration (FDA) Good Clinical Practices (GCPs),the Declaration of Helsinki, and local ethical and legal requirements.

### Materials and Methods

### Subjects: Subject Selection

Potential subjects in this study are patients who have been diagnosed with any recurrent gynecologic malignancy and in whom carboplatin is indicated. Subjects may have received up to three prior therapies. In addition to these three regimens, subjects may have received prior radiotherapy provided that < 25% of the bone marrow-producing areas have been so treated. Measurable disease is strongly encouraged but is not required for study eligibility. Subjects must be able to wait at least 21 days for the initiation of chemotherapy and must have a life expectancy of $\geq$ 12 weeks.

### Inclusion Criteria

All potential subjects must fulfill the following criteria to be eligible for this trial: be able to give signed informed consent; have histologic or cytologic confirmed gynecologic malignancy; be 18-65 years of age; the subjects of childbearing potential must have a negative serum pregnancy test within the 7 days preceding Cycle 0, Day 1, and all subjects of childbearing potential must, in the opinion of the investigator, be using an effective means of contraception; Karnofsky performance status of $\geq$ 80%; adequate bone marrow function, i.e. WBC count of $\geq$ 3000/$\mu$L and platelet count of $\geq$ 150,000/$\mu$L and $\leq$ 450,000/$\mu$L, adequate hepatic function, *i.e.* total bilirubin of $\leq$ 1.5 mg% and SGOT or SGPT of $\leq$ 3 times normal; adequate renal function, i.e.serum creatinine of $\leq$ 1.5 mg/dL or a calculated creatinine clearance of $\geq$ 60 mL/min; and a life expectancy of $\geq$ 12 wk.

### Exclusion Criteria

Potential subjects will be excluded from this trial if they meet any of the following criteria: a history or diagnosis of leukemia; a rapidly progressive disease; an uncontrolled, clinically significant dysrhythmia: are pregnant or lactating females; have a history or any platelet disorder, including idiopathic thrombocytopenia purpura, thrombotic thrombocytopenia purpura, bleeding diathesis, or any increased risk of bleeding; have a history of significant thrombotic events (including pulmonary embolism and deep vein thrombophlebitis); have a history of myocardial infarction, ischemia, transient ischemic attack, or cerebrovascular accident within the 6 months preceding Cycle 0, Day 1; have a history of central nervous system (CNS) metastases; have had more than three prior regimens of chemotherapy; have used any nitrosourea (BCNU, CCNU) chemotherapy or mitomycin-C within the 6 wk preceding Cycle 0, Day 1; have used granulocyte/macrophage colony-stimulating factor (GM-CSF) or G-CSF within the 2 wk preceding Cycle 0, Day 1; have used erythropoietin (Epogen®, Procrit®) within the 4 wk preceding Cycle 0, Day 1; have used any experimental drug within the 4 wk preceding Cycle 0, Day 1; have used any cytotoxic chemotherapy or immunotherapy within the 4 wk preceding Cycle 0, Day 1; have used any radiation therapy within the 2 wk preceding Cycle 0, Day 1, or any prior pelvic radiation; have used any surgical procedure, with the exception of line placement, within the 2 wk preceding Cycle 0, Day 1; have any other significant medical history or physical finding giving reasonable suspicion of a disease or condition that would contraindicate taking an investigational drug or that might affect the interpretation of the results of this study.

**Method of Treatment Assignment**

Three subjects will be assigned to each of the six dose levels identified in Table 17. Dose level assignment will be in ascending order of dose levels, beginning with Dose Level 1. At least 2/3 subjects must safely tolerate rhTPO administration before the subsequent dose level may be assigned. A total of 18 subjects will be enrolled in the dose-escalation phase. Once the OBD is established, 12 additional subjects will be treated. Six subjects will receive carboplatin chemotherapy together with rhTPO either at one dose below the MTD or at the OBD. The other 6 subjects will receive carboplatin without rhTPO, but may receive rhTPO in subsequent cycles after any cycle in which the subject experiences thrombocytopenia (platelet count of < 30.000/$\mu$L). Subjects will be replaced if fewer than 2 subjects in each dose level complete the Cycle 2, Day 22, evaluations. As this will be an open-label study of rhTPO, there will be no blinding.

**Study Treatment**

Subjects will receive carboplatin chemotherapy for treatment of their disease in conjunction with rhTPO.

**rhTPO**

rhTPO will be provided by Genentech, Inc. as a sterile liquid ready for parenteral administration in 3-mL glass vials. Each vial will deliver 2 mL of 0.5 mg/mL rhTPO in isotonic buffer solution (pH 7.4). Preservative-free normal saline is the only compatible diluent and flush solutions for rhTPO. The formulation does not contain a preservative and is suitable for single-dose administration only.

**a. Dosage, Administration and Storage**

Subjects will be assigned to the dose levels shown in Table 17

TABLE 17

| Dose Levels of rhTPO in Protocol TPO0702g | | |
|---|---|---|
| Dose Level | rhTPO ($\mu$g/kg) | Number of Subjects |
| 1 | 0.6 | 3 |
| 2 | 1.2 | 3 |
| 3 | 2.4 | 3 |
| 4 | 0.3 q 3d$\times$2 | 3 |
| 5 | 0.6 q 3d$\times$2 | 3 |
| 6 | 1.2 q 3d $\times$2 | 3 |
| Total | | 18 |

Subjects in Dose Levels 1, 2, and 3 will receive the assigned dose of rhTPO as a single SC injection on Day 1 of Cycle 0 and on Day 2 of Cycle 2. Subjects in Dose Levels 4, 5, and 6 will receive rhTPO as a single SC injection on Days 1 and 4 of Cycle 0 and on Days 2 and 5 of Cycle 2. If subjects participate in the OEP, they will receive rhTPO as they did in Cycle 2. There will be no rhTPO given during the follow-up period. All rhTPO injections will be prepared and administered according with anaphylaxis precautions in place. rhTPO will be shipped and must be stored under refrigeration at 2°C -8°C (35°F-46°F). rhTPO may be diluted to facilitate accurate administration.

**b. Dose Modification**

There will be no dose modification of rhTPO in this trial. The actual dose will be calculated based on the subject's actual weight during screening.

**Carboplatin Chemotherapy**

Carboplatin will be obtained from commercial sources.

**a. Dosage, Administration, and Storage**

Subjects will receive carboplatin IV over I h on Day 1 of each chemotherapy cycle. The actual dose will be calculated as AUC = 11, using Calvert's formula: total dose = target AUC $\times$ (GFR + 25). If the first cohort of subjects tolerate carboplatin treatment well *(i.e.,* platelet nadir of > 100,000/μL), the dose level of carboplatin may be increased *(e.g.*, AUC = 13). Carboplatin chemotherapy will be administered according to institutional practice and manufacturer's guidelines and must be stored in accordance with manufacturer's guidelines.

**b. Dose Modification**

There will be no dose reduction of chemotherapy due to hematologic toxicity. If a subject experiences hematologic toxicity, G-CSF may be administered as clinically indicated to help diminish potential neutropenia in subsequent chemotherapy cycles. If a subject experiences severe thrombocytopenia (i.e., platelet nadir of < 20.000/μL for > 7 days) in Cycle 2 or beyond, the subject will be removed from the study and placed in the 28-day follow-up period.

**Concomitant Therapy**

Concomitant medications are any prescription medication, over the counter preparation, or medical therapy used by the subject between the 28 days before the study period and the last day of the follow-up period. Subjects using oral contraceptives, hormonal replacement therapy, or other maintenance therapy will be asked to continue their use in a consistent manner throughout the study. All concomitant medication use and therapy should be reported to the investigator and recorded in the CRF.

**Permitted Medications**

Subjects may be administered FDA-approved medications as clinically indicated, except as described for Excluded Therapy. The list of drugs that should be avoided, in the study described in this example, because they may interfere with the function of blood clotting, including the function of the platelets includes Sallcylates, including Acuprin®, Alka-Seltzer®, Arthritis Foundation® safety coated aspirin tablets, Arthritis Strength BC® powder, Asacol® delayed release tablets, Aspirin (acetylsalicylic acid), Ascriptin®, Axotal®, Azodone®, Bayer® aspirin, BC® powder, BC® Cold powder, Bufferin®, Carisoprodol® and Aspirin tablets, Damason-P, Darvon Compound 65®, Diflunisal, Dolobid®, Ecotrin® enteric coated tablets, Empirin®, Equagesic tablets, Excedrin®, Fiorinal® capsules, tablets, and with codeine tablets, Gelpirin®, Halfprin®, Lortab ASA® tablets, Mesalamine, Methocarbamol® and aspirin tablets, Mono-Gesic® tablets, Norgesic® tablets and Norgesic Forte® tablets, Panasal 5/500®, PC-CAP® Propoxyphene Hydroehloride Compound, USP, Pentasa®, Percodan®, Robaxisal®, Rowasa® rectal suppositories or enemas, Roxiprin®, Saflex® tablets, Salicylsalicylic acid (oral), Salsalate, Salsatab® tablets, Soma® compound, and with Codeine, Sulfasalazine, Synalgos-DC® capsules, Talwin® compound, Trilisate® liquid or tablets; Ibuprofen, including Advil®, Co-Advil®, Cramp-End® tablets, Dimetapp® Sinus caplets, Haltran®, Ibuprofen. Ibuprohm®, IBU® tablets, Motrin®, Nuprin®, Sine-Aid® IB, Vicks Day-Quil® with Ibuprofen; Naproxen Sodium, including Aleve®; Ketoprofen including Orudis®; Other Non-Steroidal Anti-Inflammatory Drugs (NSAIDS) including Anaprox®, Anaprox® DS, Ansaid®, Cataflam®, Clinoril®, Daypro®, Diclofenac, Etodolac, Feldene®, Fenoprofen, Flubiprofen, Indocin®, Indomethacin, Ketorolac, Lodine®, Mefanamic acid, Nabumetone, Nalfon® tablets and Nalfon 200® puvules, Naprosyn® suspension and tablets. Oruvail® capsules, Piroxicam, Ponstel®, Relafen® tablets, Sulindac, Tolectin®, Tolmetin, Toradol®, Voltaren®; Other Agents, including coumadin, Dipyridamole, Enoxaparin, Heparin (anticoagulant therapy), Lovenox®, Persantine®, Ticlid® and Ticlopidine.

**Platelet Transfusion**

Prophylactic platelet transfusions may be administered to all subjects with a platelet count of ≤ 20,000/μL and

as clinically indicated for bleeding or perceived risk of bleeding. Platelet transfusions should be administered after the morning platelet count and pharmacokinetic sampling when possible.

**Excluded Therapy**

Aspirin, unless used in the treatment of thrombocytosis, is not permitted while on study. Non-steroidal anti-inflammatory drugs (NSAIDs) and aspirin-containing products must be avoided during this study. Pain management should include agents known not to have anti-platelet effects. Hematopoietic agents other than rhTPO and G-CSF are also excluded during the study period. Use of all experimental (non-FDA-approved for that indication) drugs must be avoided during the study. Radiation therapy may not allowed while on study without approval.

**Study Assessments**

Subjects will be evaluated for eligibility during the 14-day screening period that precedes the first rhTPO administration. Subjects will be closely monitored during the study period, OEP (if applicable), and follow-up period. Subjects will be assessed for adverse events at each visit. Actual dates of study visits may vary by ±2 days depending on subject convenience. Table 18 contains a flowsheet of screening and study evaluations. Pharmacokinetic sampling timepoints are given in Table 19, and OEP and follow-up period assessments are shown in Table 20.

| | Screen | Cycle 0 | | | | Cycle 1 | | | Cycle 2 | | |
|---|---|---|---|---|---|---|---|---|---|---|---|
| | | Baseline | Week 1 | Week 2 | Week 3 | Week 1 | Week 2 | Week 3 | Week 1 | Week 2 | Week 3 |
| Informed consent | x | | | | | | | | | | |
| Medical history | x | | | | | | | | | | |
| ECG | x[a] | | | | | | | | | | |
| Chest X-ray | x[a] | | | | | | | | | | |
| Serum pregnancy | x | | | | | | | | | | |
| Anti-TPO antibodies | | x[b] | | Day 8 | Day 15 | Day 1 | Day 8 | Day 15 | Day 1 | Day 8 | Day 8 |
| Vital Signs | | x | x[c] | | | | | | x[c] | | |
| Physical examination | x | | | | | | | | | | |
| CBC, differential, platelets[d] | x | x | Days 1-5 | 3 × week | 3 × week | 3 × week | 3 × week | 3 × week | 3 × week | 3 × week | 3 × week |
| Serum chemistry | x | | | | Day 22[e] | | | Day 22[e] | | | |
| Bone marrow biopsy and aspirate | | x | | Day 8 | | | | | | | |
| Urinalysis | x | | | | Day 22[e] | | | | | | |
| Serum TPO (1, 2, 3) | | | x[f] | | | | | | x[f] | | |
| Serum TPO (4, 5, 6) | | | x[f] | x[f] | | | | | x[f] | | |
| rh TPO administration (1, 2, 3) | | | Day 1 | | | | | | Day 2 | | |
| rh TPO administration (4, 5, 6) | | | Days 1, 4 | | | | | | Days 2, 5 | | |
| Carboplatin | | | | | | Day 1 | | | Day 1 | | |
| Assess tumor status | | | | | | x[g] | | | | | |
| Record adverse events/meds | | | Each visit | Each visit | Each visit | Each visit | Each visit | Each visit | Each visit | Each visit | Each visit |

**Table 18**
**Study Flowsheet: Study Period**

a Within 28 days of Cycle 0, Day 1.

b Three samples, at least two of which are separated by ≥24 h (one sample 5 min before the initial rhTPO administration on Day 1 of Cycle 0.

c Before and 1 h after rhTPO administration.

d CBC, differential and platelet counts will be taken at least 3 times weekly; daily counts will be done when the platelet count si <50,000/µL.

e Day 22 is equivalent to Day 1 of the following cycle.

f See Table 19

g Within 4 weeks before Cycle 1, Day 1.

EP 1 146 895 B1

Table 19

| | | | | | | | | | | | | | |
|---|---|---|---|---|---|---|---|---|---|---|---|---|---|
| **Study Flowsheet: Pharmacokinetic Sampling** | | | | | | | | | | | | | |
| Dose Groups 1.2. and 3 | | | | | | | | | | | | | |
| Cycle | 0 | | | | | | | | | | | | |
| Day | 1 | | | | | | | | 2 | 3 | 4 | 5 | 6 |
| | | 0.5 | 1 | 2 | 4 | 6 | 8 | 10 | 24 | 48 | 72 | 96 | 120 |
| Timepoint | Pre | hr | hr | hr | hr | hr | hr | hr | hr | hr | hr | hr | hr |
| Serum TPO | x | x | x | x | x | x | x | x | x | x | x | x | x |
| NB: Timepoints are given as hours after rhTPO administration on Day 1. | | | | | | | | | | | | | |
| Cycle | 2 | | | | | | | | | | | | |
| Day | 2 | | | | | | | | 3 | 4 | 5 | 6 | 7 |
| | | 0.5 | 1 | 2 | 4 | 6 | 8 | 10 | 24 | 48 | 72 | 96 | 120 |
| Timepoint | Pre | hr | hr | hr | hr | hr | hr | hr | hr | hr | hr | hr | hr |
| Serum TPO | x | x | x | x | x | x | x | x | x | x | x | x | x |
| NB: Timepoints are given as hours after rhTPO administration on Day 2. | | | | | | | | | | | | | |
| Dose Groups 4. 5, and 6 | | | | | | | | | | | | | |
| Cycle | 0 | | | | | | | | | | | | |
| Day | 1 | 4 | | | | | | | 5 | 6 | 7 | 8 | 9 |
| | | 0.5 | 1 | 2 | 4 | 6 | 8 | 10 | 24 | 48 | 96 | 120 | |
| Timepoint | Pre | Pre | hr | hr | hr | hr | hr | hr | hr | hr | hr | hr | hr |
| Serum TPO | x | x | x | x | x | x | x | x | x | x | x | x | x |
| NB: Timepoints are given as hours after rhTPO administration on Day 4. | | | | | | | | | | | | | |
| Cycle | 2 | | | | | | | | | | | | |
| Day | 2 | 5 | | | | | | | 6 | 8 | 9 | 10 | |
| | | 0.5 | 1 | 2 | 4 | 6 | 8 | 10 | 24 | 72 | 96 | 120 | |
| Timepoint | Pre | Pre | hr | hr | hr | hr | hr | hr | hr | hr | hr | hr | hr |
| Serum TPO | x | x | x | x | x | x | x | x | x | x | x | x | x |
| NB: Timepoints are given as hours after rhTPO administration on Day 5. | | | | | | | | | | | | | |

Table 20

| | Cycles 3-6 | | Follow-Up | |
|---|---|---|---|---|
| | **Week 1** | **Week 2** | **Week 3** | **Week 4** |
| **Study Flowsheet Optional Extension Period and Follow-Up Period** | | | | |
| Anti-TPO antibodies | Day 1 | Day 8 | Day 15 | Day 28 |
| Vital signs | ×[a] | | | Day 28 |
| Physical examination | | | | Day 28 |
| CBC, differential, and platelets | 3 × week[b] | 3 × week[b] | 3 × week[b] | Day 28 |

[a] Pre-cycle and before and 1 h after rhTPO administration.

[b] CBC, differential, and platelet counts will be taken at least 3 times weekly; daily counts will be done when the platelet counts I <50,000/μL.

Table 20   (continued)

| Study Flowsheet Optional Extension Period and Follow-Up Period | | | | |
|---|---|---|---|---|
| | Cycles 3-6 | | Follow-Up | |
| | Week 1 | Week 2 | Week 3 | Week 4 |
| Serum chemistry | | | Day 22[c] | Day 28 |
| rhTPO adminstration (1, 2, 3) | Day 2 | | | |
| rhTPO administration (4, 5, 6) | Days 2, 5 | | | |
| Carboplatin | Day 1 | | | |
| Record adverse events/medications | Each visit | Each visit | Each visit | Day 28 |

[c]Day 22 is equivalent to Day 1 of the following cycle.

## Screening and Pre-Treatment Assessments

All screening assessments must be completed within the 14 days preceding the initial rhTPO administration on Day 1 of Cycle 0, unless indicated otherwise. Pre-treatment (baseline) assessments will be obtained prior to rhTPO administration on Day I of Cycle 0.

### a. Screening Assessments

The following screening assessments will be taken: a signed informed consent: past/present medical history, including demographics and concomitant medication use; a physical examination; an electrocardiogram (within 28 days of Cycle 0, Day 1); a chest X-ray (within 28 days of Cycle 0, Day 1); CBC, differential, and platelet count; serum chemistry; and a serum pregnancy test (if applicable).

### b. Pre-Treatment Assessments (Baseline)

The following assessments will be taken to establish baseline values; vital signs CBC, differential, and platelet count; urinalysis; anti-TPO antibody level (three samples, at least two of which are separated by $\geq 24$ h, and one sample at 5 min before the initial rhTPO administration); and a bone marrow biopsy and aspirate.

## Assessments during Treatment

### a. Cycle 0

In cycle 0, the following assessments will be taken: vital signs (before and 1 h after rhTPO administration); CBC, differential, and platelet count (daily for Days 1-5 and then at least 3 times weekly); serum chemistry (Day 22); urinalysis (Day 22); anti-TPO antibody level (Days 8; 15, and 22); bone marrow biopsy and aspirate (Day 8; an additional bone marrow exam may be performed to evaluate progenitor cell kinetics); and serum TPO level (in selected subjects).
Assessments will be taken for dose levels 1, 2, and 3 before and 0.5, 1, 2, 4, 6, 8, 10, 24, 48, 72, 96, and 120 hours after rhTPO administration on Day 1 (see Table 19). Assessments will be taken for dose levels 4, 5, and 6 before rhTPO administration on Day 1; before and 0. 5, 1, 2, 4, 6, 8, 10, 24, 48, 96, and 120 hours after rhTPO administration on Day 4 (see Table 19). Reverse events, medications changes, and blood product use will be recorded.

### b. Cycles 1 and 2

In cycle 0, the following assessments will be taken: vital signs (before and 1 h after rhTPO administration); CBC, differential, and platelet count (at least 3 times weekly; daily counts when platelet count is $\leq 50,000/\mu L$); serum chemistry (Day 22); anti-TPO antibody levels (Days 1, 8, 15, and 22); and serum TPO level (in selected subjects).
Assessments will be taken for dose levels 1, 2, and 3: before and 0.5, 1, 2, 4, 6, 8, 10, 24, 48, 72, 96, and 120 h after rhTPO administration on Day 2 (Cycle 2 only; see Table 19). Assessments will be taken for dose levels dose levels 4, 5, and 6: before rhTPO administration on Day 2; before and 0.5, 1, 2, 4, 6, 8, 10, 24, 72, 96, and 120 h after rhTPO administration on Day 5 (see Table 19).
Radiographic studies to assess tumor status (within 4 wk before Cycle 1. Day 1), record adverse events, medications changes, and blood product use will be recorded.

### c. Optional Extension Period (Cycles 3 to 6)

Assessments will be taken for vital signs (pre-cycle and before and 1 h after rhTPO administration); CBC, differential, and platelet count (at least 3 times weekly; daily counts when platelet count is $\leq$ 50.000/$\mu$L); serum chemistry (Day 22): anti-TPO antibody level (Days 1, 8, 15, and 22); adverse events, medications changes, and blood product use.

### Follow-Up Assessments

Subject will be followed for at least 28 days beyond their last cycle with rhTPO. Subjects will also be seen as clinically indicated. Subjects with any anti-TPO antibody-related thrombocytopenia will be followed to resolution. Follow-up assessments will include the following: vital signs; symptom-directed physical examination; CBC, differential, and platelet count; serum chemistry; anti-TPO antibody level; record adverse events, medications changes, and blood product use.

### SUBJECT DISCONTINUATION

Subjects are free to withdraw from study participation at any time during the study. If a subject wishes to withdraw from the study, she should be encouraged to return to the clinic for early termination evaluation. An early termination visit should include all evaluations scheduled for Day 1 of that cycle or Follow-Up Period Day 28. Early termination visits should be scheduled as close as possible to the actual projected visit day.

Subjects will be discontinued from rhTPO administration in the event of DLT, neutralizing anti-TPO antibody formation, or disease progression at the end of Cycle 2. If a subject is discontinued from rhTPO administration, she should be encouraged to complete all regularly scheduled evaluations for that cycle and complete the follow-up period.

### STUDY DISCONTINUATION

Reasons for terminating the study may include the following: the incidence or severity of adverse events in this or other studies indicates a potential health hazard to subjects; the subject enrollment is unsatisfactory; the data recording is inaccurate or incomplete.

### STATISTICAL METHODS

### Analysis of the Conduct of the Study

Subject enrollment will be summarized by dose level. Subject disposition will be tabulated by dose level and chemotherapy cycle; reasons for rhTPO or study discontinuation will be listed. Chemotherapy and rhTPO administration will also be summarized by dose level and cycle. Frequency and duration of delays in administration will be summarized. A log of protocol violations and deviations will be maintained and summarized by dose level. Finally, subject demographic and disease-related characteristics will be summarized by dose level to assess comparability of subjects.

### Activity Analysis

Given the trial sample size and focus, the statistical analysis of activity will be primarily descriptive in nature. Platelet counts are the pharmacodynamic endpoint of primary interest. Time profiles of platelet, WBC, and neutrophil counts will be prepared for each subject over chemotherapy cycles. Absolute and relative maximum platelet change from baseline will be summarized graphically across dose levels to assess dose response.

### Pharmacokinetic Analysis

Serum TPO concentration-time profiles will be presented graphically for each subject for Cycles 0 and 2. Maximum measured serum concentration ($C_{max}$) and the time of maximum TPO concentration ($T_{max}$) will be recorded for each profile. Each profile will be evaluated by standard pharmacokinetic analysis methods. Pharmacokinetic parameters to be calculated include the area under the serum TPO concentration-time profile (AUC), the mean residence time ($MRT_{SC}$), and the half-life for the decline of serum TPO concentrations.

Pharmacokinetic parameters will be compared across the six dose levels for examination of dose proportionality. The relationships between selected pharmacokinetic parameters and measures of response will be examined.

## Safety Analysis

Demographic, physical examination, clinical laboratory, and adverse event data will be tabulated and summarized by dose level and chemotherapy cycle when appropriate. Special attention will be given to the incidence of serious adverse events and toxicities classified as Grade $\geq$ 3 according to the NCI Common Toxicity Criteria. Clinical laboratory values outside the normal range will be identified. Any subject receiving one or more doses of rhTPO will be considered evaluable for safety.

Incidence of antibodies to TPO will be tabulated, and antibody titers will be summarized by dose level and number of prior rhTPO doses. The relationship between antibody titers and subsequent platelet counts will be investigated.

## Determination of Example Size

The number of subjects at each dose level was selected to balance potential subject risk and information needed to provide preliminary evaluations of safety, pharmacokinetics, and activity. The study will enroll 3 subjects at each dose level until escalation is complete or is stopped because the MTD has been reached or the OBD has been defined. For events such as the occurrence of a specific adverse event, DLT, or formation of antibodies to TPO, this sample size guarantees at least 80% power at each dose level to detect one or more events if the true event probability is $\geq$41%.

## EXAMPLE 10

## RECOMBINANT HUMAN THROMBOPOIETIN ATTENUATES CARBOPLATIN-INDUCED SEVERE THROMBOCYTOPENIA AND THE NEED FOR PLATELET TRANSFUSIONS IN PATIENTS WITH GYNECOLOGIC MALIGNANCY

Thrombocytopenia is a significant clinical problem in the management of cancer patients that can increase the risk for bleeding complications and the need for platelet transfusions and compromise the dose-intensity of chemotherapy. The lack of other significant non-hematologic toxicities over a wide dose-range makes carboplatin a suitable agent to test the potential of a thrombopoietic agent to attenuate cumulative thrombocytopenia without compromising the doses of carboplatin for multiple cycles. To assess the clinical safety and ability of recombinant human thrombopoietin (rhTPO) to ameliorate chemotherapy-induced severe thrombocytopenia., the inventor initiated a phase I/II clinical trial of rhTPO in patients with advanced or recurrent gynecologic malignancy who were at high risk for chemotherapy-induced severe thrombocytopenia. The objectives of the trial were to assess the clinical tolerance and hematopoietic effects of rhTPO alone prior to chemotherapy, and to evaluate the clinical safety and the activity of rhTPO in ameliorating severe thrombocytopenia associated with chemotherapy, thus reducing the need for platelet transfusions following chemotherapy, and allowing patients to receive multiple cycles of chemotherapy at full doses. The use of rhTPO particularly reduces the need for platelet transfusions when used as a secondary prophylaxis.

## Method

The inventor performed a phase I/II study of rhTPO prior to and following carboplatin in 29 patients with advanced gynecologic malignancy. During the dose-escalation phase, rhTPO was administered by subcutaneous injection as a single dose (0.6, 1.2, 2.4, and 3.6 mcg/kg) prior to carboplatin, and for four doses following a second cycle of carboplatin. During dose-expansion phase (n=12). rhTPO was used at the optimal biologic dose only as a secondary prophylaxis for severe thrombocytopenia.

## Patients

Patients with any recurrent or advanced gynecologic malignancy for whom treatment with carboplatin was indicated were eligible for this trial. Patients were required to have adequate Karnofsky performance status ($\geq$80%) bone marrow (WBC count $\geq 3 \times 10^3/mm^3$, and platelet count $\geq 150 \times 10^3/mm^3$ and $\leq 450 \times 10^3/mm^3$), renal (serum creatinine $\leq$1.5 mg/dL or a calculated creatinine clearance of $\geq$60 mL/min). and hepatic (total bilirubin $\leq$1.5 mg % and SGOT or SGPT $\leq$3 times normal) functions, and a life expectancy of $\geq$3 months. Patients with a rapidly progressive disease, a lack of response to prior platinum-based therapy, a history of pelvic irradiation or high-dose chemotherapy with stem cell transplantation, surgery within two weeks, use of chemotherapy or radiotherapy within four weeks, nitrosoureas or mitomycin-C within six weeks of the study entry, $\geq$3 different types of prior chemotherapy regimens, a history of thromboembolic or bleeding disorders, or significant cardiac disease were excluded from the trial. Written informed consent was obtained from all patients before study entry in accordance with institutional guidelines.

### Study Design

The schema of the study design is shown in FIG. 1. The study included two components, a dose-escalation (safety) component and dose-expansion (activity) component using the optimal dose. During the dose-escalation part of this trial, rhTPO was administered as a single dose by subcutaneous injection 3 weeks before chemotherapy in order to evaluate clinical tolerance and the hematopoietic effects of rhTPO alone. Subsequently, patients received carboplatin intravenously over one hour at a dose calculated to provide an area under curve (AUC) of 11 using Calvert's formula [total dose = target AUC x (glomerular filtration rate [GFR] + 25)]. Upon recovery from Cycle-1, patients received carboplatin (AUC=11) to be followed by rhTPO at the same dose administered every other day for four doses (days 2, 4, 6, and 8). Thus Cycle- 1 (chemotherapy alone) served as an internal control for each patient for Cycle-2 (chemotherapy with rhTPO).

At least three patients were entered at each dose level of rhTPO (0.6, 1.2, 2.4, and 3.6 mcg/m$^2$/d). The optimal biologic dose (OBD) was defined as the lowest active dose level at which comparison between dose levels demonstrated that the trend in platelet response plateaued during Cycle-2 as compared to the next dose level. Once OBD was established, the prechemotherapy cycle of rhTPO was deleted, and 12 patients were treated with chemotherapy cycles consisting of carboplatin alone, until severe thrombocytopenia (nadir platelet count <30 $\times$ 10$^3$/mm$^3$) was observed. In the subsequent cycles, rhTPO was used at OBD as a secondary prophylaxis by same schedule [days 2, 4, 6, 8 (n=6)] or by a modified schedule [days -1, 1, 3, 5 (n=6)]. The latter schedule was used to assess whether earlier administration of TPO can further augment the degree of protection. The rationale for this schedule was based on recent preclinical studies in primates and the inventor's prior clinical experience in the sarcoma that indicated that the administration of rhTPO prior to chemotherapy may enhance platelet recovery.

After completion of the two cycles of chemotherapy, all patients with a stable or responding disease who had not experienced severe thrombocytopenia *(i.e.* days platelet count <20,000/mm$^3$ for >7 days) in Cycle-2 and who had not developed neutralizing anti-TPO antibodies were eligible to receive additional cycles of same dose of carboplatin (maximum of 6 cycles) with rhTPO. Patients received platelet transfusions for severe thrombocytopenia *(i.e.* platelet count <20,000/mm$^3$), RBC transfusions for anemia *(i.e.* hemoglobin [Hb]<8 gm/dL), and antibiotics for neutropenic fever *(i.e.* temperature $\geq$38.3°C and absolute neutrophil count <500/mm$^3$) or when clinically indicated. The use of G-CSF was not allowed during the first two cycles of chemotherapy, but was allowed in the later cycles (3 - 6) only if clinically indicated *(i.e.* as a secondary prophylaxis in those patients who experienced prolonged neutropenia or delayed neutrophil recovery).

### Clinical and Laboratory Monitoring

Before and during the course of the clinical trial, patients were monitored with complete history, physical examinations, and laboratory tests including complete blood cell counts, serum chemistry, urinalysis, chest X-ray, and electrocardiograph. Blood counts were performed three times a week until the expected nadir period was reached and then daily until the counts had recovered. Serum samples were examined for antibodies reactive to full length TPO three times prior to study and weekly while on study. Positive sera (optical density ratio $\geq$1.5) were further analyzed by anti-truncated TPO antibody assay, C-mpl blocking assay, and cell-proliferation biologic assay. Patients positive in all assays were considered to be at risk for antibody-induced thrombocytopenia. Bone marrow (BM) aspiration and biopsy were done in all patients before, and one week after rhTPO treatment, and in selected patients at a later time point (day 10 or 14) in the prechemotherapy cycle. Tumor response was evaluated by physical examination, tumor marker (CA-125) evaluation, and imaging studies performed at periodic intervals.

### Statistical Methods

Hematopoietic toxicity from Cycle-1 1 of chemotherapy (no rhTPO) was compared with the results from Cycle-2 (with rhTPO) by means of a paired T test for continuously measured variables. The end points that were considered for analysis included nadir platelet counts, and the duration of thrombocytopenia (<20,000/mm$^3$, <50,000/mm$^3$, and <100,000/mm$^3$), and the time of recovery to platelet counts $\geq$ 100,000/mm$^3$. The difference in the nadir counts (C2 - C1) was also analyzed for dose-response using linear regression analysis. The time of recovery to platelet counts to $\geq$50,000/mm$^3$ and $\geq$100,000/mm$^3$ was compared using Mann Whitney Rank Sum test. In addition, the proportion of patients requiring platelet transfusions in cycles with or without rhTPO was compared using McNemar's test.

### Recombinant Human Thrombopoietin (rhTPO)

rhTPO used in this study was obtained from Genentech, Inc. (South San Francisco, CA). rhTPO is a full length, glycosylated molecule produced in a genetically modified mammalian cell line. It was purified by standard techniques and provided in preservative-free normal saline as a diluent for injections.

### RESULTS

### Patients

Twenty-nine patients with advanced gynecologic malignancy were entered onto this trial (Table 21). All patients had a diagnosis of ovarian carcinoma except for one patient who had primary peritoneal malignancy. The majority of the patients had received at least one platinum-based regimen, and several had received additional systemic and/or intraperitoneal therapies. All but one patient were evaluable for clinical tolerance and hematopoietic response to rhTPO; one patient, who refused further treatment after one cycle of chemotherapy and did not receive rhTPO was considered inevaluable.

Table 21

| PATIENT CHARACTERISTICS n=29 | |
| --- | --- |
| Median age (range) | 57(40-70) |
| Median KPS (range) | 100 (80-100) |
| Prior Therapies: Systemic | |
| Chemotherapy | 26 |
| - Platinum based regimen | 24 |
| - Taxane based | 14 |
| Hormonal | 13 |
| Prior Therapies: Intraperitoneal | |
| Taxol | 1 |
| IFN/IL-2 | 2 |

### Pre-chemotherapy rhTPO

Effects on blood counts: Treatment with a single dose of rhTPO (n=16) in pre-chemotherapy cycle was associated with a dose-dependent increase (FIG. 2) in circulating platelet count (baseline mean, $277 \times 10^3/mm^3$; maximal mean, $462 \times 10^3/mm^3$; P<0.001). The rise in platelets was gradual with a peak effect observed on a median day 15 (FIG. 2). After the peak response, the platelet count gradually declined towards baseline. The platelet counts around day 21 (prior to chemotherapy), remained somewhat higher than the baseline level (347 vs. $277 \times 10^3/mm^3$ P<0.01). The platelets appeared normal in morphology, and no major changes were seen in white blood cell counts or in hematocrit values.

Effects on bone marrow: The rise in platelet count was associated with a significant increase in the number of BM megakaryocytes ($34 \pm 4$ vs. $48 \pm 5$, per 10 HPF. P<0.01). The megakaryocytes appeared normal in morphology: some were large in size with multilobulated nuclei and mature cytoplasm. The myeloid and erythroid cells showed normal maturation. While the day 7 bone marrows from six of 15 patients showed increased erythroid cells, the overall myeloid: erythroid cell ratios and the cellularity remained unchanged. However, all six patients who had BM examined on later time points (days 10 and 14) showed significant increases in erythroid cells (mean normoblast. 21.8% vs. 31.3%, P<0.01).

### Effects of rhTPO on Myelosuppression

Twenty-eight patients received rhTPO following carboplatin, sixteen in dose-escalation part of the study, and twelve in the dose-expansion phase.

<u>Dose Escalation Phase:</u> In this group of patients (n=16), administration of rhTPO resulted in a significant reduction in both the degree and duration of thrombocytopenia. The relationship between rhTPO dose and degree of thrombocytopenia is shown in Table 22. As compared to Cycle-1 (without rhTPO), the mean nadir platelet count in Cycle-2 (with rhTPO) was no different at 0.6 mcg/kg, and was higher by 2-fold at 1.2 mcg/kg, 1.7-fold at 2.4 mcg/kg, and 1.2-fold at 3.6 mcg/kg. There was no evidence for a linear dose response when the data was analyzed in a paired manner, using each patient as her own control (difference Cycle 2 - Cycle 1). However, the difference in nadir platelet count was significantly higher ($P$<0.03 using one-sided $t$-test) for the groups that received 1.2 and 2.4 mcg/kg as compared to the 0.6 and 3.6 mcg/kg groups (mean difference C2 - C1, - 6.0, 26.8, 26.6, and 7.0 for the doses 0.6, 1.2, 2.4, and 3.6 mcg/kg, respectively). Since there was no difference between 1.2 and 2.4 mcg/kg cohorts ($P$>0.99), 1.2 mcg/kg was found to be the lowest active dose, and was considered as OBD for this chemotherapy regimen.

Table 22

| Effects of Recombinant Human Thrombopoietin at Different Dose Levels on Carboplatin-Induced Thrombocytopenia | | | | | | |
|---|---|---|---|---|---|---|
| Dose of rhTPO (mcg/kg) (n) | Platelet Nadir ($\times 10^3/mm^3$) | | Days Platelets <50 $\times 10^3/mm^3$ | | Days Platelets <100 $\times 10^3/mm^3$ | |
| | Cycle-1 | Cycle-2 | Cycle-1 | Cycle-2 | Cycle-1 | Cycle-2 |
| 0.6 (3) | 45 ± 10 | 42 ± 15 | 2 ± 1 | 2 ± 2 | 8 ± 1 | 6 ± 2 |
| 1.2(5) | 28 ± 7 | 55 ±11 | 4 ± 1 | 1 ± 1 | 9 ± 1 | 4 ± 1 |
| 2.4(5) | 36 ± 15 | 62 ±23 | 7 ± 3 | 3 ± 2 | 10 ± 3 | 8 ± 4 |
| 3.6 (3) | 37 ± 23 | 44 ± 26 | 10 ± 6 | 7 ± 4 | 15 ± 7 | 11 ± 5 |

Data shown represents mean ± SEM values

For all doses of rhTPO combined (0.6 to 3.6 mcg/kg), the nadir platelet count was higher (53 $\times 10^3/mm^3$ vs. 35 $\times 10^3/mm^3$ $P$=.007) and the duration of thrombocytopenia shorter (days platelet count <50 $\times 10^3/mm^3$, 6 vs. 3 days, $P$=0.002, and days platelet count <100 $\times 10^3/mm^3$, 10 vs. 7 days P<0.001) in Cycle-2 as compared to Cycle-1 (Table 23).

## Table 23

### Effects of Recombinant Human Thrombopoietin Treatment on Carboplatin-Induced Thrombocytopenia

| Patient Group* | Platelet Nadir | | Days Platelets $<20 \times 10^3/mm^3$ | | Days Platelets $<50 \times 10^3/mm^3$ | | Days Platelets $<100 \times 10^3/mm^3$ | |
|---|---|---|---|---|---|---|---|---|
| (n) | C-1 | C-2 | C-1 | C-2 | C-1 | C-2 | C-1 | C-2 |
| Dose Escalation (n=16) | 35 ± 6 | 53 ± 9 | 2 ± 1 | 1 ± 1 | 6 ± 1 | 3 ± 1 | 10 ± 2 | 7 ± 2 |
| P Value | 0.007 | | 0.070 | | 0.002 | | <0.001 | |
| Secondary Prophylaxis (n=11) | 17 ± 6 | 38 ± 10 | 5 ± 1 | 1 ± 1 | 8 ± 1 | 5 ± 1 | 13 ± 1 | 9 ± 2 |
| P Value | <0.05 | | 0.002 | | 0.023 | | 0.05 | |
| All Patients (n=27) | 28 ± 4 | 49 ± 7 | 3 ± 1 | 1 ± 1 | 7 ± 1 | 4 ± 1 | 11 ± 1 | 8 ± 1 |
| P Value | <0.001 | | <0.001 | | <0.001 | | <0.001 | |
| Optimal Biologic Dose (1.2 mcg/kg) (n=16) | 20 ± 3 | 44 ± 8 | 4 ± 1 | 1 ± 1 | 7 ± 1 | 4 ± 1 | 12 ± 1 | 8 ± 1 |
| P Value | 0.007 | | 0.001 | | 0.004 | | 0.004 | |

Data shown represents mean ± SEM values

* One of the 28 evaluable patients, who qualified to receive rhTPO as a secondary prophylaxis in Cycle-3, is not included. This patient also had a higher nadir platelets (47 vs 18 x $10^3/mm^3$) in Cycle-3 (with rhTPO) as compared to Cycle-2 (without rhTPO).

Dose Expansion Phase (Secondary Prophylaxis): To better assess the ability of rhTPO (at OBD) to attenuate severe thrombocytopenia and the need for platelet transfusions, cohort of six patients received cycles of carboplatin alone until they experienced severe thrombocytopenia. rhTPO was used in the subsequent cycles as a secondary prophylaxis (1.2 mcg/kg), using the same schedule described above *(i.e.* Days 2, 4, 6, and 8). As shown in FIG. 3, rhTPO in this group of patients reduced the degree of the platelet nadir ($16 \times 10^3/mm^3$ vs. $49 \times 10^3/mm^3$), delayed the onset of the nadir, and accelerated platelet recovery such that the duration of severe thrombocytopenia $<20 \times 10^3/mm^3$ was significantly reduced (4.2 vs. 1.2 days, *P*<0.01). Five of the six patients had experienced platelet nadirs $<20 \times 10^3/mm^3$ requiring platelet transfusions in Cycle-1. Administration of rhTPO in Cycle-2 eliminated the need for platelet transfusions in 3 of these patients and reduced it in a fourth patient.

In addition, a cohort of six patients were treated with rhTPO (1.2 mcg/kg) starting from a day prior to chemotherapy (Days -1, 1, 3, 5) to determine whether the degree of protection could be augmented by this schedule. Five of the six patients in this cohort experienced severe thrombocytopenia following carboplatin alone in Cycle-1. The administration of rhTPO in Cycle-2 resulted in a reduction in the duration of severe thrombocytopenia $<20 \times 10^3/mm^3$ from 6 days to 2 days and the need for the platelet transfusion was eliminated in 3 of these 5 patients. As shown in FIG. 4A, a patient had experienced severe thrombocytopenia in Cycle-1 and required two platelet transfusions prior to platelet recovery. The use of rhTPO in Cycle-2 by this schedule, raised the platelet nadir, and eliminated the need for platelet transfusion in Cycle-2. As shown in FIG. 4B, the sixth patient did not experience severe thrombocytopenia in Cycle-1 (platelet nadir, $47 \times 10^3/mm^3$) and therefore did not receive rhTPO in Cycle-2. The Cycle-2 was accompanied by severe thrombocytopenia (platelet nadir, $18 \times 10^3/mm^3$) requiring platelet transfusion, demonstrating the cumulative nature of hematopoietic toxicity seen with carboplatin. The administration of rhTPO in Cycle-3 elevated the platelet nadir (18 *vs.* $47 \times 10^3/mm^3$) and abrogated the need for platelet transfusion. Thus, of the 12 patients that received rhTPO as a secondary prophylaxis, 11 received rhTPO in Cycle-2 and one in Cycle-3. As shown in Table 22, rhTPO used as secondary prophylaxis raised the platelet nadir by 2-fold and reduced the duration of severe thrombocytopenia by 4 days.

Platelet Transfusions: Of the 28 evaluable patients, 27 received carboplatin alone in Cycle-1 and carboplatin with rhTPO in Cycle-2. Patients were transfused platelets using the same platelet transfusion trigger (<20,000/mm^3) for cycles with or without rhTPO. The documented nadir platelet counts at which patients actually received platelet transfusions were similar for both Cycle-1 (mean 13.8 and median $16 \times 10^3/mm^3$) and Cycle-2 (mean 14.4 and median $16 \times 10^3/mm^3$). The need for platelet transfusion was markedly reduced in Cycle-2 as compared to Cycle-1 (Table 24). Specifically, in the group that received rhTPO at OBD (1.2 mcg/kg) in Cycle-2 (n=16), the proportion of patients that required the platelet transfusion was reduced from 75% in Cycle-1 to 25% in Cycle-2 P=0.01). The number of transfusions required in this group was also reduced by 69% (16 vs.5 transfusions in Cycle-1 and Cycle-2, respectively).

TABLE 24

| Incidence of Platelet Transfusions During Chemotherapy Cycles | | | |
|---|---|---|---|
| | Number (%) of Patients Requiring Platelet Transfusions | | |
| **Patient Group** | **Cycle-1 (Carboplatin)** | **Cycle-2 (Carboplatin+ rhTPO)** | **P-Value** |
| **All Patients\* (0.6 - 3.6 mcg/kg) n=27** | 16 (59%) | 7 (26%) | 0.02 |
| **Optimal Biologic Dose (1.2 mcg/kg) n=16** | 12(75%) | 4(25%) | 0.01 |

\* One of the 28 evaluable patients who qualified to receive rhTPO as secondary prophylaxis in Cycle-3, is not included. This patient required platelet transfusion in Cycle-2 (without rhTPO) and did not require in Cycle-3 (with rhTPO).

Platelet Recovery: The recovery of platelets to $\geq 50 \times 10^3/mm^3$ (*P*<0.019) and $\geq 100,000/mm^3$ (*P*<0.017) was also significantly enhanced with rhTPO (FIG. 5). 67% of patients who received rhTPO in Cycle-2 recovered their platelets by day 21 to a level $\geq 100 \times 10^3/mm^3$, as compared to 37% of patients in Cycle-1 without rhTPO.

**Effects of rhTPO on Cumulative Myelosuppression**

Twenty three of 28 patients received more than two cycles of carboplatin based on evidence of a stable or responding disease and a lack of excessive hematologic toxicity. Of these 23 patients, 13 had required platelet transfusions in Cycle-1. Ten of these 13 patients did not require platelet transfusions in Cycle-2 with rhTPO, and 7 of these ten high risk patients still remained free of platelet transfusion in Cycle-3. Overall, the need for platelet transfusion in all patients in the later cycles (Cycles 2 - 6) was 41% (37 of 91 cycles).

Severe neutropenia was generally not observed with this regimen. The mean nadir neutrophil counts (ANC) were $0.58 \times 10^3/mm^3$ and $0.79 \times 10^3/mm^3$ ($P$<0.03) for Cycles 1 and 2, respectively. The incidence of febrile neutropenia (fever $\geq$38.3°C with ANC <500/$mm^3$) was low in both cycles (3 vs. 0 in Cycles-1 and 2, respectively) and remained low for the entire study period (1 of 91 in Cycles 2-6). The mean nadir hemoglobin values were 9.1 and 8.3 ($P$<0.01) for Cycles I and 2, respectively. The anemia was cumulative in nature, requiring red cell transfusions in higher proportion of patients in later cycles (19% in Cycle-1 and 37% in Cycles 2-6).

**Tumor Response:**

Twenty-eight evaluable patients received a total of 119 cycles (median, 5 cycles; range 2 to 6) of carboplatin at full dose. Twenty-six patients had measurable disease and two had microscopic disease. Major responses were seen in 14 (54%) patients (four complete and ten partial responses). Nine patients had a minor response or stable disease and 4 had a progressive disease. In addition, of the two patients without a measurable disease, one had a serologic partial response (a decrease in CA-125 from 357 to 55).

**Clinical Tolerance**

rhTPO was well tolerated by all patients. No significant adverse events related to rhTPO or thrombocytosis were observed. During the prechemotherapy phase, few patients reported occasional headache, myalgia, mild decrease in appetite or fatigue in their symptom record diaries. However, there were no consistent symptoms nor there was a temporal relationship of these symptoms with the timing of rhTPO administration, to distinguish these from symptoms of their underlying disease. The side effects were similar for both the cycles of chemotherapy with or without rhTPO (Table 25) and were typical of those reported for carboplatin. No local skin reactions, fluid retention, thromboembolic events or major organ toxicity were seen.

Table 25

| Reported Adverse Events During Chemotherapy Cycles | | |
|---|---|---|
| **Toxicity** | **Cycle-1 Carboplatin alone n=27** | **Cycle-2 Carboplatin + rhTPO n=27** |
| Fever | 12 | 11 |
| Chills | 8 | 3 |
| Fatigue | 12(10)* | 11(10) |
| Myalgia | 10 | 3 |
| Headache | 11(4) | 10(2) |
| Bone pain | 7 | 3 |
| Nausea | 18(3) | 19(1) |
| Vomiting | 12(1) | 10 |
| Diarrhea | 8(1) | 8(1) |
| Anorexia | 14(1) | 11(5) |
| Constipation | 11(2) | 11(2) |
| Mucositis | 4 | 5 |

*\* **Numbers** in **parenthesis indicate the number of patients experiencing grade $\geq$3 toxicity**

A 50-year old patient who completed all 6 cycles of chemotherapy, presented with a weakness in the right upper extremity 6 weeks from last dose of rhTPO. The CT scan of the brain was negative and the patient recovered most of motor deficit within 24 h. suggesting a mild cerebro-vascular accident. The presence of a normal platelet count (*i.e.* 228,000/$mm^3$) and a long interval from rhTPO administration, makes this event unlikely to be related to rhTPO.

Serum samples were tested for the presence of antibodies to TPO prior to and during the study. Seven patients were noted to have antibody activity in an assay directed to the full length molecule. Three of these 7 patients had the antibody activity at the screening, even before initiating treatment with rhTPO. Three out of the remaining 4 patients showed antibody activity directed to the truncated molecule. However, C-mpl blocking activity was detected in only two of these patients. The first patient had been heavily pretreated with carboplatin and cyclophosphamide, received 5 cycles of full dose carboplatin. On day-22 of Cycle-5, low titers of the antibodies (optical density ratio, 1.65 which decreased to 1.29 by day 30) that bound to TPO were noted. Although the antibodies exhibited C-mpl blocking activity, the results of the cell proliferation bioassay indicated that these were non-neutralizing. The slow recovery of platelets to $100,000/\text{mm}^3$ (day 84) in this patient could have been attributed to her cumulative exposure to carboplatin. The second patient also had previously been treated with carboplatin and cyclophosphamide. She received 6 cycles of carboplatin and achieved a complete remission. On day 23 of Cycle-6, antibodies that bound to TPO (ODR, 1.51 which decreased to 1.05 by day 115) were detected. She had a delayed platelet recovery in Cycle-6, and coincident with that, she was noted to have a positive cell proliferation biologic assay, fifty six days after the last dose of TPO in Cycle-6 (platelet count $55.000/\text{mm}^3$). The platelet count remained stable for 4 more weeks and then recovered to $>100,000/\text{mm}^3$ by day 99. Thus, of the 7 patients with antibodies, only 2 had C-mpl activity; the other 5 patients (including 3 patients only at screening) had low titers (ODR 1.5 - 2.3) of antibodies of a transient, intermittent nature, which were non-neutralizing based on a negative C-mpl blocking as well as cell proliferation biologic assay.

Administration of rhTPO three weeks prior to chemotherapy significantly increased circulating platelet counts and bone marrow megakaryocytes. Administration of rhTPO following chemotherapy significantly reduced both the degree and duration of thrombocytopenia. In the patients (n=16) that received the optimal biologic dose of rhTPO (1.2 mcg/kg) in Cycle-2, the mean nadir platelet count was higher (44,.000 $vs.$ $20,000/\text{mm}^3$, $P<0.01$) and the duration of thrombocytopenia was shorter (days $<20,000/\text{mm}^3$, 1 $vs.$ 4 days; days $<50,000/\text{mm}^3$, 4 $vs.$ 7 days $P<0.01$) than that observed in Cycle-1 (without rhTPO). This reduced the need for platelet transfusions in this group from 75% in cycle-1 to 25% in Cycle-2 (P=0.01). Moreover, 67% of all patients treated with rhTPO recovered their platelets to $\geq100.000/\text{mm}^3$ by day 21 in Cycle-2 as compared to only 33% in Cycle-1.

## DISCUSSION

These results demonstrate that rhTPO significantly reduced both the degree and duration of severe thrombocytopenia. The need for platelet transfusion was markedly reduced, even in patients who had already experienced severe thrombocytopenia in the preceding cycles of chemotherapy. Furthermore, rhTPO enhanced the time to platelet recovery to $\geq100,000/\text{mm}^3$, maintaining the planned dose-intensity of the chemotherapy.

In this patient population, rhTPO given as a single dose prior to chemotherapy, raised circulating platelet counts in a dose-related manner. The platelet response was associated with a significant increase in the number of morphologically normal appearing bone marrow megakaryocytes. Interestingly, increases in erythroid elements were also seen in the bone marrow of several patients, consistant with the $in\ vitro$ and preclinical findings that TPO can augment erythropoiesis and with the inventor's prior clinical observation that rhTPO can expand the bone marrow erythroid and myeloid progenitor cell populations (Kobayashi $et\ al.,$ 1995; Kaushansky $et\ al.,$ 1995; Vadhan-Raj $et\ al.,$ 1997). Despite this multilineage stimulatory effect in the bone marrow, no major changes were seen in white blood cell counts or in hemoglobin levels.

In this clinical setting of a chemotherapeutic drug which routinely induces cumulative thrombocytopenia, rhTPO reduced both the depth and duration of thrombocytopenia in a dose-related manner. While no major effect was seen at the lowest dose level (0.6 mcg/kg), at the intermediate dose levels (1.2 to 2.4 mcg/kg), rhTPO raised the platelet nadir count nearly 2-fold and reduced the duration of severe thrombocytopenia by half in Cycle-2 where patients were expected to do at least as poorly as Cycle-1 without rhTPO. Since the biologic effect of rhTPO appeared to plateau at 1.2 mcg/kg, this dose was chosen for subsequent studies.

One potential concern with the dose-escalation part of the trial was that rhTPO treatment three weeks prior to chemotherapy might influence hematopoietic recovery following the first cycle of carboplatin and blunt the level of hematopoietic toxicity of the control cycle. However, the inventor's data support a lack of any positive or negative effect of TPO given 3 weeks prior to chemotherapy based on two observations. First, the platelet nadir did not increase in Cycle-1 with increasing the dose of rhTPO in the prechemotherapy cycle, suggesting a lack of positive effect of rhTPO. Second, eliminating prechemotherapy cycle of rhTPO in the dose-expansion phase also did not increase the platelet nadirs in Cycle-1, suggesting no negative effect of rhTPO. Thus, the inventor's findings indicate that TPO given 3 weeks prior to chemotherapy did not influence the platelet nadir of the control cycle and the effective dose the inventor choose for the expansion phase of the study.

To better evaluate the platelet protective effect of rhTPO, prechemotherapy rhTPO was deleted in the dose-expansion phase, and cohorts of patients were given rhTPO (1.2 mcg/kg) only as a secondary prophylaxis to prevent severe thrombocytopenia in patients who had already experienced severe thrombocytopenia in the preceding cycle of chemotherapy. Even in this compromised group of patients, rhTPO reduced the severity of the platelet nadir and shortened the duration of severe thrombocytopenia such that the need for platelet transfusions was abrogated in seven and reduced in one other, of the 11 patients who had already received platelet transfusions in the previous cycle.

The trend for a reduced need for platelet transfusions was observed throughout the study. In the patients who received rhTPO at the optimal biologic dose, the proportion of patients requiring a platelet transfusion (75% vs. 25%) as well as the number of platelet transfusion events required was markedly reduced (by 69%) from Cycle-1 to Cycle-2. Moreover, rhTPO accelerated platelet recovery such that a higher proportion of patients recovered their platelets to a level ≥1000,000/mm$^3$ by 3 weeks in Cycle-2 with rhTPO as compared to Cycle-1 without rhTPO (67% vs. 33%). By enhancing platelet recovery, rhTPO may allow delivery of chemotherapy on schedule and maintain dose-intensity.

To further assess the ability of rhTPO to allow repeated cycles of full dose chemotherapy, patients responding to antitumor therapy were allowed to continue up to six cycles of carboplatin. Despite expected cumulative hematopoietic toxicity, a proportion of patients who had required platelet transfusions in the previous cycle continue to show benefit with respect to reduced transfusion requirements in the later cycles, demonstrating the potential beneficial effect of rhTPO in overcoming cumulative thrombocytopenia. Severe neutropenia were generally not seen with this regimen. In fact, the neutrophil nadirs appeared to improve with rhTPO administration and infections remained low throughout the study.

The rationale for the design of this study was based on the inventor's clinical experience with this cytokine and this chemotherapeutic agent. Carboplatin has a delayed platelet nadir near day 16 of the cycle, and rhTPO has a prolonged half life (20 to 30 h) with a delayed peak response (median day 12) (Vadhan-Raj *et al.,* 1997; Bloedow *et al.,* 1996). Therefore, four doses of rhTPO were used every other day starting the day after chemotherapy. In addition, the inventor wished to determine whether earlier administration of rhTPO (i.e. starting 2 days prior to chemotherapy) further improved the nadir. While rhTPO showed platelet sparing effect by both the schedules, no additional benefit was noticeable by the earlier administration of the cytokine with this chemotherapeutic agent. Nevertheless, it is contemplated that with the combination chemotherapy that produce earlier platelet nadir might benefit from earlier administration of rhTPO.

In the inventor's study, the treatment with rhTPO was extremely well tolerated without any constitutional symptoms, cardiovascular symptoms, local skin reactions, and fluid retention. The results of the inventor's clinical trial have shown the effectiveness of rhTPO used as a secondary prophylaxis in carboplatin-induced severe thrombocytopenia.

In this patient population with mostly recurrent ovarian carcinoma, the objective tumor responses were seen in 54% of patients. While the importance of the platinum dose-intensity has not been established yet in these tumor types, the availability of rhTPO may now allow this question to be more safely addressed.

The results of this example demonstrate that recombinant human thrombopoietin can induce increases in platelet production, even in previously chemotherapy-treated patient populations. rhTPO administered by this route and schedule was effective in attenuating cumulative thrombocytopenia associated with carboplatin and ameliorated the need for platelet transfusions. The inventor's studies also indicate that in the high risk patients who have already experienced prior need for the platelet transfusions, the secondary prophylactic use of rhTPO can reduce the degree and duration of thrombocytopenia and prevent the need for platelet transfusions in subsequent cycles of chemotherapy. Thus in clinical settings, where maintaining dose intensity is important, secondary prophylactic use of rhTPO may allow patients to benefit from such therapy.

## EXAMPLE 11

### ENHANCED RETENTION OF *IN VITRO* FUNCTIONAL ACTIVITY OF PLATELETS FROM RECOMBINANT HUMAN THROMBOPOIETIN (rhTPO) TREATED PATIENTS FOLLOWING LONG TERM CRYOPRESERVATION WITH THROMBOSOL AND 2% DMSO

The present example demonstrates the effect of ThromboSol$^{TM}$ cryopreservation on platelets derived from patients following rhTPO treatment. Since severe thrombocytopenia in cancer patients is frequently a cumulative problem with multicycle chemotherapy, the inventor wished to determine the effect of long-term cryopreservation on the *in vitro* morphologic and functional characteristics of stored platelets. Platelets from rhTPO-treated patients (n=23) and normal donors were treated with ThromboSol$^{TM}$ and 2% DMSO, and cryopreserved for up to 6 months. The platelets were thawed at different intervals and tested for retention of functions.

### MATERIALS AND METHODS

### Patient Selection

Twenty-three patients with the diagnosis of either ovarian carcinoma (n=13) or soft tissue sarcoma (n=10) who received treatment with rhTPO as part of a phase 1-II study were examined. In patients with ovarian cancer, rhTPO was administered at doses 1.2 to 3.6 mcg/kg subcutaneously every other day for four doses following chemotherapy (carboplatin). In sarcoma patients, rhTPO was given at 1.2 mcg/kg intravenously for 3 doses; one dose a day before chemotherapy and two doses following chemotherapy (adriamycin and ifosfamide). To assess the platelet morphology and functionality of platelets (fresh as well as cryopreserved), blood samples were obtained at the time when platelets had recovered following chemotherapy and rhTPO treatment. Use of aspirin or non-steroidal anti-inflammatory agents was not allowed during the study period. In parallel, control volunteers donated blood samples under the same criteria as the patient population.

Materials

All reagents used for the storage of platelets were obtained from Sigma Chemical (St. Louis, MO). The aggregation agonists adenosine diphosphate (ADP), collagen and ristocetin were purchased from Chrono-Log Corporation (Havertown, PA). All of the reagents (amiloride, adenosine, and sodium nitroprusside) comprising the ThromboSol™ Storage Solution for cryopreservation were prepared as a 50-fold concentrate in dimethyl sulfoxide (DMSO). Phycoerythrin-labeled (PE) rabbit monoclonal anti-human platelet P-Selectin and phycoerythrin-labeled control antibodies were purchased from Becton, Dickinson (Cockeysville, MD). Phycoerythrin-labeled mouse monoclonal anti-human GP-Ib antibody was purchased from Immunotech (Cedex, France).

**Platelet Isolation and Storage**

Whole blood (70 ml) from either patients or control donors was drawn *via* venipuncture into tubes containing ACD (Becton Dickinson, Cockeysville, MD). The whole blood was held for 2-6 h at room temperature followed by centrifugation at 20°C for 12 min at 250 × g. The platelet-rich plasma (PRP) was then removed and aliquoted (20 mls/bag) into T-3101 150 ml transfer packs (CharterMed, Lakewood, NJ). An aliquot of the fresh, untreated PRP was retained for the determination of morphology and *in vitro* functional activity as described below. The 20-ml sample had the following reagent added *via* injection through a sterile port: 400 μl of a 50-fold concentrate of ThromboSol™ yielding a final DMSO concentration of 2%. The resulting ThromboSol™-treated PRP contained the following final concentrations of reagents: amiloride (0.25 mM), adenosine (0.1 mM) and sodium nitroprusside (50 μM). The treated platelets were then mixed by gentle shaking and directly inserted into a -80°C freezer in an aluminum cassette. Due to the limitations in obtainable volumes from the patients, the cryopreserved sample aliquots were randomly assigned to be stored for the indicated times.

Following storage, the platelet samples were harvested for analysis as follows: The platelet samples were thawed at 37°C in a water bath and placed on an orbital shaker set at 70 rpm for 15 min. An aliquot was removed from the bag and analyzed for cell number and mean platelet volume (MPV) using a Biochem Immunosystems System 9110 CP+ Hematology Analyzer (Allentown, PA). In addition, a freshly thawed sample was fixed for FACS analysis and evaluated for discoid morphology by light microscopy as described previously (Currie *et al.,* 1998). Fresh, air-dried smears of platelet samples were stained with Wright-Giemsa. For ultrastructural morphologic examination of freeze-thawed platelets (Vadhan-Raj *et* al., 1990), a sample was centrifuged at 950 × g for 20 min and the supernatant was removed. The sample was resuspended in 1.5 ml of fixative buffer (1.5% paraformaldehyde and 1.5% gluteraldehyde in PBS), and incubated at 4°C. The remaining platelet sample was centrifuged at 800 × g for 30 min at 22°C to remove the ThromboSol™ and the DMSO. The resulting platelet pellet was resuspended to the original volume using autologous plasma and the platelet cell number was determined as described above. The platelet sample was adjusted with autologous plasma to obtain a platelet concentration of $3 \times 10^8$ cells/ml, which was incubated at 37°C for 30 min.

**Platelet Functional Studies**

The functional assays of stirring shape change (SSC), extent of shape change (ESC), hypotonic shock response (HSR) and agonist-induced aggregation were performed as described previously (Currie *et al.,* 1998: Holme *et al.,* 1998). To determine the nature of the shape change in platelets in response to changing stirspeeds or agonist-induced activation, optical measurements were performed. SSC is an optical measurement of the discoid population present in the sample. In response to changing stir speeds, the uniform motion of a spherical platelet causes no optical deflection. In contrast, the non-uniform motion of discoid platelets causes the optical deflection. SSC was performed as follows: using an aggregometer, a platelet sample was blanked against PPP and a baseline was established. The stirrer was then turned off and the deflection from baseline was measured at its peak.

ESC is an optical measurement of the morphological discoid to spherical shape change platelets undergo in response to agonist-induced activation. For ESC, a 500μl sample was placed in an aggregometer followed by the sequential addition of 20μl of 0.1 M EDTA and 10μl of I mM ADP. The ESC was determined from the deflection from the baseline and expressed as a percentage of increase in maximal optical density (Holme et al., 1998).

To assess the overall viability and metabolism of platelets, the inventor examined the ability of platelets to respond to hypotonic stress at 15 min. For HSR, 500-$\mu$l sample was aliquoted into each of two cuvettes. 250$\mu$l of PBS was added to the first cuvette to determine the increase in transmission as a result of dilution, using an aggregometer. The second aliquot was subjected to an addition of 250$\mu$l of water and the percent recovery from hypotonic shock was determined at 15 min.

To assess whether the process of cryopreservation and thawing caused marked activation of platelets, fresh and cryopreserved platelets were examined by flow cytometric analysis for the surface expression of P-Selectin (CD-62) and surface marker GP-Ib. For FACS analysis of platelet surface markers, an aliquot of the platelet concentrate (PC) was fixed in 2% paraformaldehyde. PE-labeled, anti-P Selectin monoclonal antibody or a PE-labeled anti-GP-Ib monoclonal antibody was added to washed samples of the fixed platelets and incubated at room temperature for 1 h in the dark. A corresponding control monoclonal antibody was used in parallel to establish baseline binding. The samples were washed, diluted and then analyzed using a Coulter EPICS FACS analyzer. The presence of platelet surface markers was expressed as the percentage of cells expressing the epitope.

To determine whether cryopreserved platelets were capable of mediating functions important for hemostasis, platelet aggregation was examined using various agonists. The platelet sample was assessed for aggregation using ristocetin (25 mg/ml) or a combination of ADP (5 $\mu$M) and collagen (5 $\mu$g/ml) as the agonist. with an aggregometer (560 VS, Chrono-Log). The aggregation was determined as the percent maximum aggregation using PRP as the baseline and PPP as 100%.

### Statistical Analysis

The data is expressed as mean $\pm$ standard deviation (S.D.). The comparisons of in vitro functional activity between fresh and cryopreserved platelets from control donors and from patients who received rhTPO was done using a t-test. The comparison between patients' platelets and control platelets was performed using a t-test. The comparison between platelets stored for 1 week and platelets stored for 1 month, 3 months and 6 months was performed using a t-test.

### RESULTS

Blood samples from normal donors or rhTPO-treated patients were examined for platelet morphology and *in vitro* functional activity both prior to (fresh) and following cryopreservation (freeze-thaw) with ThromboSol™ and 2% DMSO for a short term (1 week) and for a long term (up to 6 months) storage period. The resulting morphological features and functional activity of platelets from the patient and the control groups both prior to and subsequent to short term cryopreservation with ThromboSol™ and 2% DMSO are shown in Table 26.

### Comparison of Fresh Platelet Populations

Freshly harvested platelets from the control donors and the rhTPO-treated patients were examined for the baseline cell morphology and functional activity by the series of *in vitro* assays outlined as described above and in Table 26. The platelets from both sources displayed similar characteristics in a majority of the evaluations. The platelets from the rhTPO-treated patients yielded a significantly higher cell number presumably as a consequence of the rhTPO treatment. The two platelet populations showed no significant differences in morphologic characteristics including mean platelet volume (MPV) or the percentage of platelet populations displaying discoid morphology (Table 26). The expressions of surface GP-Ib and P-Selectin were also similar for both control donor and the rhTPO-treated patient populations. The functional activities of both these platelet populations showed no significant differences in SSC, HSR, or ristocetin-induced aggregation (Table 26). However, as compared to the cells from control donors, the platelets from rhTPO-treated patients displayed significantly higher values for the ESC and lower ADP/collagen-induced aggregation (Table 26).

### Comparison of Fresh versus Cryopreserved Platelets

Following cryopreservation storage for one week at -80°C with ThromboSol™ and 2% DMSO, the platelets from rhTPO-treated patients displayed retention of morphological characteristics similar to the fresh platelets from rhTPO-treated patients (Table 26). There was no statistically significant difference in the recovery of cell number, MPV, the percentage of platelet that displayed discoid morphology ristocetin-induced aggregation or expression of the surface marker GP-Ib (Table 26). The platelets from control donors also displayed statistically similar retention of cell number, discoid morphology and the expression of GP-Ib compared to the fresh platelets from control donors (Table 26). Compared to the fresh cells from each donor populations, cryopreserved platelets from both rhTPO-treated patients and normal donors showed a statistically significant loss of functional activity including SSC, ESC, HSR and ADP/collagen-induced aggregation (Table 26). The P-Selectin expression was also significantly increased following cryopreservation storage of platelets from both these sources.

**Comparison of Cryopreserved Platelets from Normal Donors and rhTPO-Treated Patients**

Following short-term cryopreservation storage, the platelets from rhTPO-treated patients displayed statistically significantly higher retention of both morphological and functional activity parameters in comparison to platelets from normal donors (Table 26). The freeze-thawed platelets from rhTPO-treated patients maintained better retention of MPV, discoid morphology, SSC, ESC and HSR as compared to the values for platelets from normal donors (Table 26). While prior to cryopreservation, the ADP/collagen-induced aggregation was higher for control donor platelets, the two cell populations exhibited similar aggregation profiles following cryopreservation. This is due to a higher retention of agonist-induced aggregation by the platelets from rhTPO-treated patients as compared to the retention by the control platelets (73% vs. 60%, respectively versus fresh platelet values).

| TABLE 26 | | | | |
|---|---|---|---|---|
| Functional Activity of Fresh Platelets and Platelets Cryopreserved with ThromboSol and 2% DMSO: Comparison of Control Donors and TPO-Treated Patients | | | | |
| | Fresh Platelets | | Cryopreserved Platelets | |
| Morphological & Functional Assays | Control Donors | TPO-treated Patients | Control Donors | TPO-treated Patients |
| | n=20 | n=23 | n=9 | n=10 |
| Cell Number (x $10^3$/ul) | 454 ± 111 | 575 ± 235* | 448 ± 116 | 688 ± 197* |
| MPV ($u^3$) | 6.4 ± 0.6 | 6.2 ± 0.7 | 7.3 ± 0.7$^{\#}$ | 6.6 ± 0.6* |
| % Discoid | 60.7 ± 8.1 | 65.7 ± 12.3 | 57.2 ± 9.5 | 69.6 ± 6.2* |
| SSC (%) | 17.3 ± 2.7 | 18.5 ± 3.5 | 11.4 ± 1.5$^{\#}$ | 14.9 ± 2.9*$^{\#}$ |
| ESC (%) | 22.5 ± 4.3 | 25.8 ± 5.1* | 13.4 ± 2.6$^{\#}$ | 18.7 ± 3.7*$^{\#}$ |
| HSR (%) | 76.4 ± 6.6 | 82.7 ± 12.6 | 25.1 ± 10.4$^{\#}$ | 55.9 ± 14.6*$^{\#}$ |
| Ristocetin aggregation (%) | 76.2 ± 11.5 | 70.5 ± 11.7 | 55.0 ± 7.7$^{\#}$ | 61.2 ± 13.5 |
| ADP/Collagen aggregation (%) | 83.1 ± 7.7 | 74.3 ± 11.9* | 49.9 ± 17.1$^{\#}$ | 54.6 ± 14.5$^{\#}$ |
| GPIb (% expression) | 97.5 ± 1.5 | 96.6 ± 2.9 | 96.2 ± 2.0 | 96.4 ± 1.4 |
| P-Selectin (% expression) | 1.4 ± 1.6 | 3.1 ± 3.5 | 22.6 ± 14.2$^{\#}$ | 29.3 ± 12.3$^{\#}$ |

Data shown represents mean ± S.D.

*P< 0.05 for platelets from control donors as compared to platelets from TPO-treated patients.

#P<0.05 for fresh platelets as compared to cryopreserved platelets within each donor population.

78

**Long Term Cryopreservation of Platelets**

To assess the effect of long-term cryopreservation storage, platelet samples from rhTPO-treated patients and normal donors were cryopreserved using ThromboSol$^{TM}$ and 2% DMSO and stored for 1 week, 1 month, 3 months and 6 months at -80°C. Table 27 displays the results of the morphological and functional activity of platelets from rhTPO-treated patients following long-term storage. Over a 6-month storage time the platelets showed no statistically significant loss of morphology or functional activity as compared to platelets cryopreserved for 1 week (Table 27). Thus, the cryopreserved platelets from rhTPO-treated patients stored for 6 months retained cell number, percent discoid and GP-Ib expression statistically similar to fresh platelets from rhTPO-treated patients. The control donor platelets cryopreserved for extended periods displayed good retention of morphology and functional activity (Table 28). Following 1 month, 3 months and 6 months of cryopreservation storage in ThromboSol$^{TM}$ and 2% DMSO, the control donor platelets displayed no statistically significant loss of morphology or functional activity as compared to platelets cryopreserved for 1 week. The one exception was a statistical decrease in the HSR at 6 months of storage.

## TABLE 27

| Functional Activity of Platelets from rhTPO-Treated Patients Cryopreserved with ThromboSol and 2% DMSO | | | | |
|---|---|---|---|---|
| | Cryopreservation Storage Time | | | |
| Morphological & Functional Assays | | | | |
| | 1 week | 1 month | 3 months | 6 months |
| | n=10 | n=6 | n=8 | n=7 |
| Cell Number (x $10^3$/ul) | 688 ± 197 | 525.7 ± 77 | 511 ± 124 | 524 ± 201 |
| MPV ($u^3$) | 6.6 ± 0.6 | 6.7 ± 0.7 | 6.6 ± 0.6 | 7.0 ± 0.6 |
| % Discoid | 69.6 ± 6.2 | 76.5 ± 8.6 | 68.4 ± 10.2 | 62.1 ± 8.7 |
| SSC (%) | 14.9 ± 2.9 | 14.5 ± 3.5 | 12.9 ± 1.7 | 13.2 ± 2.7 |
| ESC (%) | 18.7 ± 3.7 | 17.6 ± 3.4 | 16.4 ± 3.2 | 15.4 ± 1.1 |
| HSR (%) | 55.9 ± 14.6 | 547 ± 13.5 | 44.7 ± 11.1 | 45.0 ± 13.3 |
| Ristocetin aggregation (%) | 61.2 ± 13.5 | 62.1 ± 16.9 | 50.4 ± 17.4 | 49.5 ± 12.4 |
| ADP/Collagen aggregation (%) | 54.6 ± 14.5 | 51.3 ± 12.5 | 49.4 ± 15.3 | 47.6 ± 11.4 |
| GPIb (% expression) | 96.4 ± 1.4 | 97.0 ± 1.3 | 96.4 ± 2.1 | 96.2 ± 2.1 |
| P-Selectin (% expression) | 29.3 ± 12.3 | 17.5 ± 10.0 | 24.3 ± 10.3 | 24.3 ± 10.3 |

Data shown represents mean ± S.D.

*p<0.05 for platelets stored for 1, 3 or 6 months as compared to platelets stored for 1 week.

## TABLE 28

| Functional Activity of Platelets from Control Donors Cryopreserved with ThromboSol and 2% DMSO | | | | |
|---|---|---|---|---|
| | Cryopreservation Storage Time | | | |
| Morphological & Functional Assays | | | | |
| | 1 week | 1 month | 3 months | 6 months |
| | n=9 | n=7 | n=7 | n=7 |
| Cell Number (x $10^3$/ul) | 448 ± 116 | 473 ± 106 | 483 ± 124 | 431 ± 66 |
| MPV (u$^3$) | 7.3 ± 0.7 | 7.4 ± 0.8 | 7.0 ± 0.4 | 7.4 ± 0.4 |
| % Discoid | 57.2 ± 9.5 | 57.6 ± 12.8 | 59.3 ± 10.0 | 49.6 ± 6.3 |
| SSC (%) | 11.4 ± 1.5 | 11.3 ± 1.3 | 11.9 ± 1.4 | 10.7 ± 1.7 |
| ESC (%) | 13.4 ± 2.6 | 13.2 ± 1.9 | 14.1 ± 2.7 | 12.6 ± 1.8 |
| HSR (%) | 25.1 ± 10.4 | 27.4 ± 11.6 | 23.5 ± 11.9 | 13.6 ± 8.5* |
| Ristocetin aggregation (%) | 55.0 ± 7.7 | 46.9 ± 6.7 | 55.0 ± 13.1 | 45.5 ± 18.5 |
| ADP/Collagen aggregation (%) | 49.9 ± 17.1 | 52.3 ± 14.7 | 47.9 ± 18.7 | 40.4 ± 6.4 |
| GPIb (% expression) | 96.2 ± 2.0 | 96.7 ± 1.2 | 96.8 ± 1.2 | 95.3 ± 2.5 |
| P-Selectin (% expression) | 22.6 ± 14.2 | 17.0 ± 10.7 | 22.5 ± 7.7 | 24.2 ± 13.4 |

Data shown represents mean ± S.D.

*p<0.05 for platelets stored for 1, 3 or 6 months as compared to platelets stored for 1 week.

**Morphology of Cryopreserved Platelets by Light and Electron Microscopy**

The morphology of cryopreserved platelets from rhTPO-treated patients appeared normal under light microscopy. Wright-Giemsa stained air-dried smears of fresh platelets and platelets cryopreserved with ThromboSol™ and 2% DMSO were compared. Overall, morphology appeared normal for fresh as well as cryopreserved platelets under light microscopy (1000 × magnification.)

The freeze-thawed platelets from patient samples were examined under transmission electron microscopy to assess the preservation of ultrastructural morphology following cryopreservation. Electron micrograph platelets from a rhTPO-treated patient, cryopreserved with ThromboSol™ and 2% DMSO were evaluated. Preserved platelets under low power (17,500 × magnification) showed a mixture of discoid and spherical shapes with intact membranes. Under high power (35,000 × magnification), cytoplasmic organelles including $\alpha$ granules, dense granules, glycogen particles, and the microtubular system were readily apparent. The platelets displayed a mixture of discoid and spherical shapes. These cells, for the most part, exhibited normal morphology with intact microtubular system, fuzzy coat, $\alpha$-granules and glycogen. In some platelets, the cannalicular system appeared somewhat dilated, however, the balloon forms or ghost cells were rare.

## DISCUSSION

The inventor wished to determine the effect of cryopreservation with ThrombSol and 2% DMSO on platelets derived following rhTPO treatment. The inventor's findings indicate that cryopreserved platelets from rhTPO-treated patients showed no significant loss of cell number and 100% retention discoid morphology. Moreover, the cryopreserved platelets from rhTPO-treated patients displayed 70% or greater retention of functional activity including SSC. ESC and aggregation response to various agonists as compared to the corresponding fresh cells. The inventor's findings indicate that the platelets derived from patients following rhTPO treatment can be cryopreserved with good retention of morphology and functional activity.

The cryopreserved platelets from rhTPO-treated patients showed significantly greater retention of morphology (size and discoid shape) and functional activities (SSC. ESC. HSR) compared to cryopreserved platelets from normal donors. Furthermore, there was no significant loss of morphologic and functional characteristics upon long-term storage up to 6 months as compared to platelets cryopreserved for I week. Following a short term storage (I wk), the cryopreserved platelets from rhTPO-treated patients exhibited significantly higher retention of functions including discoid morphology (70% vs. 57%), extent of shape change (19% *vs.* 13%) stirring shape change (15% *vs.* 11%) and hypotonic shock response (56% *vs.* 25%), as compared to the cryopreserved platelets from controls. There was no further significant loss of functions following cryopreservation for up to 6 months. These findings indicate that platelets from rhTPO-stimulated patients were better capable of withstanding the stress of processing, cryopreservation and the thaw procedure.

Two possible mechanisms might explain this finding. First, rhTPO enhances cytoplasmic maturation of megakaryocytes, during which there is an increase in cytoplasmic mass, granularity, formation of intracellular organelles, and acquisition of biochemical properties essential for production of functional platelets. Ultrastructural analysis on mouse bone marrow cultures grown in the presence of TPO (Zucker-Franklin and Kaushansky, 1996) as well as on human bone marrow from patients receiving rhTPO in the inventor's clinical trials has shown that TPO stimulates full megakaryocyte maturation as evidenced by the generation of platelet-specific granules, demarcation membranes and cytoplasmic fragmentation into platelets. Therefore. rhTPO-derived platelets may be more mature structurally and biochemically. Second, following TPO treatment in murine or human studies, there is an increase in newly synthesized platelets in circulation (Zucker-Franklin, 1996; Omalley *et al.*, 1996). The younger, newly synthesized platelets may perform better functionally than the older platelets. Thus, rhTPO-derived platelets may provide cells with better viability and functional integrity following cryopreservation.

The inventor's trial did not compare platelet populations, following chemotherapy, generated in the absence of rhTPO. Therefore, it is possible that similar results could be achieved with non-stimulated platelets obtained during the recovery phase of chemotherapy. Importantly thought, the use of rhTPO may further enhance the number and would increase several fold the yield of platelets that could be cryopreserved for the subsequent periods of thrombocytopenia.

In summary, cryopreservation of platelets from rhTPO-treated donors may provide a useful novel strategy for autologous or allogeneic donation for subsequent transfusions to manage treatment-related thrombocytopenia. The cryopreservation of platelets from rhTPO-stimulated donors, using ThromboSol™ and 2% DMSO, may represent a novel, useful strategy for the storage of platelets from autologous or normal donors. Clearly, this efficacy of this strategy can only be established by achieving circulating viable platelets that are capable of mediating the critical functions of hemostasis.

**EXAMPLE 12**

**A PILOT STUDY OF TRANSFUSION OF rhTPO-DERIVED AUTOLOGOUS PLATELETS CRYOPRESERVED WITH THROMBOSOL (TC) AND 2% DMSO IN PATIENTS WITH GYNECOLOGIC MALIGNANCY RECEIVING CARBOPLATIN**

This example describes methods of how to determine the safety and feasibility of transfusion of rhTPO-derived autologous platelets cryopreserved with TC and 2% DMSO, to evaluate the effects on the recovery and functional activity of the autologous transfused platelets, and how to document the tumor response to carboplatin at AUC = 11 in GYN malignancy.

The inventor have shown that administration of one or two doses of recombinant human TPO (rhTPO) in cancer patients prior to chemotherapy is a potent stimulus for prolonged platelet production. In chemotherapy-naive patients, rhTPO at 2.4 mcg/kg dose, administered as a single or two divided doses, raised platelet counts to one million ($10^6/\mu L$) or higher in several patients. The platelets produced in response to rhTPO exhibited normal morphology and aggregation functions. This finding raises the possibility that rhTPO can be given to normal donors or cancer patients for autologous donation prior to myelosuppresive therapy.

ThromboSol (TC) is a newly developed platelet storage solution which consists of selected second messenger effecters that inhibit specific activation pathways endogenous to platelets, resulting in cells that are biochemically stabilized and protected against the cold storage lesions (Connor *et al.,* 1996; Currie *et al.,* 1997). Studies have shown that ThromboSol storage solution allows for the storage of platelet concentrates (PC) at 4°C for 9 days. Furthermore, the application of ThromboSol to the cryopreservation of platelets from normal donors allowed for ease of processing, a reduction of the DMSO to 2% and excellent retention of cell number and in *vitro* functional activity.

The inventor has investigated the effect of ThromboSol cryopreservation on platelets derived from patients who received rhTPO on phase I-II clinical trials. The inventor's findings showed that platelets from patients following rhTPO treatment, when cryopreserved with TC and 2% DMSO exhibit higher recovery of functional activities, including retention of discoid morphology, and extent of shape change, hypotonic shock response, ADP/collagen induced aggregation, and a lower expression of P-selectin as compared to control platelets cryopreserved with TC and 2% DMSO or 6% DMSO alone. This finding indicates that cryopreservation of rhTPO-derived autologous or allogeneic platelets with TC and 2% DMSO may provide a useful approach for subsequent transfusions to manage treatment-related thrombocytopenia.

Carboplatin has a broad spectrum of anti-tumor activity and is used in the treatment of ovarian cancer, cervical cancer, germ cell tumors, mesothelioma, small cell lung cancer, bladder cancer, and head and neck cancer. Carboplatin is a suitable chemotherapy agent for this study because of the lack of non-hematologic toxicities at doses up to 1600 mg/m². Its major toxicity is myelosuppression, especially thrombocytopenia. Ozols *et al.* (Ozols *et al.*, 1987) treated 30 patients with recurrent ovarian cancer with high doses of carboplatin (800 mg/m²) without cytokine support. Major responses were seen in 27% of patients; myelosuppression was the most common toxicity observed. Gore *et al.* (1987) delivered doses of up to 1600 mg/m² to lung cancer and mesothelioma patients. Relatively minor gastrointestinal, necrologic, and otologic toxicities were observed; however, fairly significant myelosuppression was seen.

The inventor has completed a clinical trial in patients with recurrent gynecologic malignancy using carboplatin at AUC 11 with or without rhTPO support. The major tumor response was observed in 50% of the 25 evaluable patients (including 4 patients with CR) on this trial. However, the incidence of thrombocytopenia requiring platelet transfusions is high (10 of 12 patients treated with rhTPO as secondary prophylaxis) in cycle-1 without rhTPO.

**Study Design**

In this study, the inventor will use rhTPO prior to chemotherapy to increase platelets that will be collected, cryopreserved with TC and 2% DMSO, and thawed when needed for transfusion to manage carboplatin-induced thrombocytopenia in patients with gynecologic malignancy. Carboplatin is a commercially available chemotherapeutic agent. Twenty evaluable patients will be treated on this study. If this strategy of transfusion of rhTPO-derived autologous platelets cryopreserved with TO and 2% DMSO is found safe and effective, a formal Phase D-III protocol will be generated. Data will be collected and entered on PDMS by the research nurse assigned to the study. Deaths clearly related to treatment or unexpected life-threatening toxicities should be reported immediately to the Study Chairman, who, in turn, must notify the Surveillance Committee.

Preclinical safety studies have been conducted in normal mice, rhesus monkeys, and chimpanzees. In preclinical studies, administration of TPO results in a rapid rise in platelet counts by several fold. In the marine model of myelosuppression, recombinant murine TPO has been shown to be effective in abrogating thrombocytopenia in mice rendered pancytopenic by carboplatin and irradiation. The major safety concerns that were raised as a result of extensive preclinical studies include immunogenicity and resultant thrombocytopenia.

Recombinant human TPO (rhTPO) is currently undergoing testing in several Phase I-II clinical studies in patients receiving both myelosuppressive and myeloablative chemotherapy. Single and multiple IV doses of rhTPO have been demonstrated to be safe and without evidence of constitutional toxicities (such as fever, rigors, or hypotension). There have been two thrombotic events (deep vein thrombosis) reported that were considered to be possibly related to rhTPO in over 300 subjects treated. Anti-TPO antibodies have been observed in 10% of IV rhTPO-treated subjects; these antibodies revere transient and non-neutralizing. To date, over 25 subjects have received rhTPO by subcutaneous (SC) injection, and anti-TPO antibodies have been observed in 10% of these. Potentially neutralizing antibodies were observed in a single subject who had received multiple SC injections of rhTPO over 5 months and had experienced prolonged thrombocytopenia prior to recovery. It is unclear whether the thrombocytopenia was due to the presence of antibodies or to the cumulative effects of myelosuppressive chemotherapy.

**Clinical Pharmacokinetics**

Serum TPO concentrations have been measured after IV bolus injection of rhTPO to subjects with sarcoma or malignant neoplasm. During the pre chemotherapy cycle, the initial maximum TPO concentrations ($C_{max}$) are proportional to code, ranging from 5 to 50 ng/mL after the 0.3 and 2.4 $\mu$g/kg doses, respectively. This indicates that rhTPO appears to distribute into an initial distribution volume (60 mL/kg) similar to plasma volume. The apparent terminal elimination half-life for TPO is 20-30 h. The area under the TPO concentration-time profile (AUC) increases disproportionally with increasing dose, probably reflecting a dose-related saturation of the TPO clearance process, which is thought to occur in platelets primarily by receptor-mediated endocytosis.

**rhTPO Preparation and Administration**

rhTPO will be provided by Genentech, Inc., One DNA Way, South San Francisco, CA 94080, as a sterile liquid ready for parenteral administration. Each 3-mL glass vial of active compound contains 2 mL. rhTPO will be provided as 0.1 mg/mL in buffered solution. rhTPO will be shipped on ice and must be stored under refrigeration at 2°C-8°C (35°F-46°F). The formulation does not contain a preservative and is suitable for single-dose administration only. Exposure to bright light should be avoided. The solution should not be frozen, nor used past the expiration date stamped on the vial.

Throughout the study, clinical pharmacists or other designated study staff will prepare the rhTPO injections based on the subject's weight as measured at baseline. The clinical pharmacist will provide the study staff with syringes of rhTPO labeled with the subject ID number (and any other identifier), the protocol number and the amount of rhTPO (in micrograms and milliliters). Injections of diluted rhTPO must be administered within 4 h of preparation.

**ThromboSol Formulation**

The development of ThromboSol is based on the following principles. Platelets circulate in the body poised to respond to extrinsic signals generated at sites of tissue injury. In the absence of an external stimulus, circulating platelets are maintained in a state of tonic inhibition by factors which act synergistically via specific second messenger pathways. Their acute sensitivity to a multiplicity of both physical and biochemical stimuli has made platelets extremely difficult to maintain outside the environment of the circulatory system. This endogenous tonic inhibition of platelet activation is lost following harvesting and storage of platelets. Thus, the storage lesion may be due in part to the absence of specific modulation of these activation pathways. The approach taken in the development of the ThromboSol formulation was to mimic the endogenous inhibition of platelet activation by directly stimulating the specific second messenger pathways which prevent activation and thus render the platelets refractory to storage lesion.

In developing ThromboSol, each component has been examined along with various combinations, for the ability to reversibly inhibit the response of platelets to a series of known agonists. The reversible inhibition induced by this formulation effectively reduces the development of the platelet storage lesion when platelets are cryopreserved. The following is a brief description of the second messenger pathways manipulated in order to control activation of platelets during storage, and the specific inhibitors used to abrogate the initiation of the respective pathways. The effectiveness of each reagent was demonstrated by analyzing the ability of the second messenger effecters to reversibly block *in vitro* ADP- and collagen-induced aggregation.

*Na+/H+ Exchanger*

A functionally active $Na^+$-$H^+$ exchanger is essential for the activation of platelets due to its ability to lower the internal pH and mobilize Ca++, both of which lead to an activation event. Amiloride, which is an inhibitor of the $Na^+$-$H^+$ exchanger, effectively and reversibly blocks the in *vitro* aggregation of platelets.

*Cyclic AMP (cAMP)*

The stimulation of adenylate cyclase yields an increase in the endogenous cytopiasmic cAMP concentration, which inhibits the activation of platelets. This system of platelet inhibition is physiologically relevant in that the natural in vivo platelet effector, prostacyclin, released from endothelial cells, blocks the activation of circulating platelets by the stimulation of cAMP production. Adenosine is an alternative reagent which increases platelet cAMP levels and reversibly inhibits *in* vitro aggregation of platelets.

*Cyclic GMP (cGMP)*

Similar to the cAMP system, the stimulation of guanylate cyclase which leads to an increased cytoplasmic concentration of cGMP. inhibits the activation pathway in platelets. cGMP acts via the same pathway as the physiologically induced effects of endothelial derived relaxation factor. The reagent sodium nitroprusside (NP) which generates increased cGMP in platelets, reversibly inhibits agonist-induced aggregation.

The demonstration of the superiority of ThromboSol-treated PC versus control PC was established using a series of *in vitro* assay systems routinely employed in the platelet field to determine platelet function. The first was the recovery of cell number, in which the cell number following the freeze/thaw cycle is determined using a Hematology Analyzer and compared to the value for fresh platelets. The second was discoid morphology, in which platelets are analyzed for the retention of discoid morphology by light microscopy. 100 cells are observed using a 40x lens and the percentage of the population displaying a discoid shape is determined. The third was the extent of shape change (ESC), ESC is a measurement of the discoid to spherical shape change platelets undergo in response to agonist stimulation, and ESC is a measurement of retention of platelet activation function. The fourth was hypotonic shock response (HSR). HSR is a measurement of a platelet ability to re-establish equilibrium in response to the addition of hypotonic solution. HSR is a determination of platelet viability and is scored from 0-100%. The fifth was FACS analysis of platelet membrane surface markers. FACS is employed to determine the percent of platelets in a population which express specific platelet protein epitopes. The platelet mariners measured are GPIb which is a positive marker present on all platelets and CD62 (P-selectin) which is an activation marker. The loss of GPIb is indicative of platelet plasma membrane damage. In contrast, the surface exposure of CD62 is an indication of spontaneous activation, since its expression is the result of activation-induced degranulation.

Table 29 displays the results of these functional assays on platelets from normal donors, cryopreserved with 6% DMSO alone or ThromboSol with 2% DMSO:

Table 29 -

| Retention of *In Vitro* Functional Activator of Cryopreserved S.D.U. Platelets | | |
|---|---|---|
| | mean $\pm$ std. dev. (n=8) | |
| Functional Assay | 6% DMSO | TC/2% DMSO |
| Cell number ($\times$ 10$^3$/µL) | 1582 $\pm$ 200 | 1957 $\pm$ 235 |
| Percent Discoid | 49.8 $\pm$ 5.8 | 65.8* $\pm$ 8.7 |
| P-Selectin (% cell expressing) | 50.4 $\pm$ 10.3 | 39.6* $\pm$ 10.8 |
| ESC (% increase in O.D.) | 6.9 $\pm$ 5.4 | 13.5* $\pm$ 4.4 |
| HSR (% recovery) | 25.7 $\pm$ 8.9 | 45.9* $\pm$ 9.7 |
| ADP/Collagen Aggregation (%) | 38.8 $\pm$ 15.1 | 31.9 $\pm$ 8.7 |
| Ristocetin Aggregation (%) | 55.1 $\pm$ 16.4 | 52.6 $\pm$ 21.9 |
| Gplb Expression (%) | 92.6 $\pm$ 2.4 | 91.7 $\pm$ 1.3 |

*Statistically significant difference ($p<0.05$)

These findings indicate that the ThromboSol stabilization system yields an excellent recovery of cell number. Moreover, the retention of functional activity for the ThromboSol-treated platelets is distinctly higher than the platelets cryopreserved with 6% DMSO.

The results of the development of a second messenger formulation to abrogate the storage lesion events clearly demonstrate that ThromboSol is capable of protecting platelets under storage conditions which typically leads to damage of the cells and loss of functional activity. The development of a ThromboSol solution for cryopreservation will allow the storage of platelets at -80°C, a temperature that is easily maintained with conventional freezers. Furthermore, the reduction in DMSO concentration or the replacement of DMSO with alternative, nontoxic cryoprotectants that are already approved for human use may allow for the direct transfusion of the stored units, thus eliminating the wash step. The abolition of the wash procedure would allow for extended storage following the thaw process. The results of the extended post-thaw storage would be the ability to store and ship cryopreserved units. The ability to provide long-term storage of platelets, with a post-thaw population of cells displaying characteristics similar to fresh platelets, would solve many of the blood banking inventory supply problems.

### Patient Inclusion Criteria

To be included in the study, the patients must meet the following criteria. Patients' should have gynecologic malignancy for whom treatment with carboplatin is indicated. The patients' age $\geq$ 15 years (for ages 13 to less than 15, at the discretion of the inventor). Adequate hematologic (ANC $\geq$1500/mm$^3$, platelet count $\geq$150,000/mm$^3$ and Hb $\geq$ 10 gm/dl), renal (serum Cr $\geq$ 1.5), and hepatic functions (total bilirubin $\geq$1.5, SGPT or SCOT $\geq$3 $\times$ normal) must be indicated. Life expectancy should be $\geq$ 3 months and Kamofsky Performance Status should be $\geq$ 80. The patients should have signed an informed consent form. The patients' should be non-reactive to FDA required blood donor infectious disease testing.

### Patient Exclusion Criteria

Patients should be exclude for the following reasons. The patients have rapidly progressive disease. The patients are pregnant or lactating women. The patients have a comorbid condition which renders patients at high risk of treatment complication. The patients' have a positive history of CNS metastasis. The patients have significant cardiac disease (NYHA Class III or IV) or dysarrhythmia, or recent history of MI or ischemia, transient ischemic attack or CVA within the 6 months of study entry. The patients have taken prior chemotherapy, immunotherapy, any experimental drug within 4 wk, use of myeloid (G-CSF or GM-CSF) growth factors within 2 wk or erythropoietin within 4 wk. The patients have used any nitrosourea (BCNU, CCNU) or mitomycin -C within 6 wk. The patients have experienced a prior surged or RT within 2 wk of study entry. The patients have a prior history of pelvic irradiation. The patients have a history of prior high dose chemotherapy with stem cell transplant or a history of prolonged thrombocytopenia ($\geq$ 2 wk). The patients have a history of leukemia. The patients have a history of any platelet disorders including ITP, TTP or bleeding disorders. The patients have a history of > 3 prior chemotherapy regimens (all platinum regimens will be counted as 1 regimen). The patients have demonstrated lack of response to platinum-based therapy.

### Treatment Plan

All patients will be registered in the patient data management system (PDMS). Patients will be randomized to group A (n = 10) or group B (n = 10).

### *Prechemotherapy Phase:*

All patients will receive rhTPO in prechemotherapy phase apheresis of platelets as follows: First, rhTPO will be administered at 1.2 mcg/kg i.v. $\times$ 2 doses (days 1 and 4). Second, plateletpheresis will be due starting from day 12 or when platelet count reached a level $\geq$750,000/mm$^3$ (whichever comes first). The pheresis can be repeated for up to a maximum of four days to collect 250 units of platelets (~30 $\times$ 10$^{11}$ platelets). The sterile ThromboSol/DMSO solution is added to the platelet concentrate and mixed to ensure a homogenous distribution of the ThromboSol/DMSO into platelets. The final DMSO concentration will be 2% by volume. Third, the platelets in the ThromboSol storage solution will be split into 5 platelet storage bags, each with approximately 6 $\times$ 10$^{11}$ platelets. The platelet bags will be placed in freezing cassettes and placed directly at -80°C in a standard freezer.

Fourth. when needed for transfusion. the freezing cassette will be removed from the freezer and the treated platelet unit will be placed directly into a 37°C water bath until the unit is completely thawed. The thawed platelet sample will be placed on a shaker and rotated at 22°C for 30 min to allow for recovery. Fifth, the platelet unit will be washed by centrifugation at 950 $\times$ g for 20 min to remove ThromboSol and DMSO. The platelets will be resuspended in autologous plasma. Sixth, a 2 ml aliquot will be removed for in *vitro* testing. This platelet sample will be tested for retention of functional activity as described (percent discoid morphology, ESC, HSR). These tests will be performed at LifeCell Corporation. Seventh, in select patients (for the first autologous transfusion event), an aliquot of the ThromboSol-treated cryopreserved platelets will be radiolabeled with [111] In according to standard techniques. Following labeling, platelet samples will be washed twice *via* centrifugation with the Tyrodes (pH=7.2) buffer and finally resuspended to the original volume with autologous plasma. The specific activity of the radiolabeled platelets will be determined for the sample. A final platelet sample will contain 30 uCi of [111]In. The radiolabeled sample of platelets will be mixed with the non-labeled platelets. Gentle agitation will be applied to allow for complete mixing of two platelet populations.

Eighth, the platelets will be reinfused to the patient. Nineth, following infusion, in patients who are participating in platelet survival study, a 10 ml blood sample will be drawn into a vacutainer tube at 2 and 24 h post infusion, and 2, 4, 6, and 10 day post transfusion. A sample of the infused platelet population will be retained to analyze *via* gamma counting for the retention of radiolabeled platelets. An aliquot of the whole blood will be counted, and a sample will be centrifuged at $400 \times$ g for 12 min to generate a platelet rich plasma fraction. The PRP will be measured for radioactivity of the isotope in the platelet fraction. Finally a platelet poor plasma fraction will be generated by centrifugation at 950 $\times$ g for 22 min and the total $^{111}$In remaining in the plasma will be determined. The initial clearance rates and the half-life of circulation will be determined by comparison of the injected dose to the radiolabeled platelets obtained at the various time-points. The calculation of post-transfusion percent recoveries and survivals will be performed using multiple hit analysis after correction for plasma activity as described previously. Using computer programs, the average numerical life span and the residual life span will be calculated. Tenth, post transfusion platelet counts will be done at 10 min - I h and 18 - 24 h time points.

**Chemotherapy Cycles**

All patients will receive carboplatin at a dose calculated using Calvert's formula with AUC of 11. The cycles of chemotherapy will be repeated every 3 wk upon recovery of blood counts. The first cycle of chemotherapy will start 3 wk from the first dose of rhTPO.

**Platelet Transfusions**

All patients will be transfused with platelets when nadir platelet count is < 15,000/mm$^3$ as follows:

Group A:

    Cycle-1: Allogeneic single donor platelets
    Cycle-2: Autologous cryopreserved platelets

Group B:

    Cycle-1: Autologous cryopreserved platelets
    Cycle-2: Allogeneic single donor platelets

Every effort will be made to transfuse allogeneic single donor platelets in Cycle-1 (group A) and Cycle-2 (group B). However, when single donor platelets are not available, approximately equivalent units of random donor platelets (10 units) will be transfused. The autologous platelets, when used (Cycle-2 for group A and Cycle-1 for group B), will also contain approximately equivalent number of platelets (10 units).

All patients with at least a stable or responding disease may continue up to a total of 6 cycles of carboplatin. During cycles 3-6, all patients (group A and B) will be transfused autologous cryopreserved platelets during thrombocytopenia, as long as they are available.

**Pretreatment Evaluation**

Patients will have complete history and physical examination with tumor measurements. Laboratory studies will include CBC with differential and platelet counts, serum chemistry, serum pregnancy test (if applicable), urinalysis, anti-TPO antibody level, Blood bank screening tests for infectious diseases. Chest x-ray, EKG, and other radiologic studies for tumor measurements will be taken.

**Evaluation During Study**

Subjects will be closely monitored for safety, physical findings and tumor response while on the study. Subjects will be assessed for adverse events at each visit during the study and follow up period. Vital signs will be measured at each visit as well as before and after each rhTPO administration. In addition, laboratory evaluation will be done as follows. First, CBC differential and platelet counts will be monitored at least three times a week. In addition, daily platelet counts will be done when platelet counts are <50,000/mm$^3$. Second, following platelet transfusion, 1 h and 24 h post-transfusion platelet count will be checked. Third, serum chemistry and anti-TPO antibody will be checked prior to each cycle of chemotherapy. Fourth, in 5 patients willing to participate for platelet survival study, blood will be drawn and described. Fifth, in select patients, platelet functions (ex vivo) will be tested 24 h after the transfusion of allogeneic and autologous platelets.

**Criteria for Evaluation**

*Safety Evaluation*

Patients will be closely monitored for safety evaluation as described above. Patients will be assessed for adverse events related to the treatment as well as platelet transfusions at each visit during the study period as well as follow up period.

*Efficacy Transfusion*

The primary measure of efficacy of platelet transfusions would be the increment in platelet count at 1 h and 24 h post transfusion as measured by corrected count increment (CCI) according to the following formula:

$$CCI = \frac{(Post\ Tx\ count - Pre\ Tx\ count)\ BSA}{No.\ of\ Plt\ txed \times 10^{11}}$$

In select patients (~5 patients) $^{111}$In labeled platelet survival studies will be done following autologous as well as allogeneic platelet transfusion and post transfusion percent recoveries and survivals will be calculated as described above. In select patients (~5 patients) template bleeding time will be done post transfusion of autologous and allogenic platelets to assess the correction of bleeding time abnormality associated with low platelet counts.

*Tumor Response*

Standard response criteria for gynecologic malignancy will be used.

**Criteria for Discontinuing Therapy**

Standard response criteria for gynecologic malignancy will be used. The therapy will be discontinued if there is progressive disease after an initial response or failure to minimum of two courses of therapy, unacceptable toxicity defined as unpredictable, irreversible, or grade 4 non-hematologic toxicity, non-compliance by patient with protocol requirements, or the patient refuses to continue the protocol.

**Statistical Considerations**

This is a pilot clinical study to evaluate the safety and feasibility of transfusing rhTPO-stimulated autologous platelets cryopreserved with ThromboSol and 2% DMSO. Given the pilot nature of the study and small sample size, simple descriptive statistics will be prepared at the trials conclusion. The morphology, cell recovery, and the functional activity of freeze-thawed platelets will be assessed. The platelet increments (1 h and 24 h post transfusion) observed with cryopreserved autologous platelets and fresh allogeneic single donor platelets will be tabulated by the cycle number. Similarly, the other measures of platelet survival (i.e. $^{111}$In labeled platelet studies) and platelet function (*i.e.* correction of template bleeding time) will also be tabulated by cycle using autologous cryopreserved and allogeneic fresh platelets. The data generated from this trial would help design future formal phase II-III trial, if this strategy is found safe and effective.

## EXAMPLE 13

### CRYOPRESERVATION OF PLATELETS FROM RECOMBINANT HUMAN THROMBOPOIETIN (rhTPO)-TREATED DONORS USING THROMBOSOL™ AND 2% DMSO.

### Study Design

Platelets were isolated from rhTPO-treated patients following chemotherapy. ThromboSol and DMSO (2% final) were added to the PRP and platelets were frozen in a -80°C freezer. The platelets were thawed after 48 hr and assayed for in vitro functional activity.

Results

Table 30 displays the functional parameters of platelets cryopreserved with ThromboSol and 2% DMSO (mean +/- standard deviation).

Table 30

| Assay (n=9) | Fresh | Cryopreserved |
|---|---|---|
| Cell# (% of fresh) | 100 | 98 |
| MPV(u$^3$) | 6.1+/-1.0 | 6.8 +/-0.7 |
| % discoid | 69.6 +- 6.3 | 67.9 +- 4.9 |
| Extent of shape change (%) | 26.9 +/- 5.7 | 18.6 +/- 3.7 |
| Hypotonic shock response (%) | 75.9 +/- 16.5 | 54.0 +/- 11.6 |
| Ristocetin aggregation (%) | 75.6 +/- 10.7 | 61.1 +/- 15.3 |
| ADP/Collagen aggregation (%) | 80.7 +/- 14.6 | 54.3 +/- 13.3 |
| GPIb (% expression) | 97.3 +/- 1.1 | 96.3 +/- 1.4 |
| CD62 (% expression) | 6.6 +/- 4.4 | 28.7 +/- 12.0 |

### Conclusions.

rhTPO-stimulated platelets cryopreserved with ThromboSol and 2% DMSO maintain excellent cell number and functional activity and may represent a viable method for the storage of autologous platelets.

## EXAMPLE 14

### ENHANCED RETENTION OF IN VITRO FUNCTIONAL ACTIVITY OF RHTPO-INDUCED PLATELETS FOLLOWING CRYOPRESERVATION WITH THROMBOSOL™ AND 2% DMSO.

The ability to cryopreserve rhTPO-stimulated platelets would enable the use of autologous platelets during post-chemotherapy thrombocytopenia. Unfortunately, the currently approved methods for platelet cryopreservation, suing 5% or 6% DMSO, are labor-intensive and result in a loss of cell number and functional activity. ThromboSol is a platelet stabilizing solution composed of second messenger effectors that inhibit specific activation pathways endogenous to platelets. Previous data has demonstrated that the application of ThromboSol to the cryopreservation of platelets allowed for ease of processing, a reduction of the DMSO to 2% and excellent post-thaw retention of cell number and functional activity. This study investigates the effect of ThromboSol cryopreservation on platelets derived from patients following rhTPO treatment. PRP isolated from rhTPO-treated patients or control volunteers was tested for platelet functional activity in vitro. The samples were then treated with ThromboSol and 2% DMSO and placed directly into a -80°C freezer. Following storage, the platelets were thawed and tested for retention of platelet functional activity in vitro. Table 31 displays the results obtained from platelets from 10 post-chemotherapy TPO-treated patents and from healthy volunteers prior and subsequent to cryopreservation.

Table 31

| Comparison of Functional Activity of Platelets +/- rhTPO Treatment (mean) | | | | |
|---|---|---|---|---|
| | Fresh | | Cryopreserved | |
| Assay | Control | rhTPO | Control | rhTPO |
| Cell number (x10³/ul) | 443 | 723 | 424 | 687 |
| MPV (u³) | 6.5 | 6.2 | 7.1 | 6.7 |
| % discoid | 56.3 | 70.6 | 50.0 | 67.4 |
| Stirring shape change (%) | 18.7 | 19.2 | 11.8 | 14.5 |
| Extent of shape change (%) | 24.9 | 28.0 | 14.9 | 18.3 |
| Hypotonic shock response (%) | 78.0 | 80.6 | 37.4 | 54.6 |
| Ristocetin aggregation (%) | 71.6 | 75.1 | 58.1 | 59.0 |
| ADP/collagen aggregation (%) | 83.5 | 79.0 | 47.2 | 52.7 |
| GPlb (% expression) | 96.5 | 97.6 | 96.5 | 96.6 |
| CD62 (% expression) | 0.6 | 4.5 | 39.9 | 29.9 |

Both the rhTPO-induced and the control platelets showed similar functional activity in vitro when assayed as fresh cells. Following cryopreservation with ThromboSol and 2% DMSO, the platelets from rhTPO-treated patients showed higher retention of many in vitro functional activities and appeared overall morphologically normal by light and ultrastructural morphology. Furthermore, preliminary data indicates that the rhTPO-stimulated platelets maintain this high level in vitro functional activity following 1 month and 3 months of cryopreserved storage. Use of ThromboSol and 2% DMSO, therefore represents a potentially useful method to cryopreserve autologous rhTPO-induced platelets for post-chemotherapy transfusion.

All of the methods disclosed and claimed herein can be made and executed without undue experimentation in light of the present disclosure.

## REFERENCES

The following references, to the extent that they provide exemplary procedural or other details supplementary to those set forth herein, are specifically incorporated herein by reference.

U.S. Patent No. 5,851,984
U.S. Patent No. 5,851,451
U.S. Patent No. 5,837,693
U.S. Patent No. 5,837,544
U.S. Patent No. 5,830,682
U.S. Patent No. 5,830,647
U.S. Patent No. 5,814,517
U.S. Patent No. 5,795,569
U.S. Patent No. 5,792,850
U.S. Patent No. 5,766,897
U.S. Patent No. 5,766,581
U.S. Patent No. 5,756,083
U.S. Patent No. 5,753,462
U.S. Patent No. 5,744,587
U.S. Patent No. 5,741,899
U.S. Patent No. 5,733,746
U.S. Patent No. 5,712,094
U.S. Patent No. 5,707,803
U.S. Patent No. 5,696,250
U.S. Patent No. 5,648,219
U.S. Patent No. 5,641,655
U.S. Patent No. 5,593,666

U.S. Patent No. 5,571,686
U.S. Patent No. 5,384,331
U.S. Patent No. 5,310,550
U.S. Patent No. 5.260.417
U.S. Patent No. 5,250,732
U.S. Patent No. 5,155,211
U.S. Patent No. 5,126,325
U.S. Patent No. 4,894.440

Alvarez, Gates, Brady, "Complications from intra-aortic balloon counterpulsation: a review of 303 cardiac surgical patients," *European Journal of Cardiothorac Surgery,* 6:530-535. 1992.

Alvarez, Rogge, Tarrand, Lichtiger, "Bacterial contamination of cellular components. A retrospective review at a large cancer center," *Ann. Clin. Lab. Sci.,* 25:283-290, 1995.

American Society of Clinical Oncology, "Recommendations for the use of hematopoietic colony-stimulating factors: evidence-based, clinical practice guidelines," *J. Clin. Oncol.,* 12:2471-2508, 1994.

Balduini, Mazzucco, Sinigaglia. Grignani. Bertolino, Noris, Pacchiarini, Torti, Salvaneschi, "Cryopreservation of human platelets using dimethyl sulfoxide and glycerol-glucose: Effects on "*in vitro*" platelet function," *Haematologica,* 78:101-106, 1993.

Ballem, Segal, Stratton, Gemsheimer, Adamson, Slichter, "Mechanisms of thrombocytopenia in, chronic autoimmune thrombocytopenic purpura." *J. Clin. Invest.,* 80:33 40, 1987.

Bartley, Bogenberger, Hunt, Li. Lu. Martin *et al.*, "Identification and cloning of a megakaryocyte growth and development factor that is a ligand for the cytokine receptor, Mpl. *Cell.,* 77:1117-1124, 1994.

Bartley, Bogenberger, Hunt, Li, Lu. Martin *et al.*, "Identification and cloning of a megakaryocyte growth and development factor that is a ligand for the cytokine receptor Mpl," *Cell*, 77:1117-24,1994.

Bartley, Bogenberger, Hunt, Li, Lu, Martin, "Identification and cloning of a megakaryocyte growth and development factor that is a ligand for the cytokine receptor Mpl," *Cell*, 77:1117-1124, 1994.

Basser, Rasko, Clarke *et al.*, "Randomized, blinded, placebo-controlled phase I trial of pegylated recombinant human megakaryocyte growth and development factor with filgrastim after dose-intensive chemotherapy in patients with advanced cancer," *Blood,* 89(9):31 18-3128, 1997.

Bensinger, Singer, Appelbaum, Lilleby, Longin, Rowley *et al.*, "Autologous transplantation with perioheral blood mononuclear cells collected after administration of recombinant granulocyte stimulatng factor," *Blood,* 81: 3158-3163, 1993.

Biesma, Willemse, Mulder, Sleijfr, Gietma, Mull *et al.*, "Effects of interleukin-3 after chemotherapy for advanced ovarian cancer." *Blood.* 80:1141-1148, 1992.

Bloedow, Vadhan-Raj, Paton *et al.*, "Pharmacokinetics of recombinant human thrombopoietin (rhTPO) after intravenous administration in cancer patients," ASH Abstract, 1996.

Bock. Schleuning, Heim, Mempel, "Cryopreservation of human platelets with dimethyl sulfoxide: changes in biochemistry and cell function," *Transfusion,* 35:921-924, 1995.

Broxmeyer, Cooper, Vadhan-Raj, "Cell cycle status of erythroid (BFU-E), progenitor cells from the bone marrows of patients on a clincial trial with purified recombinant human granulocyte-macrophage colony-stimulating factor," *Exp. Hematol.,* 17:455-459, 1989.

Bruno, Briddell, Hoffman, "Effect of recombinant and purified hematopoietic growth factors on human megakaryocyte colony formation," *Exp. Hematol.*, 16:371-377, 1988.

Calvert. Lind, Ghazal-Aswad *et al.*, "Carboplatin and granulocyte colony-stimulating factor as first-line treatment for epithelial ovarian cancer: A phase I dose-intensity escalation study," *Seminars in Oncology,* 21(5) Suppl 12: 1-6, 1994.

Combination Cancer Chemotherapy Regimens, Roger W. Anderson and William J. Dana, Eds., Laderley Laboratories (1991) and

Connor, Currie, Allan, Livesey, "Recovery of *in vitro* functional activity of platelet concentrates stored at 4°C and treated with second-messenger effectors," *Transfusion,* 36:691-698, 1996.

Crawford, Kreisman, Garewal, Jones, Shoemaker, Pupa *et al.*, "A pharmacodynamic investigation of recombinant human granulocyte colony stimulating factor (r-metHuG-CSF) schedule variation in patients with small cell lung cancer (SCLC) given CAE chemotherapy," *Proc. Am. Soc. Clin. Oncol.,* 11;299, 1992.

Crawford, Ozer, Stoller *et al.*, "Reduction by granulocyte colony-stimulating factor of fever and neutropenia induced by chemotherapy in patients with small-cell lung cancer (r-metHuG-CSF)," *N. Engl. J. Med.*, 325:164-170, 1991.

Crawford, Ozer, Stoller, Johnson, Lyman. Tabbara *et al.*, "Reduction by granulocyte colony-stimulating factor of fever and neutropenia induced by chemotherapy in patients with small-cell lung cancer," *N. Engl. J. Med.*, 325: 164-170, 1991.

Currie. Harper, Allan. Connor. "Inhibition of cytokine accumulation and bacterial growth during storage of platelet concentrates at 4°C with retention of *in vitro* functional activity," *Transfusion,* 37:18-24, 1997.

Currie, Livesey, Harper, Connor, "Cryopreservation of single donor platelets with a reduced dimethyl sulfoxide concentration by the addition of second messenger effectors: Enhanced retention of *in vitro* functional activity," *Transfusion,* 38:160-167, 1998.

Cwirla *et al.*, "Peptide Agonist of the thrombopoietin receptor as potent as the natural cytokine," Science, 276: 1696-1699, 1997.

D'Hondt, Weynants, Humblet *et al.,* "Dose-dependent interleukin-3 stimulation of thrombopoiesis and neutropoiesis in patients with small-cell lung carcinoma before and following chemotherapy: a placebo-controlled randomized phase II study," *J. Clin. Oncol.,* 11:2063-2071, 1993.

Daly, Schiffer, Aisner, Wiernik. "Successful transfusion of platelets cryopreserved for more than 3 years," *Blood,* 54:1023-1027, 1979.

de Sauvage, Hass, Spencer, Malloy, Gurney Spencer *et al.*, "Stimulation of megakaryocytopoiesis and thrombopoiesis by the c-Mpl ligand," *Nature,* 369:533-8, 1994.

de Sauvage, Hass, Spencer, Malloy, Gurney, Spencer *et al.*, "Stimulation of megakaryocytopoiesis and thrombopoiesis by the c-Mpl ligand, *Nature,* 369:533-538, 1994.

de Sauvage, Hass, Spencer, Malloy, Gurneyh, Spencer, "Stimulation of megakaryocytopoiesis and thrombopoiesis by the c-MPL ligand," *Nature,* 369:533-538, 1994.

Djerassi, Farber, Roy, Cavins, "Preparation and *in vivo* circulation of human platelets preserved with combined dimethyl-sulfoxide and dextrose," *Transfusion,* 6:572-576, 1966.

Emmons, Reid, Cohen, Meng, Young, Dunbar *et al.*, "Human thrombopoietin levels are hgh when thrombocytopenia is due to megakaryocyte deficiency and low when due to increased platelet destruction," *Blood,* 87: 4068-4071, 1996.

Fanucchi, Glaspy, Crawford *et al.,* "Effects of polyethylene glycol-conjugated recombinant human megakaryocyte growth and development factor on platelet counts after chemotherapy for lung cancer," *N. Engl. J. Med.*, 336: 404-409, 1997.

Fanucchi, Glaspy, Crawford, Garst, Figlin, Sheridan *et al.*, Efects of polyethylene glycol-conjugated recombinant human megakaryocyte growth and development factor on platelet counts after chemotherapy for lung cancer," *N. Engl. J. Med.,* 336:404409, 1997.

Farese, Hunt, Boone, Mac Vittie, "Recombinant human megakaryocyte growth and development factor stimulates thrombocytopoiesis in normal nonhuman primates." *Blood,* 86:54-59, 1995.

Farese. Hunt. boone, MacVittle, "Recombinant human megakaryocyte growth and development factor thrombocytopoiesis in normal nonhuman primates, *Blood,* 86:54-59, 1995.

Fielder, Gurney, Stefanich, Marian, Moore, Carver-Moore et al., "Regulation of thrombopoietin levels by c-mpl-mediated binding to platelets," *Blood,* 87:2154-2161, 1996.

Gore, Calvert, Smith, "High dose carboplatin in the treatment of lung cancer and mesothelioma. A Phase I dose escalation study," *Eur. J. Cancer*, 23:1391-7, 1987.

Gore. Calvert, Smith, "High dose carboplatin in the treatment of lung cancer and mesothelioma: A phase I dose escalation study," *Eur. J. Cancer*, 23:1391-1397, 1987.

Graylee, Arora, Lavender, "Predictive value of blood clotting tests in cardiac surgical patients." *Ann Thor Sur,* 58: 216-221, 1994.

Gruney, Wong, Henzel, de Sauvage, "Distinct regions of the c-Mpl cytoplasmic domain are coupled to the JAK-STAT signal transduction pathway and Shc phosphorylation," *Proc. Natl. Acad. Sci. USA,* 92:5292-6, 1995.

Hammond, Csiba. Canin, Hockman, Souza, Layton *et al.*, "Chronic neutropenia. A new canine model induced by human granulocyte colony-stimulating factor." *J. Clin. Invest.,* 87:704-10, 1991.

Hayman and Schiffer, "Platelet transfusion therapy for the cancer patient," *Semin. Oncol.*, 17:198-209, 1990.

Hayman and Schifler, "Platelet transfusion therapy for the cancer patient," *Semin. Oncol.,* 17:198-209,1990.

Hobson and Fuller, "Species selection for safety evaluation of biotechnology products." *Prog. Clin. Biol. Res.*, *235: 55-71,* 1987.

Holme, Moroff, Murphy *et al.* "A multi-laboratory evaluation of *in vitro* platelet assays: the tests for extent of shape change and response to hypotonic shock," *Transfusion,* 38:31-40, 1998.

Kaushansky, "Thrombopoietin: the primary regulator of platelet production," *Blood,* 86:419-431, 1995.

Kaushansky, "Thrombopoietin: the primary regulator of platelet production," *Blood,* 86:419-431, 1995.

Kaushansky, Broudy, Grossmann *et al.*, "Thrombopoietin expands erythroid progenitors, increases red cell production, and enhances erythroid recovery after myelosuppressive therapy," *J. Clin. Invest.*, 96:1683-1687, 1995.

Kaushansky, Lok, Holly, Broudy, Lin, Bailey *et al.*, "Promotion of megakaryocyte progenitor expansion and differentiation by the c-Mpl ligand thrombopoietin," *Nature,* 369:568 71, 1994.

Kaushansky, Lok, Holly, Broudy, Lin, Bailey *et al.*, "Promotion of megakaryocyte progenitor expansion and differentiation by the c-Mpl ligand thrombopoietin," *Nature*, 369:568-571, 1994.

Keleman, Cserhati, Tanos, "Demonstration and some properties of human thrombopoietin in thrombocythemic sera," *Acts Haematol (Basel)*, 20:350-355, 1958.

Kobayashi, Laver, Kato, Miyazaki, Ogawa, "Recombinant human thrombopoietin (Mpl Ligand) enhances proliferation of erythroid progenitors," *Blood,* 86(7):2494-2499, 1995.

Korbling, Huh, Durrett, Mirza, Miller, Engel *et al.*, "Allogeneic blood stem cell transplantation: peripheralization and yield of donor-derived primitive hematopoietic progenitor cells (CD34+ Thy-1dim) and lymphoid subsets, and possible predictors of engraftment and graft-vs-host disease," *Blood,* 86:2442-2848, 1995.

Krishan, "Rapid flow cytofluorometric analysis of mammalian cell cycle by propidium iodide staining," *J. Cell Biol.*, 66:188-193, 1975.

Kuter and Rosenberg, "Appearance of a megakaryocyte growth-promoting activity, megapoietin, during acute thrombocytopenia in the rabbit," *Blood,* 84:1464-72, 1994.

Lazarus. Herzig, Warm. Fishman. "Transfusion experience with platelet concentrates stored for 24 to 72 hours at 22°C," *Transfusion*, 22(1):39, 1982.

Lazarus, Kaniecki-Green, Warm, Aikawa, Herzig, "Therapeutic effectiveness of frozen platelet concentrates for transfusion," *Blood,* 57:243-249, 1981.

Levin, "Thrombopoietin-clinically realized," *N. Engl. J. Med.*, 336:434-436, 1997.

Lok, Kaushansky, Holly, Kuijper, Lofton-Day, Oort *et al.*, "Cloning and expression of murine thrombopoietin cDNA and stimulation of platelet production *in vivo*," *Nature,* 369:565-8, 1994.

Lok, Kaushansky, Holly, Kuijper, Lofton-Day, Oort *et al.*, "Coning and expression of murine thrombopoietin cDNA and stimulation of platelet production *in vivo*," *Nature,* 369:565-568, 1994.

Lok, Kaushansky, Holly, Kuijper, Lofton-Day, Oort, "Cloning and expression of murine thrombopoietin cDNA and stimulation of platelet production *in vivo*," *Nature,* 369:565-568, 1994.

Murphy, "Platelet storage for transfusion," *Seminars in Hematology,* 22:165-177, 1985.

Murray, Luen, Bruno, Estrada, Cohen, Hellman *et al.,* Effects of thrombopoietin on megakaryocytes and progenitor cell propulation in bone marrow and pripheral blood of sarcoma patients [Abstract], *Blood*, 88(Supp 1-2):351a, 1996.

Murray, Mandich, Bruno, DiGusto, Fu, Sutherland *et al.*, "Fetal bone marrow CD34+ CD41 + cells are enriched for multipotent hematopoietic progenitors, but not or pluripotent stem cells," *Exp. Hematol.*, 24:236-245, 1996.

Neidhart, Mangalik, Stidley, tebich, Sarmiento, Pfile *et al.*, "Dosing regimen of granulocyte-macrophage colony-stimulating factor to support dose-intensive chemotherapy," *J. Clin. Oncol.*, 10:1460-1469, 1992.

Nemunaitis, Rabinowe, Singer, Bierman, Vose, Freedman *et al.*, "Recombinant granulocyte-macrophage colony-stimulating factor after autologous bone marrow transplanation for lymphoid cancer," *N. Engl. J. Med.*, 324: 1773-1778, 1991.

Neumunaitis, Rabinowe, Singer "Recombinant granulocyte macrophage colony-stimulating factor after autologous bone marrow transplantation for lymphoid cancer," *N. Engl. J. Med.,* 324:1773-1778, 1991.

O'Malley. Rasko. Basser, McGrath. Cebon. Grigg, Hopkins. Cohen, O'Byrne. Green. Fox, Berndt, Begley, "Administration of pegylated recombinant human megakaryocyte growth and development factor to humans stimulates the production of functional platelets that show no evidence of *in vivo* activation," Blood, 88:3288-3298, 1996.

Owens, Holme, Heaton, Sawyer, Cardinali, "Post-transfusion recovery of function of 5-day stored platelet concentrates," *Br. J. Haematol.*, 80:539, 1992.

Ozols, Ostchega, Curt, Young, "High-dose carboplatin in refractory ovarian cancer patients," *J. Clin. Oncol., 5: 197-201,* 1987.

Ozols, Ostchega, Curt, Young, "High dose carboplatin in refractory ovarian cancer patients," *J. Clin. Oncol.,* 5: 197-201, 1987.

Pirisi, Fabris, Soardo, Cecchin, Toniutto, Bartoli, "Thrombocytopenia of chronic liver disease corrected by erythropoietin treatment," *J. Hepatol.*, 21:376 80, 1994.

Rodak, "Diagnostic Hematology, Saunders (ed), Phil. PA, 554-558, 1995.

Schiffer, "Prevention of alloimmunization against platelets," *Blood,* 77:1-4, 1997.

Schiffer, Aisner, Wiernik, "Clinical experience with transfusion of cryopreserved platelets," *British Journal of Haematology,* 34:377-385, 1976.

Schiffer, Aisner, Wiernik, "Frozen autologous platelet transfusion for patients with leukemia," *The New England Journal of Medicine,* 299:7-11, 1978.

Schiffer, Buchholz, Wiernik, "Intensive multiunit plateletpheresis of normal donors," *Transfusion*, 14:388-394, 1974.

Shea, Mason, Stomiolo, Newton, Breslin, Mullen *et al.*, "Sequential cycles of high-dose carboplatin administered with recombinant human granulocyte-macrophage colony-stimulating factor and repeated infusions of autologous peripheral-blood progenitor cells: a novel and effective method for delivering multiple courses of dose-intensive therapy," *J. Clin. Oncol.*, 10:464-473, 1992.

Slichter. "Platelet transfusion therapy," *Hematol. Oncol. Clin. North Am.*, 4:291-311. 1990.

Smith. Longo, Alvord, Janik, Sharfman, Gause *et al.,* "The effects of treatment with interleukin-1α on platelet recovery after high-dose carboplatin," *N. Engl. J. Med.,* 328:756-761, 1993.

Smith. Urba, Curti *et al.*: The toxic and hematologic effects of interleukin-1 alpha administered in a phase I trial to patients with advanced malignancies. J Clin Oncol 10:1141-1152,1992

Socinski, Cannistra, Elias, Antman, Schnipper, Griffin *et al.*, "Granulocytemacrophage colony-stimulating factor expands the circulating haemopoietic circulating haemopoietic progenitor cell compartments in man," *Lancet.*, 1194-1198, 1988.

Souyri, Vigon, Penciolelli. Heard, Tambourin, Wendling, "A putative truncated cytokine receptor gene transduced by the myeloproliferative leukemia virus immortalizes hematopoietic progenitors," *Cell,* 63:1137-47, 1990.

Stoll, Blum, Pasquale, Murphy, "Thrombocytopenia with decreased megakaryocytes," *Evaluation and Prognosi,. Ann. Intern. Med.*, 94:170-5, 1981.

Surgenor. Wallace, Hao, Chapman, "Collection and transfusion of blood in the United States, 1982-1988," *N. Engl. J. Med.*, 322:1646-1651, 1990.

Surgenor, Wallace, Hao, Chapman, "Collection and transfusion of blood in the Untied States, 1982-1988," *The New England Journal of Medicine,* 322:1646-1650, 1990.

Suzuki. Matsumoto, Hashimoto *et al.*. "Carboplatin-based combination chemotherapy for testicular cancer: Relationship among administration dose of carboplatin. renal function and myelosuppression," *Acta Urol. Jpn.*, 41: 775-780, 1995.

Tepler, Elias, Smith II *et al.*, "A randomized placebo-controlled trial of recombinant human interleukin-11 in cancer patients with severe thrombocytopenia due to chemotherapy," *Blood,* 87(9):3607-3614, 1996.

Tepler, Elias, Smith, Hussein, Rosen, Chang *et al.*, "A randomized placebo-controlled trial of recombinant human interleukin-11 in cancer patients with severe thrombocytopenia due lo chemotherapy," *Blood,* 87:3607-3614, 1996.

The Cerenex Handbook. Robert S. Benjamin. Ed.. Cerenex Pahrmaceuticals, Research Triangle Park, N.C. (1993).

Thomas, Thibodeaux. Errert, Mathias, Marian, Meng *et al., "In vivo* biological effects of various forms of thrombopoietin in a murine model ol transient pancytopenia." *Stem Cells,* 1(Suppl 1):246-255, 1996.

Thomas, Thibodeaux. Errett *et al., "In vivo* biological effects of various forms of thrombopoietin in a murine model of transient pancytopenia," *Stem Cells,* 1(Suppl.1):246-255, 1996.

Towell, Levine, Knight in, Anderson, "A comparison of frozen and fresh platelet concentrates in the support of thrombocytopenic patients," *Transfusion,* 26:525-530, 1986.

Vadhan-Raj, Verschraegen, McGarry, Bueso-Ramos, Kudelka, Freedman, Edwards, Bevers, Levenback, Gershenson, Jones, Yang, Bast, Kavanagh, "Recombinant human thrombopoietin (rhTPO) attenuates high-dose carboplatin (C)-induced thrombocytopenia in patients with gynecologic malignancy," American Society of Hematology 39 Annual Meeting, San Diego, California, December 59, 1997.

Vadhan-Raj, Broxmeyer, Andreeff, Bandres, Buescher, Benjamin *et al., "In vivo* biologic effects of PIXY321, a synthetic hybrid protein of recombinant human granulocyte-macrophage colony-stimulating factor and interleukin-3 in cancer patients with normal hematopoiesis: a phase 1 study," *Blood,* 86:2098-2105, 1995.

Vadhan-Raj, Broxmeyer, Hittelman, Papadopoulos, Chawla, Fenoglio, "Abrogating chemotherapy-inuced myelosuppression by recombinant granulocyte-macrophage colony-stimulating factor in patients with sarcoma: protection at the progenitor cell level," 10:1266-1277, 1992.

Vadhan-Raj, Jeha, Buescher, LeMaistre, Yee, Lu, Lloreta, Hoots, Hittelman, Gutterman, Broxmeyer, "Stimulation of myelopoiesis in a patient with congenital neutropenia: Biology and nature of response to recombinant human granulocyte-macrophage colony-stimulating factor," *Blood, 75:858-864,* 1990.

Vadhan-Raj, Kudelka, Garrison *et al.*, "Effects of interleukin-1 alpha on carboplatin-induced thrombocytopenia in patients with recurrent ovarian cancer." *J. Clin. Oncol.,* 12:704-714, 1994.

Vadhan-Raj, Kudelka, Garrison, Gano, Edwards. Freedman *et al.,* Effects of interleukin-lα on carboplatin-induced thrombocytopenia in patients with recurrent ovarian cancer," *J, Clin. Oncol.*, 12:707-714, 1994.

Vadhan-Raj, Murray, Bueso-Ramos, Patel, Reddy, Hoots. Johnston, Papadopolous, Hittelman, Johnston, Yang, Paton, Cohen, Hellmann, Benjamin, Broxmeyer, "Stimulation of megakaryocyte and platelet production by a single dose of recombinant human thrombopoietin in patients with cancer," *Annals of Internal Medicine*, 126:673-681, 1997.

Vadhan-Raj, Papadopoulos, Burgess *et al.,* "Effects of PIXY321, a granulocyte-macrophage colony-stimulating factor/interleukin-3 fusion protein, on chemotherapy-induced multilineage myelosuppression in patients with sarcoma," *J. Clin. Oncol.,* 12:715-724, 1994.

Vadhan-Raj, Papadopoulos, Burgess, Linke, Patel, Hays *et al.*, "Effects of PIXY321, a granulocyte-macrophage colony-stimulating factor/interleukin-3 fusion protein, on chemotherapy-induced multilineage myelosuppression in patients with sarcoma," *J. Clin. Oncol.*, 12:715-724, 1994.

Vadhan-Raj, Patel, Broxmeyer *et al.*, "Phase I-II investigation of recombinant human thrombopoietin (rhTPO) in patients with sarcoma receiving high dose chemotherapy (CT) with adriamycin (A) and ifosfamide (I)," *Blood,* 88 (10) Suppl 1:448a, 1996.

Vadhan-Raj, Patel, Broxmeyer, Bueso-Ramos, Reddy, Papadopolous *et al.*, "Phase I-II investigation of recombinant human thrombopoietin (rhTPO) in patients with sarcoma receiving high dose chemotherapy (CT) with aoriamycin (A) and ifosfamide [Abstract]," *Blood*, 88(Suppl 1-2):448a, 1996.

Valeri, Feingold, Marchionni, "A simple method for freezing human platelets using 6% dimethylsulfoxide and storage at 80°C," *Blood,* 43:131-136, 1974.

Veldhuis, Willemse, Sleijfer, van der Graaf, Groen, Limburg *et al.*, "Toxicity and efficacy of escalating dosages of recombinant human interleukin-6 after chemotherapy in patients with breast cancer or non-small-cell lung cancer," *J. Clin. Oncol.*, 13:2585-2593, 1995.

Vose, Bierman, Kessinger, Coccia, Anderson, Oldham *et al.*, "The use of recombinant human granulocyte-macrophage colony stimulating factor for the treatment of delayed engraftment following high dose therapy and autologous hematopoietic stem cell transplantation for lymphoid malignancies," *Bone Marrow Transplant,* 7:139-143, 1991.

Wallace, Churchill, Surgenor, An, Cho, McGurk, Murphy, "Collection and transfusion of blood and blood components in the United States. 1992." *Transfusion.* 35:802-808, 1995

Wallace, Churchill, Surgenor, Cho, McGurk, "Collection and transfusion of blood and blood components in the United States, 1992," *Transfusion,* 35:802-812., 1995.

Weber, Yang, Topalian et al., "Phase I trial of subcutaneous interleukin-6 in patients with advanced malignancies," *J. Clin. Oncol.*, 11:499-506, 1993.

Wending, Maraskovsky, Debili, Florindo, Teepe, Titeaux, "C-mpl is a humoral regulator of megakaryocytopoiesis." *Nature*, 369:571-574, 1994.

Wendling, Maraskovshy, Debili, Florindo, Teepe, Titeaux *et al.,* "cMpl is a humoral regulator of megakaryocytopoiesis," *Nature*, 369:571-574, 1994;

Wendling, Maraskovsky, Debili, Florindo, Teepe, Titeux *et al.*, "c-Mpl ligand is a humoral regulator of megakaryocytopoiesis," *Nature*, 369:571-4, 1994.

Yomtovian, Lazarus, Goodnough, Hirschler, Morrissey, Jacobs, "A prospective microbiologic surveillance program to detect and prevent the transfusion of bacterially contaminated platelets," *Transfusion,* 33:902-909, 1993.

Young, Bruno, Luens, Wu, Backer, Murray, "Thrombopoietin stimulates megakaryocytopoiesis, myelopoiesis, and expansion of CD 34+ progenitor cells from single CD34$^+$Thy-1$^+$Lin$^-$ primitive progenitor cells," *Blood*, 88(5): 1619-1631, 1996.

Zucker-Franklin and Kaushansky, "Effect of thrombopoietin on the development of megakaryocytes and platelets: An ultrastructural analysis," *Blood*, 88:1632-1638, 1996.

Zucker-Franklin, "Megakaryocyte and platelet structure in thrombocyto-poiesis: The effect of cytokines," *Stem Cells,* 14:1-17, 1996.

**Claims**

1.  Use of a composition comprising TPO (thrombopoietin) for the manufacture of a medicament for treating a human having or at risk of thrombocytopenia, wherein the treatment is to comprise the steps of:

    (a) administration to a human of an agent which can cause early-nadir thromobocytopenia; and

    (b) administration to the human of one or more priming doses of a composition comprising TPO prior to administering the agent.

2.  Use according to claim 1 a composition comprising TPO for the manufacture of a medicament for treating a human having or at risk of thrombocytopenia, wherein the treatment is to comprise the steps of:

    (a) administration to a human of an agent which can cause early-nadir thromobocytopenia; and

    (b) administration to the human of one or more priming doses of said composition comprising TPO prior to administering the agent; and

    (c) administration to the human of one or more post-doses of a TPO composition after administering the agent.

3.  The use of claim 2, wherein at least two priming-doses of the TPO composition are to be administered, preferably at least three priming-doses of the TPO composition are to be administered, preferably at least four priming-doses of the TPO composition are to be administered, preferably at least five priming-doses of the TPO composition are to be administered preferably at least six priming-doses of the TPO composition are to be administered.

**4.** The use of claim 2, wherein at least two post-doses of the TPO composition are to be administered, preferably at least three post-doses of the TPO composition are to be administered, preferably at least four post-doses of the TPO composition are to be administered, preferably at least five post-doses of the TPO composition are to be administered, preferably at least six post-doses of the TPO composition are to be administered.

**5.** The use of claim 2, wherein the priming or post-doses are to be administered other than daily.

**6.** The use of claim 1, wherein up to 6 priming-doses of the TPO composition are to be administered.

**7.** The use of claim 1 or 2, wherein the priming-doses are to be administered other than daily.

**8.** The use of claim 1 or 2, wherein the nadir of said agent is of from about 10 to about 14 days.

**9.** The use of claim 8, wherein said agent is ifosfamide, adriamycin or a combination thereof.

**10.** The use of claim 1 or 2, wherein said agent is to be administered over a period of from about 3 to about 5 days.

**11.** The use of claim 10, wherein said agent is ifosfamide, adriamycin or a combination thereof.

**12.** Use of a composition comprising TPO for the manufacture of a medicament for treating a human having or at risk of thrombocytopenia, wherein the treatment is to comprise the steps of:

   (a) administration to a human of an agent which can cause thromobocytopenia; and

   (b) administration to the human of two or more post-doses of a composition comprising TPO after administering the agent, wherein the post-doses are administered other than daily.

**13.** The use of claim 12, wherein up to 15 post-doses of the TPO composition are to be administered.

**14.** The use of claim 12, wherein the nadir of said agent is of from about 12 to about 25 days.

**15.** The use of claim 14, wherein the nadir of said agent is of from about 15 to about 18 days.

**16.** The use of claim 15, wherein said agent is carboplatin.

**17.** The use of claim 12, wherein said agent is to be administered over a period of from about 1 to about 5 days.

**18.** The use of claim 17, wherein said agent is to be administered over a period of from about 1 to about 2 days.

**19.** The use of claim 17, wherein said agent is carboplatin.

**20.** Use of composition comprising TPO for the manufacture of a medicament for treating a human having thrombocytopenia, wherein said composition comprising TPO is to be administered to a human in one or more doses of said composition comprising TPO, wherein said doses are to be administered other than daily.

**21.** The use of claim 7, 20 or 24, wherein said doses are to be administered every other day.

**22.** The use of claim 20, wherein said thrombocytopenia is caused by mylodysplasian, aplastic anemia, conjential thrombocyopenia, immune thrombocytopenia, acquired thrombocytopenia, liver disease, bacterial infections, viral infections, or a combination thereof.

**23.** Use according to claim 12 of TPO in the manufacture of a medicament to be administered one or more times to a human from about 10 to 8 days prior to, or about 16 to about 21 days after, administration of an agent which can cause thrombocytopenia.

**24.** Use according to claim 12 of TPO in the manufacture of a medicament to be administered to a human one or more times from about 10 to 2 days prior to administration of an agent which can cause thrombocytopenia, and one or more times up to 21 days after such an administration of an agent.

**Patentansprüche**

1. Verwendung einer Zusammensetzung umfassend TPO (Thrombopoietin) zur Herstellung eines Medikaments zur Behandlung eines Menschen, der eine Thrombozytopenie oder ein Risiko dafür aufweist, wobei die Behandlung die Schritte umfaßt von:

   (a) Verabreichung eines Mittels an einen Menschen, das frühe-Nadir Thrombozytopenie verursachen kann; und

   (b) Verabreichung einer oder mehrerer vorbereitender Dosen einer Zusammensetzung umfassend TPO an den Menschen, vor der Verabreichung des Mittels.

2. Verwendung nach Anspruch 1 einer Zusammensetzung umfassend TPO zur Herstellung eines Medikaments zur Behandlung eines Menschen, der eine Thrombozytopenie oder ein Risiko dafür aufweist, wobei die Behandlung die Schritte umfaßt von:

   a) Verabreichen eines Mittels an einen Menschen, daß eine frühe-nadir Thrombozytopenie verursachen kann; und

   b) Verabreichen einer oder mehrerer vorbereitender Dosen der Zusammensetzung umfassend TPO, vor der Verabreichung des Mittels an den Menschen; und

   c) Verabreichung einer oder mehrerer Anschluß-Dosen einer TPO Zusammensetzung nach Verabreichung des Mittels an den Menschen.

3. Verwendung nach Anspruch 2, wobei mindestens zwei vorbereitende-Dosen der TPO-Zusammensetzung verabreicht werden müssen, bevorzugterweise mindestens drei vorbereitende Dosen der TPO-Zusammensetzung verabreicht werden müssen, bevorzugterweise mindestens vier vorbereitende Dosen der TPO-Zusammensetzung verabreicht werden müssen, bevorzugterweise mindestens fünf vorbereitende Dosen der TPO-Zusammensetzung verabreicht werden müssen, bevorzugterweise mindestens sechs vorbereitende Dosen der TPO-Zusammensetzung verabreicht werden müssen.

4. Verwendung nach Anspruch 2, wobei mindestens zwei Anschluß-Dosen der TPO-Zusammensetzung verabreicht werden müssen, bevorzugterweise mindestens drei Anschluß-Dosen der TPO-Zusammensetzung verabreicht werden müssen, bevorzugterweise mindestens vier Anschluß-Dosen der TPO-Zusammensetzung verabreicht werden müssen, bevorzugterweise mindestens fünf Anschluß-Dosen der TPO-Zusammensetzung verabreicht werden müssen, bevorzugterweise mindestens sechs Anschluß-Dosen der TPO-Zusammensetzung verabreicht werden müssen.

5. Verwendung nach Anspruch 2, wobei die vorbereitenden oder Anschluß-Dosis anders als täglich verabreicht werden müssen.

6. Verwendung nach Anspruch 1, wobei bis zu sechs vorbereitende Dosen der TPO-Zusammensetzung verabreicht werden müssen.

7. Verwendung nach Anspruch 1 oder 2, wobei die vorbereitenden Dosen anders als täglich verabreicht werden müssen.

8. Verwendung nach Anspruch 1 oder 2, wobei der Nadir des Mittels von ungefähr bis ungefähr 14 Tage ist.

9. Verwendung nach Anspruch 8, wobei das Mittel Ifosfamid, Adriamycin oder eine Kombination davon ist.

10. Verwendung nach Anspruch 1 oder 2, wobei das Mittel über eine Zeitdauer von ungefähr 3 bis ungefähr 5 Tage verabreicht werden muß.

11. Verwendung nach Anspruch 10, wobei das Mittel Ifosfamid, Adriamycin oder eine Kombination davon ist.

12. Verwendung einer Zusammensetzung umfassend TPO zur Herstellung eines Medikaments zur Behandlung eines Menschen, der Thrombozytopenie oder ein Risiko dafür aufweist, wobei die Behandlung die Schritte umfaßt von:

(a) Verabreichung eines Mittels an einen Menschen, das Thrombozytopenie verursachen kann; und

(b) Verabreichung an den Menschen von zwei oder mehr Anschluß-Dosen einer Zusammensetzung umfassend TPO nach Verabreichung des Mittels, wobei die Anschluß-Dosen anders als täglich verabreicht werden.

13. Verwendung nach Anspruch 12, wobei bis zu 15 Anschluß-Dosen der TPO-Zusammensetzung verabreicht werden müssen.

14. Verwendung nach Anspruch 12, wobei der Nadir des Mittels von ungefähr 12 bis ungefähr 25 Tage ist.

15. Verwendung nach Anspruch 14, wobei der Nadir des Mittels von ungefähr 15 bis ungefähr 18 Tage ist.

16. Verwendung nach Anspruch 15, wobei das Mittel Carboplatin ist.

17. Verwendung nach Anspruch 12, wobei das Mittel über eine Zeitdauer von ungefähr 1 bis ungefähr 5 Tage zu verabreichen ist.

18. Verwendung nach Anspruch 17, wobei das Mittel über eine Zeitdauer von ungefähr 1 bis ungefähr 2 Tage zu verabreichen ist.

19. Zusammensetzung nach Anspruch 17, wobei das Mittel Carboplatin ist.

20. Verwendung einer Zusammensetzung umfassend TPO zur Herstellung eines Medikaments zur Behandlung eines Menschen, der Thrombozytopenie aufweist, wobei die Zusammensetzung, die TPO umfaßt, an einen Menschen in einer oder mehreren Dosen der Zusammensetzung, die TPO umfaßt, verabreicht wird, wobei die Dosen anders als täglich verabreicht werden müssen.

21. Verwendung nach Anspruch 7, 20 oder 24, wobei die Dosen jeden zweiten Tag verabreicht werden müssen.

22. Verwendung nach Anspruch 20, wobei die Thrombozytopenie verursacht wird durch Myelodysplasie, aplastische Anämie, vererbte Thromobzytopenie, Immunthrombozytopenie, erworbene Thrombozytopenie, Lebererkrankung, bakterielle Infektionen, virale Infektionen oder eine Kombination davon.

23. Verwendung nach Anspruch 12 von TPO bei der Herstellung eines Medikaments, das an einen Menschen ein oder mehrere Male von ungefähr 10 bis ungefähr 8 Tage vor oder ungefähr 16 bis ungefähr 21 Tage nach Verabreichung eines Mittels, das Thrombozytopenie verursacht, verabreicht werden muß.

24. Verwendung nach Anspruch 12 von TPO bei der Herstellung eines Medikaments, das an einen Menschen ein oder mehrere Male von ungefähr 10 bis ungefähr 2 Tage vor der Verabreichung eines Mittels, das Thrombozytopenie verursachen kann, und ein oder mehrere Male bis zu 21 Tage nach solch einer Verabreichung eines Mittels verabreicht werden muß.

**Revendications**

1. Utilisation d'une composition comprenant de la TPO (thrombopoiétine) pour la fabrication d'un médicament destiné à traiter un humain présentant ou risquant de présenter une thrombocytopénie, dans laquelle le traitement doit comprendre les étapes consistant à :

(a) administrer à un être humain un agent qui peut provoquer une thrombocytopénie à nadir précoce ; et

(b) administrer à l'être humain une ou plusieurs doses d'amorçage d'une composition comprenant de la TPO avant l'administration de l'agent.

2. Utilisation selon la revendication 1, d'une composition comprenant de la TPO pour la fabrication d'un médicament destiné à traiter un être humain présentant ou risquant de présenter une thrombocytopénie, dans laquelle le traitement doit comprendre les étapes consistant à :

(a) administrer à un être humain un agent pouvant provoquer une thrombocytopénie à nadir précoce; et

(b) administrer à l'être humain une ou plusieurs doses d'amorçages de ladite composition comprenant de la TPO avant l'administration de l'agent ; et

(c) administrer à l'être humain une ou plusieurs post-doses d'une composition de TPO après l'administration de l'agent.

3. Utilisation selon la revendication 2, dans laquelle au moins deux doses d'amorçage de la composition de TPO doivent être administrées, de préférence au moins trois doses d'amorçage de la composition de TPO doivent être administrées, de préférence au moins quatre doses d'amorçage de la composition de TPO doivent être administrées, de préférence au moins cinq doses d'amorçage de la composition doivent être administrées, de préférence au moins six doses d'amorçage de la composition doivent être administrées.

4. Utilisation selon la revendication 2, dans laquelle au moins deux post-doses de la composition de TPO doivent être administrées, de préférence au moins trois post-doses de la composition de TPO doivent être administrées, de préférence au moins quatre post-doses de la composition de TPO doivent être administrées, de préférence au moins cinq post-doses de la composition doivent être administrées, de préférence au moins six post-doses de la composition doivent être administrées.

5. Utilisation selon la revendication 2, dans laquelle les doses d'amorçage ou les post-doses doivent être administrées autrement que quotidiennement.

6. Utilisation selon la revendication 1, dans laquelle jusqu'à 6 doses d'amorçage de la composition de TPO doivent être administrées.

7. Utilisation selon la revendication 1 ou selon la revendication 2, dans laquelle les doses d'amorçage doivent être administrées autrement que quotidiennement.

8. Utilisation selon la revendication 1 ou la revendication 2, dans laquelle le nadir dudit agent est environ 10 à environ 14 jours.

9. Utilisation selon la revendication 8, dans laquelle ledit agent est l'ifosfamide, l'adriamycine ou une combinaison de ceux-ci.

10. Utilisation selon la revendication 1 ou la revendication 2, dans laquelle ledit agent doit être administré pendant une période comprise entre environ 3 jours et environ 5 jours.

11. Utilisation selon la revendication 10, dans laquelle ledit agent est l'ifosfamide, l'adriamycine ou une combinaison de ceux-ci.

12. Utilisation d'une composition comprenant de la TPO pour la production d'un médicament destiné à traiter un être humain présentant ou risquant de présenter une thrombocytopénie, dans laquelle le traitement doit comprendre les étapes consistant à :

(a) administrer à un être humain un agent qui peut provoquer une thrombocytopénie ; et

(b) administrer à l'être humain une post-dose ou plus d'une composition comprenant de la TPO après l'administration de l'agent, dans laquelle les post-doses sont administrées autrement que quotidiennement.

13. Utilisation selon la revendication 12, dans laquelle jusqu'à 15 post-doses de la composition de TPO doivent être administrées.

14. Utilisation selon la revendication 12, dans laquelle le nadir dudit agent est environ 12 à environ 25 jours.

15. Utilisation selon la revendication 14, dans laquelle le nadir dudit agent est environ 15 à environ 18 jours.

**16.** Utilisation selon la revendication 15, dans laquelle ledit agent est la carboplatine.

**17.** Utilisation selon la revendication 12, dans laquelle l'agent doit être administré pendant une période d'environ 1 à environ 5 jours.

**18.** Utilisation selon la revendication 17, dans laquelle l'agent doit être administré pendant une période d'environ 1 à environ 2 jours.

**19.** Composition selon la revendication 17, dans laquelle ledit agent est la carboplatine.

**20.** Utilisation d'une composition comprenant de la TPO pour la fabrication d'un médicament destiné à traiter un être humain présentant une thrombocytopénie, dans laquelle ladite composition comprenant de la TPO doit être administrée à un être humain en une ou plusieurs doses de ladite composition comprenant de la TPO, dans laquelle les doses doivent être administrées autrement que quotidiennement.

**21.** Utilisation selon la revendication 7, 20, ou 24, dans laquelle lesdites doses doivent être administrées tous les deux jours.

**22.** Utilisation selon la revendication 20, dans laquelle ladite thrombocytopénie est provoquée par une myélodysplasie, une anémie aplastique, une thrombocytopénie constitutionnelle, une thrombocytopénie immunitaire, une thrombocytopénie acquise, une pathologie du foie, des infections bactériennes, ou une combinaison de celles-ci.

**23.** Utilisation selon la revendication 12 de TPO dans la fabrication d'un médicament à administrer une ou plusieurs fois à un être humain environ 10 à 8 jours avant, ou environ 16 à environ 21 jours après, l'administration d'un agent qui peut provoquer une thrombocytopénie.

**24.** Utilisation selon la revendication 12 de TPO dans la fabrication d'un médicament destiné à être administré à un être humain une ou plusieurs fois environ 12 à 2 jours avant l'administration d'un agent qui peut provoquer une thrombocytopénie, et une ou plusieurs fois jusqu'à 21 jours après une telle administration d'un agent.

FIG. 1

FIG. 2

FIG. 3

FIG. 4A

**FIG. 4B**

FIG. 5

FIG. 6

FIG. 7

FIG. 8